(19) **Europäisches Patentamt**
**European Patent Office**
**Office européen des brevets**

(11) **EP 4 204 591 B1**

(12) **EUROPEAN PATENT SPECIFICATION**

(45) Date of publication and mention
of the grant of the patent:
**25.12.2024 Bulletin 2024/52**

(21) Application number: **21769705.1**

(22) Date of filing: **27.08.2021**

(51) International Patent Classification (IPC):
**C12Q 1/6886** *(2018.01)*

(52) Cooperative Patent Classification (CPC):
**C12Q 1/6886; C12Q 2600/106; C12Q 2600/158**

(86) International application number:
**PCT/EP2021/073802**

(87) International publication number:
**WO 2022/043530 (03.03.2022 Gazette 2022/09)**

(54) **METHOD OF ASSESSING THE CIRCADIAN RHYTHM OF A SUBJECT HAVING CANCER AND/OR ASSESSING A TIMING OF ADMINISTRATION OF A MEDICAMENT TO SAID SUBJECT HAVING CANCER**

VERFAHREN ZUR BEWERTUNG DES ZIRKADIANEN RHYTHMUS EINES SUBJEKTS MIT KREBS UND/ODER BEWERTUNG EINES ZEITPUNKTS ZUR VERABREICHUNG EINES MEDIKAMENTS AN DAS SUBJEKT MIT KREBS

PROCÉDÉ D'ÉVALUATION DU RYTHME CIRCADIEN D'UN SUJET AYANT UN CANCER ET/OU D'UNE SYNCHRONISATION D'ADMINISTRATION D'UN MÉDICAMENT AUDIT UN SUJET AYANT UN CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.08.2020 EP 20193231**

(43) Date of publication of application:
**05.07.2023 Bulletin 2023/27**

(73) Proprietor: **Moreira Borralho Relógio, Angela 22085 Hamburg (DE)**

(72) Inventors:
• **HESSE, Janina**
**13629 Berlin (DE)**
• **AKHONDZADEH BASTI, Alireza**
**14167 Berlin (DE)**
• **MOREIRA BORRALHO RELÓGIO, Angela**
**10439 Berlin (DE)**

(74) Representative: **Kilger, Ute Boehmert & Boehmert Anwaltspartnerschaft mbB Pettenkoferstrasse 22 80336 München (DE)**

(56) References cited:
**WO-A2-2011/094745    US-A1- 2018 057 886**

• INOKAWA HIROSHI ET AL: "Evaluation of multidrug cancer chronotherapy based on cell cycle model under influences of circadian clock", 2016 38TH ANNUAL INTERNATIONAL CONFERENCE OF THE IEEE ENGINEERING IN MEDICINE AND BIOLOGY SOCIETY (EMBC), IEEE, 16 August 2016 (2016-08-16), pages 1439 - 1442, XP032979431, [retrieved on 20161013], DOI: 10.1109/EMBC.2016.7590979

• SHERRATT MICHAEL J ET AL: "Circadian rhythms in skin and other elastic tissues", MATRIX BIOLOGY, ELSEVIER, NL, vol. 84, 15 August 2019 (2019-08-15), pages 97 - 110, XP086035835, ISSN: 0945-053X, [retrieved on 20190815], DOI: 10.1016/J.MATBIO.2019.08.004

• EMILY A. SLAT ET AL: "Cell-intrinsic, Bmal1-dependent Circadian Regulation of Temozolomide Sensitivity in Glioblastoma", JOURNAL OF BIOLOGICAL RHYTHMS., vol. 32, no. 2, 1 April 2017 (2017-04-01), GB, pages 121 - 129, XP055766051, ISSN: 0748-7304, DOI: 10.1177/0748730417696788

- SHUBONI-MULLIGAN DORELA D ET AL: "Radiation chronotherapy-clinical impact of treatment time-of-day: a systematic review", JOURNAL OF NEURO-ONCOLOGY, SPRINGER US, NEW YORK, vol. 145, no. 3, 15 November 2019 (2019-11-15), pages 415 - 427, XP036936920, ISSN: 0167-594X, [retrieved on 20191115], DOI: 10.1007/S11060-019-03332-7
- FUSTIN JEAN-MICHEL ET AL: "Rhythm on a chip: circadian entrainment in vitro is the next frontier in body-on-a chip technology", CURRENT OPINION IN PHARMACOLOGY, ELSEVIER SCIENCE PUBLISHERS, NL, vol. 48, 1 October 2019 (2019-10-01), pages 127 - 136, XP085903464, ISSN: 1471-4892, [retrieved on 20191007], DOI: 10.1016/J.COPH.2019.09.005
- GIANLUIGI MAZZOCCOLI ET AL: "Comparison of circadian characteristics for cytotoxic lymphocyte subsets in non-small cell lung cancer patients versus controls", CLINICAL AND EXPERIMENTAL MEDICINE, SPRINGER-VERLAG, MI, vol. 12, no. 3, 11 September 2011 (2011-09-11), pages 181 - 194, XP035091134, ISSN: 1591-9528, DOI: 10.1007/S10238-011-0153-6
- TAKEDA N ET AL: "Cardiovascular disease, chronopharmacotherapy, and the molecular clock", ADVANCED DRUG DELIVERY REVIEWS, ELSEVIER, AMSTERDAM , NL, vol. 62, no. 9-10, 31 July 2010 (2010-07-31), pages 956 - 966, XP027212619, ISSN: 0169-409X, [retrieved on 20100731]
- DICKMEIS T ET AL: "Glucocorticoids and circadian clock control of cell proliferation: At the interface between three dynamic systems", MOLECULAR AND CELLULAR ENDOCRINOLOGY, ELSEVIER IRELAND LTD, IE, vol. 331, no. 1, 1 January 2011 (2011-01-01), pages 11 - 22, XP027484881, ISSN: 0303-7207, [retrieved on 20101109], DOI: 10.1016/J.MCE.2010.09.001
- HAUS ET AL: "Chronobiology in the endocrine system", ADVANCED DRUG DELIVERY REVIEWS, ELSEVIER, AMSTERDAM , NL, vol. 59, no. 9-10, 9 October 2007 (2007-10-09), pages 985 - 1014, XP022289545, ISSN: 0169-409X, DOI: 10.1016/J.ADDR.2007.01.001
- SOLOVYOV I A ET AL: "Genetic control of circadian rhythms and aging", RUSSIAN JOURNAL OF GENETICS, MOSCOW, RU, vol. 52, no. 4, 19 May 2016 (2016-05-19), pages 343 - 361, XP035707820, ISSN: 1022-7954, [retrieved on 20160519], DOI: 10.1134/S1022795416040104
- ROISIN SULLIVAN ET AL: "An optimised saliva collection method to produce high-yield, high-quality RNA for translational research", PLOS ONE, vol. 15, no. 3, 9 March 2020 (2020-03-09), pages e0229791, XP055765879, DOI: 10.1371/journal.pone.0229791
- JESSICA A. DIETZ ET AL: "Optimal Techniques for mRNA Extraction from Neonatal Salivary Supernatant", NEONATOLOGY, vol. 101, no. 1, 1 January 2012 (2012-01-01), CH, pages 55 - 60, XP055765757, ISSN: 1661-7800, DOI: 10.1159/000328026
- ANNABELLE BALLESTA ET AL: "A Combined Experimental and Mathematical Approach for Molecular-based Optimization of Irinotecan Circadian Delivery", PLOS COMPUTATIONAL BIOLOGY, vol. 7, no. 9, 8 September 2011 (2011-09-08), pages e1002143, XP055768530, DOI: 10.1371/journal.pcbi.1002143
- CONSTANCE AHOWESSO ET AL: "Sex and Dosing-Time Dependencies in Irinotecan-Induced Circadian Disruption", CHRONOBIOLOGY INTERNATIONAL, vol. 28, no. 5, 1 May 2011 (2011-05-01), GB, pages 458 - 470, XP055768533, ISSN: 0742-0528, DOI: 10.3109/07420528.2011.569043
- DONG DONG ET AL: "Circadian rhythm in pharmacokinetics and its relevance to chronotherapy", BIOCHEMICAL PHARMACOLOGY, ELSEVIER, US, vol. 178, 22 May 2020 (2020-05-22), XP086219947, ISSN: 0006-2952, [retrieved on 20200522], DOI: 10.1016/J.BCP.2020.114045
- INNOMINATO P F ET AL: "Chronotherapy and the molecular clock: Clinical implications in oncology", ADVANCED DRUG DELIVERY REVIEWS, ELSEVIER, AMSTERDAM , NL, vol. 62, no. 9-10, 31 July 2010 (2010-07-31), pages 979 - 1001, XP027212621, ISSN: 0169-409X, [retrieved on 20100623]
- OHDO S ET AL: "Chronopharmacological strategies: Intra- and inter-individual variability of molecular clock", ADVANCED DRUG DELIVERY REVIEWS, ELSEVIER, AMSTERDAM , NL, vol. 62, no. 9-10, 31 July 2010 (2010-07-31), pages 885 - 897, XP027212613, ISSN: 0169-409X, [retrieved on 20100602]
- RANA SOBIA ET AL: "Circadian rhythm and its role in malignancy", JOURNAL OF CIRCADIAN RHYTHMS, BIOMED CENTRAL, LONDON, GB, vol. 8, no. 1, 31 March 2010 (2010-03-31), pages 3, XP021079322, ISSN: 1740-3391, DOI: 10.1186/1740-3391-8-3
- PRASANNA V ASHOK KUMAR ET AL: "It's About Time: Advances in Understanding the Circadian Regulation of DNA Damage and Repair in Carcinogenesis and Cancer Treatment Outcomes", THE YALE JOURNAL OF BIOLOGY & MEDICINE, 1 June 2019 (2019-06-01), United States, pages 305 - 316, XP055766015, Retrieved from the Internet <URL:https://www.ncbi.nlm.nih.gov/pmc/articles/PMC6585512/pdf/yjbm_92_2_305.pdf>

**(Cont. next page)**

Remarks:
The complete document including Reference Table(s) and the Sequence Listing(s) can be downloaded from the EPO website

Remarks:
The complete document including Reference Table(s) and the Sequence Listing(s) can be downloaded from the EPO website

## Description

Subject matter of the invention is a method of assessing the circadian rhythm of a subject having cancer and/or assessing a timing of administration of a medicament to said subject having cancer, wherein said method comprises the steps of:

- Providing at least three samples of saliva, more preferably four samples of saliva, from said subject, wherein said samples have been taken at different time points over the day;
- Determining gene expression the following genes in each of said samples:

  ○ of at least two members of genes for the core-clock network, in particular of at least two members of the group comprising ARNTL (BMAL1), ARNTL2, CLOCK, PER1, PER2, PER3, NPAS2, CRY1, CRY2, NR1D1, NR1D2, RORA, RORB, RORC in particular ARNTL (BMAL1) and PER2, and
  ○ at least one gene involved in the metabolism of a medicament to be administered to said subject having cancer, including Ces2, and
  ○ at least one drug target gene that is a target of the medicament to be administered, and

- Assessing and predicting by means of a computational step based on said expression levels of said genes over the day the circadian rhythm of said subject and/or assessing a timing of administration of said medicament to said subject, comprising assessing the optimal time of administration of said medicament to said subject and/or assessing the non-optimal time of administration of said medicament to said subject.

## State of the art

The biological clock (also known as circadian clock) regulates several aspects of physiology and behavior via cellular and molecular mechanisms and plays a vital role in maintaining proper human health. This is no wonder, since about half of all human genes are rhythmically expressed in at least one tissue. The disruption of circadian rhythms is associated to several diseases including sleep disorders, depression, diabetes, Alzheimer's disease, obesity and cancer. This disruption might result from conflicting external (environmental) or internal (feeding/resting) signals that are not in synchrony with the internal biological time. This not only affects shift workers, but also most people subjected to societal routine (social jet lag). Furthermore, a desynchronized circadian clock was shown to negatively affect an individual's wellbeing, in particular in the context of metabolism, as well as physical and mental (cognitive) performance.

Recent studies, including work from our research group have reported a role for clock dysregulation in the pathologies mentioned above, raising increased awareness from scientists, clinicians and the public. Thus, it is crucial to characterize the individual's internal clock and to adjust the external/internal factors, to avoid or to overcome circadian rhythm disruption. Moreover, the time for certain activities, such as sleep, sports or medicine intake, can be optimized based on the individual's internal timing. A proper functioning circadian clock, synchronized with the individual's behavioral habits, will improve fitness and reduce therapy/recovery time in patients.

Yet, the available methods for clock assessment are either not very accurate or mostly invasive and often require medical assistance over a period of several hours (tedious, time-consuming, and cost-intensive). So far, there are the following methods among the molecular approaches to determine the biological clock in humans:

1. Determination of time-point at which endogenous melatonin (or cortisol), reaches a predefined threshold value concentration (dim-light-melatonin-onset, aka DLMO). To do this, several blood or saliva samples (usually every 0.5-1 hour) under dim-light conditions are taken to determine the internal clock.

2. Blood samples (with one or more sampling times) are also used to identify biomarkers using a machine learning approach that could determine the expression phase of "time-indicating" genes and thus the internal clock timing.

3. In addition, the circadian phase (peak time of secretion/expression) in hair samples (hair follicle cells) or urine samples could reflect that of the individual behavioral rhythm. This strategy is more suitable for assessing the human peripheral clock.

As mentioned above, current methods of clock assessment are either invasive, require medical supervision, are time-consuming or do not provide detailed information on the gene expression level. Saliva plays numerous protective roles for oral tissue maintenance in humans. Adequate salivary flow and saliva content are directly related to health status. Previous studies have shown the potential of saliva and salivary transcriptome as a diagnostic tool, which underlines the importance of saliva sampling as a non-invasive diagnostic method. Time-course saliva sampling is also commonly used for estimating the evening dim-light melatonin onset (DLMO) in humans in order to determine their circadian phase (peak time of secretion/expression); a method that requires controlled dim-light conditions during the entire sampling time of 5-6h.

The circadian rhythm was previously modeled with different approaches, starting with models that simply show oscillations such as phase-oscillators, and going up to molecular models, which model (part of) the molecular interactions underlying the circadian clock. In the present disclosure it is focused on molecular models, because these contain biological information that might be useful for predictions. Molecular models with simple feedback loops are often based on Goodwin's oscillator, e.g. (Ruoff and Rensing 1996), but the level of detail may also be extensive (Forger and Peskin 2003).

An objective is to provide a model at an intermediate state of complexity, complex enough to capture a significant part of the genetic network, but if the model is too complex, we cannot fit our data to the model without significant overfitting. Relogio et al. have published a model at this level of complexity, with 19 dynamical variables, which we use in the following (Relógio et al. 2011).

Other studies with mammalian clock models are named below, including respective literal citations from the respective papers.

(Becker-Weimann et al. 2004): "We present a mathematical model that reflects the essential features of the mammalian circadian oscillator to characterize the differential roles of negative and positive feedback loops. The oscillations that are obtained have a 24-h period and are robust toward parameter variations even when the positive feedback is replaced by a constantly expressed activator. This demonstrates the crucial role of the negative feedback for rhythm generation." [7 dynamical variables]

(Forger and Peskin 2003): "Here we develop a detailed distinctly mammalian model by using mass action kinetics. Parameters for our model are found from experimental data by using a coordinate search method. The model accurately predicts the phase of entrainment, amplitude of oscillation, and shape of time profiles of clock mRNAs and proteins and is also robust to parameter changes and mutations." [74 dynamical variables] There also is a stochastic version of this model (Forger and Peskin 2005).

(Leloup and Goldbeter 2003): "We present a computational model for the mammalian circadian clock based on the intertwined positive and negative regulatory loops involving the Per, Cry, Bmal1, Clock, and Rev-Erb $\alpha$ genes. In agreement with experimental observations, the model can give rise to sustained circadian oscillations in continuous darkness, characterized by an antiphase relationship between Per/Cry/Rev-Erb$\alpha$ and Bmal1 mRNAs. Sustained oscillations correspond to the rhythms autonomously generated by suprachiasmatic nuclei. For other parameter values, damped oscillations can also be obtained in the model. These oscillations, which transform into sustained oscillations when coupled to a periodic signal, correspond to rhythms produced by peripheral tissues." [19 dynamical variables] Bifurcation analysis of this model published in (Leloup and Goldbeter 2004).

(Mirsky et al. 2009): "In this study, we built a mathematical model from the regulatory structure of the intracellular circadian clock in mice and identified its parameters using an iterative evolutionary strategy, with minimum cost achieved through conformance to phase separations seen in cell-autonomous oscillators. The model was evaluated against the experimentally observed cell-autonomous circadian phenotypes of gene knockouts, particularly retention of rhythmicity and changes in expression level of molecular clock components." "Most importantly, our model addresses the overlapping but differential functions of CRY1 and CRY2 in the clock mechanism: They antagonistically regulate period length and differentially control rhythm persistence and amplitude" [21 dynamical variables] This model focuses on the phase relationship between different genes.

(Kim and Forger 2012): "To understand the biochemical mechanisms of this timekeeping, we have developed a detailed mathematical model of the mammalian circadian clock. Our model can accurately predict diverse experimental data including the phenotypes of mutations or knockdown of clock genes as well as the time courses and relative expression of clock transcripts and proteins. Using this model, we show how a universal motif of circadian timekeeping, where repressors tightly bind activators rather than directly binding to DNA, can generate oscillations when activators and repressors are in stoichiometric balance." [Ref: Kim, Jae Kyoung, and Daniel B Forger. 2012. "A Mechanism for Robust Circadian Timekeeping via Stoichiometric Balance." Molecular Systems Biology 8 (December): 630. https://doi.org/10.1038/msb.2012.62.]

(Jolley et al. 2014): Focus on a new mechanism via D-box, and modern parameter estimation.

"Genetic Control of Circadian Rhythms and Aging" by I. A. Solovyov et al., discusses how living organisms have evolved to adapt to environmental changes, developing molecular oscillators to synchronize biological processes with environmental rhythms. The study explores the association between circadian rhythm genes and aging, reviewing the function of oscillators at different levels and their connection to aging genetic determinants and the pathogenesis of age-related diseases. It also presents comparative analyses of the expression of circadian genes in animal tissues with varying rates of aging. The article emphasizes the significant role of circadian genes in physiological processes and their changes in expression during aging.

Besides these models of the mammalian circadian clock, various models have been published for non-mammalian systems, and intensively investigated, e.g. by bifurcation analysis.

"An optimised saliva collection method to produce high-yield, high-quality RNA for translational research," by Roisin Sullivan, discloses a study that compares different saliva collection and processing methods to enhance RNA yield and quality for diagnostic purposes. It reports that using RNAlater as a preservative increased mean RNA yield and improved RNA Integrity Number (RIN). The study further suggests that saliva can be stored at room temperature without significant RNA degradation and that overnight fasting prior to collection may result in higher RNA concentrations. The findings also indicate that their optimized method is feasible for clinical settings and could potentially enable at-home sample collection for research laboratory analysis.

"Optimal Techniques for mRNA Extraction from Neonatal Salivary Supernatant," by Jessica A. Deitz et al., was published in Neonatology 2012. The study aimed to develop enhanced techniques to extract cell-free RNA from neonatal salivary supernatant, comparing two modified extraction methods that yielded successful mRNA extraction from all samples. The modified protocol using the QIAamp Viral RNA Mini Kit (method 2) was found to be more effective in yielding RNA compared to the RNAprotect Saliva Mini Kit (method 1). Additionally, paired microarray analyses between neonatal whole saliva and supernatant suggested distinct biological functions unique to each, highlighting the potential for noninvasive monitoring of systemic organ development in neonates.

WO 2011/094745 A2 discloses a biological sample collection system designed for collecting and preserving biological samples, specifically saliva, urine, and stool, for DNA and/or RNA extraction. The system comprises a biological sample collecting wand with a detachable handle and a sample collector that includes protrusions and apertures. The sample collector can be inserted into a buffer container with a solution for preserving the collected biological sample. The system is advantageous for its ease of use, quick collection times, and the ability to maintain the integrity of samples for genetic testing and other analyses requiring DNA or RNA.

"Sex and Dosing-Time Dependencies in Irinotecan-Induced Circadian Disruption" by Constance Ahowesso et al. discloses that irinotecan, an anticancer drug, disrupts circadian rhythms in mice, with effects varying according to sex and dosing time. The study shows that female mice experience more severe circadian disruptions when the drug is administered at a time associated with worst tolerability, while male mice display moderate disruptions regardless of dosing time. Baseline circadian rhythms in rest-activity, body temperature, and plasma corticosterone were more pronounced in females. The findings underscore the need for sex-specific, optimally-timed chemotherapy schedules to minimize circadian disruption and improve tolerability.

## Summary of the invention

Subject matter of the invention is a method of assessing the circadian rhythm of a subject having cancer and/or assessing a timing of administration of a medicament to said subject having cancer, wherein said method comprises the steps of:

- Providing at least three samples of saliva, more preferably four samples of saliva, from said subject, wherein said samples have been taken at different time points over the day;
- Determining gene expression of at least the following genes in each of said samples:

    ◦ of at least two members of the core-clock network in each of said samples, in particular of at least two members of the following genes, of the groups comprising ARNTL (BMAL1), ARNTL2, CLOCK, PER1, PER2, PER3, NPAS2 , CRY1, CRY2, NR1D1, NR1D2, RORA, RORB, RORC, in particular ARNTL (BMAL1) and PER2, and
    ◦ at least one gene involved in the metabolism of a medicament to be administered to said subject having cancer, including Ces2, and
    ◦ at least one drug target gene that is a target of the medicament to be administered, and

- Assessing and predicting by means of a computational step based on said expression levels of said genes over the day the circadian rhythm of said subject and/or assessing a timing of administration of said medicament to said subject, comprising assessing the optimal time of administration of said medicament to said subject and/or assessing the non-optimal time of administration of said medicament to said subject.

In one embodiment of the method according to the present invention gene expression is determined using a method selected from quantitative PCR (RT-qPCR), NanoString, sequencing and microarray. Any other method for determining gene expression may be used.

In one embodiment of the method according to the present invention gene expression is determined using quantitative PCR (RT-qPCR). https://pubmed.ncbi.nlm.nih.gov/15956331

In one embodiment of the method according to the present invention gene expression is determined using NanoString. Geiss G, et al., Direct multiplexed measurement of gene expression with color-coded probe pairs, 26: 317-25 (2008)., Nature Biotechnology, Feb 08, 2008.

BMAL1is also known as ARNTL, Aryl hydrocarbon receptor nuclear translocator-like protein 1 (ARNTL) Or Brain and Muscle ARNT-Like 1 (BMAL1).

The sequence of cDNA ARNTL (BMAL1) comprises SEQ ID No.1:
>ENST00000389707.8 ARNTL-201 cdna:protein_coding

```
ATGGCAGACCAGAGAATGGACATTTCTTCAACCATCAGTGATTTCATGTCCCCGGGCCCC
ACCGACCTGCTTTCCAGCTCTCTTGGTACCAGTGGTGTGGATTGCAACCGCAAACGGAAA
GGCAGCTCCACTGACTACCAAGAAAGCATGGACACAGACAAAGATGACCCTCATGGAAGG
TTAGAATATACAGAACACCAAGGAAGGATAAAAAATGCAAGGGAAGCTCACAGTCAGATT
GAAAAGCGGCGTCGGGATAAAATGAACAGTTTTATAGATGAATTGGCTTCTTTGGTACCA
ACATGCAACGCAATGTCCAGGAAATTAGATAAACTTACTGTGCTAAGGATGGCTGTTCAG
CACATGAAAACATTAAGAGGTGCCACCAATCCATACACAGAAGCAAACTACAAACCAACT
TTTCTATCAGACGATGAATTGAAACACCTCATTCTCAGGGCAGCAGATGGATTTTTGTTT
```

```
GTCGTAGGATGTGACCGAGGGAAGATACTCTTTGTCTCAGAGTCTGTCTTCAAGATCCTC
AACTACAGCCAGAATGATCTGATTGGTCAGAGTTTGTTTGACTACCTGCATCCTAAAGAT
ATTGCCAAAGTCAAGGAGCAGCTCCTCCTCTGACACCGCACCCCGGGAGCGGCTCATA
GATGCAAAAACTGGACTTCCAGTTAAAACAGATATAACCCCTGGGCCATCTCGATTATGT
TCTGGAGCACGACGTTCTTTCTTCTGTAGGATGAAGTGTAACAGGCCTTCAGTAAAGGTT
GAAGACAAGGACTTCCCCTCTACCTGCTCAAAGAAAAAAGATCGAAAAAGCTTCTGCACA
ATCCACAGCACAGGCTATTTGAAAAGCTGGCCACCCACAAAGATGGGGCTGGATGAAGAC
AACGAACCAGACAATGAGGGGTGTAACCTCAGCTGCCTCGTCGCAATTGGACGACTGCAT
TCTCATGTAGTTCCACAACCAGTGAACGGGGAAATCAGGGTGAAATCTATGGAATATGTT
TCTCGGCACGCGATAGATGGAAAGTTTGTTTTTGTAGACCAGAGGGCAACAGCTATTTTG
GCATATTTACCACAAGAACTTCTAGGCACATCGTGTTATGAATATTTTCACCAAGATGAC
ATAGGACATCTTGCAGAATGTCATAGGCAAGTTTTACAGACGAGAGAAAAAATTACAACT
AATTGCTATAAATTTAAAATCAAAGATGGTTCTTTTATCACACTACGGAGTCGATGGTTC
AGTTTCATGAACCCTTGGACCAAGGAAGTAGAATATATTGTCTCAACTAACACTGTTGTT
TTAGCCAACGTCCTGGAAGGCGGGGACCCAACCTTCCCACAGCTCACAGCATCCCCCCAC
AGCATGGACAGCATGCTGCCCTCTGGAGAAGGTGGCCCAAAGAGGACCCACCCCACTGTT
CCAGGGATTCCAGGGGGAACCCGGGCTGGGGCAGGAAAAATAGGCCGAATGATTGCTGAG
GAAATCATGGAAATCCACAGGATAAGAGGGTCATCGCCTTCTAGCTGTGGCTCCAGCCCA
TTGAACATCACGAGTACGCCTCCCCCTGATGCCTCTTCTCCAGGAGGCAAGAAGATTTTA
AATGGAGGGACTCCAGACATTCCTTCCAGTGGCCTACTATCAGGCCAGGCTCAGGAGAAC
CCAGGTTATCCATATTCTGATAGTTCTTCTATTCTTGGTGAGAACCCCCACATAGGTATA
GACATGATTGACAACGACCAAGGATCAAGTAGTCCCAGTAATGATGAGGCAGCAATGGCT
GTCATCATGAGCCTCTTGGAAGCAGATGCTGGACTGGGTGGCCCTGTTGACTTTAGTGAC
TTGCCATGGCCGCTGTAA
```

The sequence of cDNA ARNTL2 comprises SEQ ID No.2
>ENST00000266503.9 ARNTL2-202 cdna:protein_coding

ATGGCGGCGGAAGAGGAGGCTGCGGCGGGAGGTAAAGTGTTGAGAGAGGAGAACCAGTGC
ATTGCTCCTGTGGTTTCCAGCCGCGTGAGTCCAGGGACAAGACCAACAGCTATGGGGTCT
TTCAGCTCACACATGACAGAGTTTCCACGAAAACGCAAAGGAAGTGATTCAGACCCATCC
CAGTCAGGAATCATGACAGAAAAGTGGTGGAAAAGCTTTCTCAGAATCCCCTTACCTAT
CTTCTTTCAACAAGGATAGAAATATCAGCCTCCAGTGGCAGCAGAGTGGAAGATGGTGAA
CACCAAGTTAAAATGAAGGCCTTCAGAGAAGCTCATAGCCAAACTGAAAAGCGGAGGAGA
GATAAAATGAATAACCTGATTGAAGAACTGTCTGCAATGATCCCTCAGTGCAACCCCATG
GCGCGTAAACTGGACAAACTTACAGTTTTAAGAATGGCTGTTCAACACTTGAGATCTTTA
AAAGGCTTGACAAATTCTTATGTGGGAAGTAATTATAGACCATCATTTCTTCAGGATAAT
GAGCTCAGACATTTAATCCTTAAGACTGCAGAAGGCTTCTTATTTGTGGTTGGATGTGAA
AGAGGAAAAATTCTCTTCGTTTCTAAGTCAGTCTCCAAAATACTTAATTATGATCAGGCT
AGTTTGACTGGACAAAGCTTATTTGACTTCTTACATCCAAAAGATGTTGCCAAAGTAAAG
GAACAACTTTCTTCTTTTGATATTTCACCAAGAGAAAAGCTAATAGATGCCAAAACTGGT
TTGCAAGTTCACAGTAATCTCCACGCTGGAAGGACACGTGTGTATTCTGGCTCAAGACGA
TCTTTTTTCTGTCGGATAAAGAGTTGTAAAATCTCTGTCAAAGAAGAGCATGGATGCTTA
CCCAACTCAAAGAAGAAAGAGCACAGAAAATTCTATACTATCCATTGCACTGGTTACTTG
AGAAGCTGGCCTCCAAATATTGTTGGAATGGAAGAAGAAAGGAACAGTAAGAAAGACAAC


AGTAATTTTACCTGCCTTGTGGCCATTGGAAGATTACAGCCATATATTGTTCCACAGAAC
AGTGGAGAGATTAATGTGAAACCAACTGAATTTATAACCCGGTTTGCAGTGAATGGAAAA
TTTGTCTATGTAGATCAAAGGGCAACAGCGATTTTAGGATATCTGCCTCAGGAACTTTTG
GGAACTTCTTGTTATGAATATTTTCATCAAGATGACCACAATAATTTGACTGACAAGCAC
AAAGCAGTTCTACAGAGTAAGGAGAAAATACTTACAGATTCCTACAAATTCAGAGCAAAA
GATGGCTCTTTTGTAACTTTAAAAAGCCAATGGTTTAGTTTCACAAATCCTTGGACAAAA
GAACTGGAATATATTGTATCTGTCAACACTTTAGTTTTGGGACATAGTGAGCCTGGAGAA
GCATCATTTTTACCTTGTAGCTCTCAATCATCAGAAGAATCCTCTAGACAGTCCTGTATG
AGTGTACCTGGAATGTCTACTGGAACAGTACTTGGTGCTGGTAGTATTGGAACAGATATT
GCAAATGAAATTCTGGATTTACAGAGGTTACAGTCTTCTTCATACCTTGATGATTCGAGT
CCAACAGGTTTAATGAAAGATACTCATACTGTAAACTGCAGGAGTATGTCAAATAAGGAG
TTGTTTCCACCAAGTCCTTCTGAAATGGGGGAGCTAGAGGCTACCAGGCAAAACCAGAGT
ACTGTTGCTGTCCACAGCCATGAGCCACTCCTCAGTGATGGTGCACAGTTGGATTTCGAT
GCCCTATGTGACAATGATGACACAGCCATGGCTGCATTTATGAATTACTTAGAAGCAGAG
GGGGGCCTGGGAGACCCTGGGGACTTCAGTGACATCCAGTGGACCCTCTAG

The sequence of cDNA PER1 comprises SEQ ID No.3
>ENST00000317276.9 PER1-201 cdna:protein_coding

```
ATGAGTGGCCCCCTAGAAGGGGCTGATGGGGGAGGGGACCCCAGGCCTGGGGAATCATTT
TGTCCTGGGGGCGTCCCATCCCCTGGGCCCCCACAGCACCGGCCTTGCCCAGGCCCCAGC
CTGGCCGATGACACCGATGCCAACAGCAATGGTTCAAGTGGCAATGAGTCCAACGGGCAT
GAGTCTAGAGGCGCATCTCAGCGGAGCTCACACAGCTCCTCCTCAGGCAACGGCAAGGAC
TCAGCCCTGCTGGAGACCACTGAGAGCAGCAAGAGCACAAACTCTCAGAGCCCATCCCCA
CCCAGCAGTTCCATTGCCTACAGCCTCCTGAGTGCCAGCTCAGAGCAGGACAACCCGTCC
ACCAGTGGCTGCAGCAGTGAACAGTCAGCCCGGGCAAGGACTCAGAAGGAACTCATGACA
GCACTTCGAGAGCTCAAGCTTCGACTGCCGCCAGAGCGCCGGGGGCAAGGGCCGCTCTGGG
ACCCTGGCCACGCTGCAGTACGCACTGGCCTGTGTCAAGCAGGTGCAGGCCAACCAGGAA
TACTACCAGCAGTGGAGCCTGGAGGAGGGCGAGCCTTGCTCCATGGACATGTCCACCTAT
ACCCTGGAGGAGCTGGAGCACATCACGTCTGAGTACACACTTCAGAACCAGGATACCTTC
TCAGTGGCTGTCTCCTTCCTGACGGGCCGAATCGTCTACATTTCGGAGCAGGCAGCCGTC
CTGCTGCGTTGCAAGCGGGACGTGTTCCGGGGTACCCGCTTCTCTGAGCTCCTGGCTCCC
CAGGATGTGGGAGTCTTCTATGGTTCCACTGCTCCATCTCGCCTGCCCACCTGGGGCACA
GGGGCCTCAGCAGGTTCAGGCCTCAGGGACTTTACCCAGGAGAAGTCCGTCTTCTGCCGT
ATCAGAGGAGGTCCTGACCGGGATCCAGGGCCTCGGTACCAGCCATTCCGCCTAACCCCG
TATGTGACCAAGATCCGGGTCTCAGATGGGGCCCCTGCACAGCCGTGCTGCCTGCTGATT
GCAGAGCGCATCCATTCGGGTTACGAAGCTCCCCGGATACCCCCTGACAAGAGGATTTTC
ACTACGCGGCACACACCCAGCTGCCTCTTCCAGGATGTGGATGAAAGGGCTGCCCCCCTG
CTGGGCTACCTGCCCCAGGACCTCCTGGGGGCCCCAGTGCTCCTGTTCCTGCATCCTGAG
GACCGACCCCTCATGCTGGCTATCCACAAGAAGATTCTGCAGTTGGCGGGCCAGCCCTTT
GACCACTCCCCTATCCGCTTCTGTGCCCGCAACGGGGAGTATGTCACCATGGACACCAGC
TGGGCTGGCTTTGTGCACCCCTGGAGCCGCAAGGTAGCCTTCGTGTTGGGCCGCCACAAA
GTACGCACGGCCCCCCTGAATGAGGACGTGTTCACTCCCCCGGCCCCCAGCCCAGCTCCC
TCCCTGGACACTGATATCCAGGAGCTGTCAGAGCAGATCCACCGGCTGCTGCTGCAGCCC
```

GTCCACAGCCCCAGCCCCACGGGACTCTGTGGAGTCGGCGCCGTGACATCCCCAGGCCCT
CTCCACAGCCCTGGGTCCTCCAGTGATAGCAACGGGGGTGATGCAGAGGGGCCTGGGCCT
CCTGCGCCAGTGACTTTCCAGCAGATCTGTAAGGATGTGCATCTGGTGAAGCACCAGGGC
CAGCAGCTTTTTATTGAGTCTCGGGCCCGGCCTCAGTCCCGGCCCCGCCTCCCTGCTACA
GGCACGTTCAAGGCCAAGGCCCTTCCCTGCCAATCCCCAGACCCAGAGCTGGAGGCGGGT
TCTGCTCCCGTCCAGGCCCCACTAGCCTTGGTCCCTGAGGAGGCCGAGAGGAAAGAAGCC
TCCAGCTGCTCCTACCAGCAGATCAACTGCCTGGACAGCATCCTCAGGTACCTGGAGAGC
TGCAACCTCCCCAGCACCACTAAGCGTAAATGTGCCTCCTCCTCCTATACCACCTCC
TCAGCCTCTGACGACGACAGGCAGAGGACAGGTCCAGTCTCTGTGGGGACCAAGAAAGAT
CCGCCGTCAGCAGCGCTGTCTGGGGAGGGGGCCACCCCACGGAAGGAGCCAGTGGTGGGA
GGCACCCTGAGCCCGCTCGCCCTGGCCAATAAGGCGGAGAGTGTGGTGTCCGTCACCAGT
CAGTGTAGCTTCAGCTCCACCATCGTCCATGTGGGAGACAAGAAGCCCCCGGAGTCGGAC
ATCATCATGATGGAGGACCTGCCTGGCCTAGCCCCAGGCCCAGCCCCCAGCCCAGCCCCC
AGCCCCACAGTAGCCCCTGACCAGCCCCAGACGCCTACCGTCCAGTGGGGCTGACCAAG
GCCGTGCTGTCCCTGCACACACAGAAGGAAGAGCAAGCCTTCCTCAGCCGCTTCCGAGAC
CTGGGCAGGCTGCGTGGACTCGACAGCTCTTCCACAGCTCCCTCAGCCCTTGGCGAGCGA
GGCTGCCACCACGGCCCCGCACCCCCAAGCCGCCGACACCACTGCCGATCCAAAGCCAAG
CGCTCACGCCACCACCAGAACCCTCGGGCTGAAGCGCCCTGCTATGTCTCACACCCCTCA
CCCGTGCCACCCTCCACCCCCTGGCCCACCCCACCAGCCACTACCCCCTTCCCAGCGGTT
GTCCAGCCCTACCCTCTCCCAGTGTTCTCTCCTCGAGGAGGCCCCCAGCCTCTTCCCCCT
GCTCCCACATCTGTGCCCCCAGCTGCTTTCCCCGCCCCTTTGGTGACCCCAATGGTGGCC
TTGGTGCTCCCTAACTATCGTTCCCAACCCCATCCAGCTATCCTTATGGGGCACTCCAG
ACCCCTGCTGAAGGGCCTCCCACTCCTGCCTCGCACTCCCCTTCTCCATCCTTGCCCGCC
CTCGCCCCGAGTCCTCCTCACCGCCCGGACTCTCCACTGTTCAACTCGAGATGCAGCTCT
CCACTCCAGCTCAATCTGCTGCAGCTGGAGGAGCTCCCCCGTGCTGAGGGGGCTGCTGTT
GCAGGAGGCCCTGGGAGCAGTGCCGGGCCCCCACCTCCCAGTGCGGAGGCTGCTGAGCCA
GAGGCCAGACTGGCGGAGGTCACTGAGTCCTCCAATCAGGACGCACTTTCCGGCTCCAGT
GACCTGCTCGAACTTCTGCTGCAAGAGGACTCGCGCTCCGGCACAGGCTCCGCAGCCTCG
GGCTCCTTGGGCTCTGGCTTGGGCTCTGGGTCTGGTTCAGGCTCCCATGAAGGGGGCAGC
ACCTCAGCCAGCATCACTCGCAGCAGCCAGAGCAGCCACACAAGCAAATACTTTGGCAGC
ATCGACTCTTCCGAGGCTGAGGCTGGGGCTGCTCGGGGCGGGGCTGAGCCTGGGGACCAG
GTGATTAAGTACGTGCTCCAGGATCCCATTTGGCTGCTCATGGCCAATGCTGACCAGCGC
GTCATGATGACCTACCAGGTGCCCTCCAGGGACATGACCTCTGTGCTGAAGCAGGATCGG
GAGCGGCTCCGAGCCATGCAGAAGCAGCAGCCTCGGTTTTCTGAGGACCAGCGGCGGGAA
CTGGGTGCTGTGCACTCCTGGGTCCGGAAGGGCCAACTGCCTCGGGCTCTTGATGTGATG
GCCTGTGTGGACTGTGGGAGCAGCACCCAAGATCCTGGTCACCCTGATGACCCACTCTTC
TCAGAGCTGGATGGACTGGGGCTGGAGCCCATGGAAGAGGGTGGAGGCGAGCAGGGCAGC
AGCGGTGGCGGCAGTGGTGAGGGAGAGGGCTGCGAGGAGGCCCAAGGCGGGGCCAAGGCT
TCAAGCTCTCAGGACTTGGCTATGGAGGAGGAGGAAGAAGGCAGGAGCTCATCCAGTCCA
GCCTTACCTACAGCAGGAAACTGCACCAGCTAG

The sequence of PER2 cDNA comprises SEQ ID No.4:
>ENST00000254657.8 PER2-201 cdna:protein_coding

ATGAATGGATACGCGGAATTTCCGCCCAGCCCCAGTAACCCCACCAAGGAGCCCGTGGAG
CCCCAGCCCAGCCAGGTCCCACTGCAGGAAGATGTGGACATGAGCAGTGGCTCCAGTGGA
CATGAGACCAACGAAAACTGCTCCACGGGGCGGGACTCGCAGGGCAGTGACTGTGACGAC
AGTGGGAAGGAGCTGGGGATGCTGGTGGAGCCACCGGATGCCCGCCAGAGTCCAGATACC
TTTAGCCTGATGATGGCAAAATCTGAACACAACCCATCTACAAGTGGCTGCAGTAGCGAC
CAGTCTTCGAAAGTGGACACACACAAAGAACTGATAAAAACACTAAAGGAGCTGAAGGTC
CACCTCCCTGCAGACAAGAAGGCCAAGGGCAAGGCCAGTACGCTGGCCACCTTGAAGTAC
GCCCTCAGGAGCGTGAAGCAGGTGAAAGCCAATGAAGAGTATTACCAGCTGCTGATGTCC
AGCGAGGGTCACCCCTGTGGAGCAGACGTGCCCTCCTACACCGTGGAGGAGATGGAGAGC
GTTACCTCTGAGCACATTGTGAAGAATGCCGATATGTTTGCGGTGGCCGTGTCCCTGGTG
TCTGGGAAGATCCTGTACATCTCTGACCAGGTTGCATCCATATTTCACTGTAAAAGAGAT
GCCTTCAGCGATGCCAAGTTTGTGGAGTTCCTGGCGCCTCACGATGTGGGCGTGTTCCAC
AGTTTCACCTCCCCGTACAAGCTTCCCTTGTGGAGCATGTGCAGTGGAGCAGATTCTTTT
ACTCAAGAATGCATGGAGGAGAAATCTTTCTTTTGCCGTGTCAGTGTCCGGAAAAGCCAC
GAGAATGAAATCCGCTACCACCCCTTCCGCATGACGCCCTACCTGGTCAAGGTGCGGGAC
CAACAAGGTGCTGAGAGTCAGCTTTGCTGCCTTCTGCTGGCAGAGAGAGTGCACTCTGGT
TATGAAGCCCCTAGAATTCCTCCTGAAAAGAGAATTTTTACAACCACCCATACACCAAAT
TGTTTGTTCCAGGATGTGGATGAAAGGGCGGTCCCTCTCCTGGGCTACCTACCTCAGGAC
CTGATTGAAACCCCAGTGCTCGTGCAGCTCCACCCTAGTGACAGGCCCTTGATGCTGGCC
ATCCACAAAAAGATCCTGCAGTCAGGCGGGCAGCCTTTCGACTATTCTCCCATTCGGTTT
CGCGCCCGGAACGGAGAGTACATCACGTTGGACACCAGCTGGTCCAGCTTCATCAACCCA
TGGAGCAGGAAAATCTCCTTCATCATTGGGAGGCACAAAGTCAGGGTGGGCCCTTTGAAT
GAGGACGTGTTTGCAGCCCACCCCTGCACAGAGGAGAAGGCCCTGCACCCCAGCATTCAG
GAGCTCACAGAGCAGATCCACCGGCTCCTGCTGCAGCCCGTCCCCCACAGCGGCTCCAGT
GGCTACGGGAGTCTGGGCAGCAACGGGTCCCACGAGCACCTTATGAGCCAGACCTCCTCC
AGCGACAGCAACGGCCATGAGGACTCACGCCGGAGGAGAGCCGAAATTTGTAAAAATGGT
AACAAGACCAAAAATAGAAGTCATTATTCTCATGAATCTGGAGAACAAAAGAAAAAATCC
GTTACAGAAATGCAAACTAATCCCCCAGCTGAGAAGAAAGCTGTCCCTGCCATGGAAAAG
GACAGCCTGGGGGTCAGCTTCCCCGAGGAGTTGGCCTGCAAGAACCAGCCCACCTGCTCC
TACCAGCAGATCAGCTGCTTGGACAGCGTCATCAGGTACTTGGAGAGCTGCAATGAGGCT
GCCACCCTGAAGAGGAAATGCGAGTTCCCAGCAAACGTCCCAGCGCTAAGGTCCAGTGAT
AAGCGGAAGGCCACAGTCAGCCCAGGGCCACACGCTGGAGAGGCAGAGCCGCCCTCCAGG
GTGAACAGCCGCACGGGAGTAGGTACGCACCTGACCTCGCTGGCACTGCCGGGCAAGGCA
GAGAGTGTGGCGTCGCTCACCAGCCAGTGCAGCTACAGCAGCACCATCGTCCATGTGGGA
GACAAGAAGCCGCAGCCGGAGTTAGAGATGGTGGAAGATGCTGCGAGTGGGCCAGAATCC
CTGGACTGCCTGGCGGGCCCTGCCCTGGCCTGTGGTCTCAGCCAAGAGAAGGAGCCCTTC
AAGAAGCTGGGCCTCACCAAGGAGGTACTCGCTGCACACACACAGAAGGAGGAGCAGAGC
TTCCTGCAGAAGTTCAAAGAAATAAGAAAACTCAGCATTTTCCAGTCCCACTGCCATTAC
TACTTGCAAGAAAGATCCAAGGGGCAGCCAAGTGAACGAACTGCCCCTGGACTAAGAAAT
ACTTCCGGAATAGATTCACCTTGGAAAAAAACAGGAAAGAACAGAAAATTGAAGTCCAAG
CGGGTCAAACCTCGAGACTCATCTGAGAGCACCGGATCTGGGGGGCCCGTGTCCGCCCGG
CCCCCGCTGGTGGGCTTGAACGCCACAGCCTGGTCACCCTCAGACACGTCCCAGTCCAGC
TGCCCAGCCGTGCCCTTTCCCGCCCCAGTGCCAGCAGCTTATTCACTGCCCGTGTTTCCA
GCGCCAGGGACTGTGGCAGCACCCCCGGCACCTCCCCACGCCAGCTTCACAGTGCCTGCT
GTGCCCGTGGACCTCCAGCACCAGTTTGCAGTCCAGCCCCCACCTTTCCCTGCCCCTTTG

GCGCCTGTCATGGCATTCATGCTACCCAGTTATTCCTTCCCCTCGGGGACCCCAAACCTG
CCCCAGGCCTTCTTCCCCAGCCAGCCTCAGTTTCCGAGCCACCCCACACTCACATCCGAG
ATGGCCTCTGCCTCACAGCCTGAGTTCCCCAGCCGGACCTCGATCCCCAGACAGCCATGT
GCTTGTCCAGCCACCCGGGCCACCCCACCATCGGCCATGGGTAGGGCCTCCCCACCGCTC
TTTCAGTCCCGCAGCAGCTCGCCCCTGCAGCTCAACCTGCTGCAGCTGGAGGAAGCCCCT
GAGGGTGGCACTGGAGCCATGGGGACCACAGGGGCCACAGAGACAGCAGCTGTAGGGGCG
GACTGCAAACCTGGCACTTCTCGGGACCAGCAGCCGAAGGCGCCTCTGACCCGTGATGAA
CCCTCAGACACACAGAACAGTGACGCCCTTTCCACGTCAAGCGGCCTCCTAAACCTCCTG
CTGAATGAGGACCTCTGCTCAGCCTCGGGCTCTGCTGCTTCGGAGTCTCTGGGCTCCGGC
TCACTGGGCTGCGACGCCTCCCCGAGTGGGGCAGGCAGTAGTGACACAAGTCATACCAGC
AAATATTTTGGAAGCATTGACTCCTCAGAGAATAATCACAAAGCAAAAATGAACACTGGT
ATGGAAGAAAGTGAGCATTTCATTAAGTGCGTCCTGCAGGATCCATCTGGCTGCTGATG
GCAGATGCGGACAGCAGCGTCATGATGACGTACCAGCTGCCTTCCCGAAATTTAGAAGCG
GTTTTGAAGGAGGACAGAGAGAAGCTGAAGCTCCTACAGAAACTCCAGCCCAGGTTCACG
GAGAGTCAGAAGCAGGAGCTGCGCGAGGTCCACCAGTGGATGCAGACGGGCGGCCTGCCC
GCAGCCATCGACGTGGCAGAATGTGTTTACTGTGAAAACAAGGAAAAAGGTAATATTTGC
ATACCATATGAGGAAGATATTCCTTCTCTGGGACTCAGCGAAGTGTCGGACACCAAAGAA
GACGAAAATGGATCCCCCTTGAATCACAGGATCGAAGAGCAGACGTAA

The sequence of PER3 cDNA comprises SEQ ID No.5:
>ENST00000613533.4 PER3-208 cdna:protein_coding

ATGCCCCGCGGGGAAGCTCCTGGCCCCGGGAGACGGGGGGGCTAAGGACGAGGCCCTGGGC
GAAGAATCGGGGGAGCGGTGGAGCCCCGAGTTCCATCTGCAGAGGAAATTGGCGGACAGC
AGCCACAGTGAACAGCAAGATCGAAACAGAGTTTCTGAAGAACTTATCATGGTTGTCCAA
GAAATGAAAAAATACTTCCCCTCGGAGAGACGCAATAAACCAAGCACTCTAGATGCCCTC
AACTATGCTCTCCGCTGTGTCCACAGCGTTCAAGCAAACAGTGAGTTTTTCCAGATTCTC
AGTCAGAATGGAGCACCTCAGGCAGATGTGAGCATGTACAGTCTTGAGGAGCTGGCCACT
ATCGCTTCAGAACACACTTCCAAAAACACAGATACCTTTGTGGCAGTATTTTCATTTCTG
TCTGGAAGGTTAGTGCACATTTCTGAACAGGCTGCTTTGATCCTGAATCGTAAGAAAGAT
GTCCTGGCGTCTTCTCACTTTGTTGACCTGCTTGCACCTCAAGACATGAGGGTATTCTAC
GCGCACACTGCCAGAGCTCAGCTTCCTTTCTGGAACAACTGGACCCAAAGAGCAGCTGCA
CGGTATGAATGTGCTCCGGTGAAACCTTTTTTCTGCAGGATCCGTGGAGGTGAAGACAGA
AAGCAAGAGAAGTGTCACTCCCCATTCCGGATCATCCCCTATCTGATTCATGTACATCAC
CCTGCCCAGCCAGAATTGGAATCGGAACCTTGCTGTCTCACTGTGGTTGAAAAGATTCAC
TCTGGTTATGAAGCTCCTCGGATCCCAGTGAATAAAAGAATCTTCACCACCACACACACC
CCAGGGTGTGTTTTTCTTGAAGTAGATGAAAAAGCAGTGCCTTTGCTGGGTTACCTACCT
CAGGACCTGATTGGAACATCGATCCTAAGCTACCTGCACCCTGAAGATCGTTCTCTGATG
GTTGCCATACACCAAAAAGTTTTGAAGTATGCAGGGCATCCTCCCTTTGAACATTCTCCC
ATTCGATTTTGTACTCAAAACGGAGACTACATCATACTGGATTCCAGTTGGTCCAGCTTT
GTGAATCCCTGGAGCCGGAAGATTTCTTTCATCATTGGTCGGCATAAAGTTCGAACGAGC
CCACTAAATGAGGATGTTTTTGCTACCAAAATTAAAAAGATGAACGATAATGACAAAGAC
ATAACAGAATTACAAGAACAAATTTACAAACTTCTCTTACAGCCAGTTCACGTGAGCGTG
TCCAGCGGCTACGGGAGCCTGGGGAGCAGCGGGTCGCAGGAGCAGCTTGTCAGCATCGCC
TCCTCCAGTGAGGCCAGTGGGCACCGTGTGGAGGAGACGAAGGCGGAGCAGATGACCTTG

CAGCAGGTCTATGCCAGTGTGAACAAAATTAAAAATCTGGGTCAGCAGCTCTACATTGAG
TCAATGACCAAATCATCATTCAAGCCAGTGACGGGGACACGCACAGAACCGAATGGTGGT
GGTGAGTCAGCGAATGGTGGTGGTGAATGTAAGACCTTTACTTCCTTCCACCAAACACTG
AAAAACAATAGTGTGTACACTGAGCCCTGTGAGGATTTGAGGAACGATGAGCACAGCCCA
TCCTATCAACAGATCAACTGTATCGACAGTGTCATCAGATACCTGAAGAGCTACAACATT
CCAGCTTTGAAAAGAAAGTGTATCTCCTGTACAAATACAACTTCTTCCTCCTCAGAAGAA
GACAAACAGAACCACAAGGCAGATGATGTCCAAGCCTTACAAGCTGGTTTGCAAATCCCA
GCCATACCTAAATCAGAAATGCCAACAAATGGACGGTCCATAGACACAGGAGGAGGAGCT
CCACAGATCCTGTCCACGGCGATGCTGAGCTTGGGGTCGGGCATAAGCCAATGCGGTTAC
AGCAGCACCATTGTCCATGTCCCACCCCCAGAGACAGCCAGGGATGCTACCCTCTTCTGT
GAGCCCTGGACCCTGAACATGCAGCCAGCCCCTTTGACCTCGGAAGAATTTAAACACGTG
GGGCTCACAGCGGCTGTTCTGTCAGCGCACACCCAGAAGGAAGAGCAGAATTATGTTGAT
AAATTCCGAGAAAAGATCCTGTCATCACCCTACAGCTCCTATCTTCAGCAAGAAAGCAGG
AGCAAAGCTAAATATTCATATTTTCAAGGAGATTCTACTTCCAAGCAGACGCGGTCGGCC
GGCTGCAGGAAAGGGAAGCACAAGCGGAAGAAGCTGCCGGAGCCGCCAGACAGCAGCAGC
TCGAACACCGGCTCTGGTCCCCGCAGGGGAGCGCATCAGAACGCACAGCCCTGCTGCCCC
TCCGCGGCCTCCTCTCCGCACACCTCGAGCCCGACCTTCCCACCTGCCGCCATGGTGCCC
AGCCAGGCCCCTTACCTCGTCCCAGCTTTTCCCCTCCCAGCCGCGACCTCACCCGGAAGA
GAATACGCAGCCCCCGGAACTGCACCGGAAGGCCTGCATGGGCTGCCCTTGTCCGAGGGC
TTGCAGCCTTACCCAGCTTTCCCTTTTCCTTACTTGGATACTTTTATGACCGTTTTCCTG
CCTGACCCCCCTGTCTGTCCTCTGTTGTCGCCATCGTTTTTGCCATGTCCATTCCTGGGG
GCGACAGCCTCTTCTGCGATATCACCCTCAATGTCGTCAGCAATGAGTCCAACTCTGGAC
CCACCCCCTTCAGTCACCAGCCAAAGGAGAGAGGAGGAAAAGTGGGAGGCACAAAGCGAG
GGGCACCCGTTCATTACTTCGAGAAGCAGCTCACCCTTGCAGTTAAACTTACTTCAGGAA
GAGATGCCCAGACCCTCTGAATCTCCAGATCAGATGAGAAGGAACACGTGCCCACAAACT
GAGTATCAGTGTGTTACAGGCAACAATGGCAGTGAGAGCAGTCCTGCTACTACCGGTGCA
CTGTCCACGGGGTCACCTCCCAGGGAGAATCCATCCCATCCTACTGCCAGCGCTCTGTCC
ACAGGATCGCCTCCCATGAAGAATCCATCCCATCCTACTGCCAGCGCTCTGTCCACAGGA
TCGCCTCCCATGAAGAATCCATCCCATCCTACTGCCAGCACACTGTCCATGGGATTGCCT
CCCAGCAGGACTCCATCCCATCCTACTGCCACTGTTCTGTCCACGGGGTCACCTCCCAGC
GAATCCCCATCCAGAACTGGTTCAGCAGCATCAGGAAGCAGCGACAGCAGTATATACCTT
ACTAGTAGTGTTTATTCTTCTAAAATCTCCCAAAATGGGCAGCAATCTCAGGACGTACAG
AAAAAAGAAACATTTCCTAATGTCGCCGAAGAGCCCATCTGGAGAATGATACGGCAGACA
CCTGAGCGCATTCTCATGACATACCAGGTACCTGAGAGGGTTAAAGAAGTTGTACTAAAA
GAAGACCTGGAAAAGCTAGAAGTATGAGGCAGCAGCAGCCCCAGTTTTCTCATGGGCAA
AAGGAGGAGCTGGCTAAGGTGTATAATTGGATTCAAAGCCAGACTGTCACTCAAGAAATC
GACATTCAAGCCTGTGTCACTTGTGAAAATGAAGATTCAGCTGATGGTGCGGCCACATCC
TGTGGTCAGGTTCTGGTAGAAGACAGCTGTTGA

The sequence of cDNA CLOCK comprises SEQ ID No.6
>ENST00000513440.6 CLOCK-211 cdna:protein_coding

ATGTTGTTTACCGTAAGCTGTAGTAAAATGAGCTCGATTGTTGACAGAGATGACAGTAGT
ATTTTTGATGGGTTGGTGGAAGAAGATGACAAGGACAAAGCGAAAAGAGTATCTAGAAAC
AAATCTGAAAAGAAACGTAGAGATCAATTTAATGTTCTCATTAAAGAACTGGGATCCATG

CTTCCTGGTAATGCTAGAAAGATGGACAAATCTACTGTTCTGCAGAAAAGCATTGATTTT
TTACGAAAACATAAAGAAATCACTGCACAGTCAGATGCTAGTGAAATTCGACAGGACTGG
AAACCTACATTCCTTAGTAATGAAGAGTTTACACAATTAATGTTAGAGGCTCTTGATGGT
TTTTTTTTAGCAATCATGACAGATGGAAGCATAATATATGTGTCTGAGAGTGTAACTTCA
TTACTTGAACATTTACCATCTGATCTTGTGGATCAAAGTATATTTAATTTTATCCCAGAA
GGGGAACATTCAGAGGTTTATAAAATACTCTCTACTCATCTGCTGGAAAGTGATTCATTA
ACCCCAGAATATTTAAAATCAAAAAATCAGTTAGAATTCTGTTGTCACATGCTGCGAGGA
ACAATAGACCCAAAGGAGCCATCTACCTATGAATATGTAAAATTTATAGGAAATTTCAAA
TCTTTAAACAGTGTATCCTCTTCAGCACACAATGGTTTTGAAGGAACTATACAACGCACA
CATAGGCCATCTTATGAAGATAGAGTTTGTTTTGTAGCTACTGTCAGGTTAGCTACACCT
CAGTTCATCAAGGAAATGTGCACTGTTGAAGAACCCAATGAAGAGTTTACATCTAGACAT
AGTTTAGAATGGAAGTTTCTGTTTCTAGATCACAGGGCACCACCCATAATAGGGTATTTG
CCATTTGAAGTTCTGGGAACATCAGGCTATGATTACTATCATGTGGATGACCTAGAAAAT
TTGGCAAAATGTCATGAGCACTTAATGCAATATGGGAAAGGCAAATCATGTTATTATAGG
TTCCTGACTAAGGGGCAACAGTGGATTTGGCTTCAGACTCATTATTATATCACTTACCAT
CAGTGGAATTCAAGGCCAGAGTTTATTGTTTGTACTCACACTGTAGTAAGTTATGCAGAA
GTTAGGGCTGAAAGACGACGAGAACTTGGCATTGAAGAGTCTCTTCCTGAGACAGCTGCT
GACAAAAGCCAAGATTCTGGGTCAGATAATCGTATAAACACAGTCAGTCTCAAGGAAGCA
TTGGAAAGGTTTGATCACAGCCCAACCCCTTCTGCCTCTTCTCGGAGTTCAAGAAAATCA
TCTCACACGGCCGTCTCAGACCCTTCCTCAACACCAACCAAGATCCCGACGGATACGAGC
ACTCCACCCAGGCAGCATTTACCAGCTCATGAGAAGATGGTGCAAAGAAGGTCATCATTT
AGTAGTCAGTCCATAAATTCCCAGTCTGTTGGTTCATCATTAACACAGCCAGTGATGTCT
CAAGCTACAAATTTACCAATTCCACAAGGCATGTCCCAGTTTCAGTTTTCAGCTCAATTA
GGAGCCATGCAACATCTGAAAGACCAATTGGAACAACGGACACGCATGATAGAAGCAAAT
ATTCATCGGCAACAAGAAGAACTAAGAAAAATTCAAGAACAACTTCAGATGGTCCATGGT
CAGGGGCTGCAGATGTTTTTGCAACAATCAAATCCTGGGTTGAATTTTGGTTCCGTTCAA
CTTTCTTCTGGAAATTCATCTAATATCCAGCAACTTGCACCTATAAATATGCAAGGCCAA
GTTGTTCCTACTAACCAGATTCAAAGTGGAATGAATACTGGACACATTGGCACAACTCAG
CACATGATACAACAACAGACTTTACAGAGTACATCAACTCAGAGTCAACAAAATGTACTG
AGTGGGCACAGTCAGCAAACATCTCTACCCAGTCAGACACAGAGCACTCTTACAGCCCCA
CTGTATAACACTATGGTGATTTCTCAGCCTGCAGCCGGAAGCATGGTCCAGATTCCATCT
AGTATGCCACAAAACAGCACCCAGAGTGCTGCAGTAACTACATTCACTCAGGACAGGCAG
ATAAGATTTTCTCAAGGTCAACAACTTGTGACCAAATTAGTGACTGCTCCTGTAGCTTGT
GGGGCAGTCATGGTACCTAGTACTATGCTTATGGGCCAGGTGGTGACTGCATATCCTACT
TTTGCTACACAACAGCAACAGTCACAGACATTGTCAGTAACGCAGCAGCAGCAGCAGCAG
AGCTCCCAGGAGCAGCAGCTCACTTCAGTTCAGCAACCATCTCAGGCTCAGCTGACCCAG
CCACCGCAACAATTTTTACAGACTTCTAGGTTGCTCCATGGGAATCCCTCAACTCAACTC
ATTCTCTCTGCTGCATTTCCTCTACAACAGAGCACCTTCCCTCAGTCACATCACCAGCAA
CATCAGTCTCAGCAACAGCAGCAACTCAGCCGGCACAGGACTGACAGCTTGCCCGACCCT
TCCAAGGTTCAACCACAGTAG

The sequence of cDNA NPAS2 comprises SEQ ID No.7
>ENST00000335681.10 NPAS2-201 cdna:protein_coding

ATGGATGAAGATGAGAAAGACAGAGCCAAGAGAGCTTCTCGAAACAAGTCTGAGAAGAAG

CGTCGGGACCAGTTCAATGTTCTCATCAAGAGCTCAGTTCCATGCTCCCTGGCAACACG
CGGAAAATGGACAAAACCACCGTGTTGGAAAAGGTCATCGGATTTTTGCAGAAACACAAT
GAAGTCTCAGCGCAAACGGAAATCTGTGACATTCAGCAAGACTGGAAGCCTTCATTCCTC
AGTAATGAAGAATTCACCCAGCTGATGTTGGAGGCATTAGATGGCTTCATTATCGCAGTG
ACAACAGACGGCAGCATCATCTATGTCTCTGACAGTATCACGCCTCTCCTTGGGCATTTA
CCGTCGGATGTCATGGATCAGAATTTGTTAAATTTCCTCCCAGAACAAGAACATTCAGAA
GTTTATAAAATCCTTTCTTCCCATATGCTTGTGACGGATTCCCCCTCCCCAGAATACTTA
AAATCTGACAGCGATTTAGAGTTTTATTGCCATCTTCTCAGAGGCAGCTTGAACCCAAAG
GAATTTCCAACTTATGAATACATAAAATTTGTAGGAAATTTTCGCTCTTACAACAATGTG
CCTAGCCCCTCCTGTAATGGTTTTGACAACACCCTTTCAAGACCTTGCCGGGTGCCACTA
GGAAAGGAGGTTTGCTTCATTGCCACCGTTCGTCTGGCAACACCACAATTCTTAAAGGAA
ATGTGCATAGTTGACGAACCTTTAGAGGAATTCACTTCAAGGCATAGCTTGGAATGGAAA
TTTTTATTTCTGGATCACAGAGCACCTCCAATCATAGGATACCTGCCTTTTGAAGTGCTG
GGAACCTCAGGCTATGACTACTACCACATTGATGACCTGGAGCTCCTGGCCAGGTGTCAC
CAGCACCTGATGCAGTTTGGCAAAGGGAAGTCGTGTTGCTACCGGTTTCTGACCAAAGGT
CAGCAGTGGATCTGGCTGCAGACTCACTACTACATCACCTACCATCAGTGGAACTCCAAG
CCCGAGTTCATCGTGTGCACACTCGGTGGTCAGTTACGCAGATGTCCGGGTGGAAAGG
AGGCAGGAGCTGGCTCTGGAAGACCCGCCATCCGAGGCCCTCCACTCCTCAGCACTAAAG
GACAAGGGCTCAAGCCTGGAACCTCGGCAGCACTTTAACACACTCGACGTGGGTGCCTCG
GGCCTTAATACCAGTCATTCGCCATCGGCGTCCTCAAGAAGTTCCCACAAATCCTCGCAC
ACAGCCATGTCAGAACCCACCTCCACTCCCACCAAGCTGATGGCAGAGGCCAGCACCCCG
GCTTTGCCAAGATCAGCCACCCTGCCCCAAGAGTTACCTGTCCCCGGGCTCAGCCAGGCA
GCCACCATGCCGGCCCCTCTGCCTTCCCCATCGTCCTGCGACCTCACACAGCAGCTCCTG
CCTCAGACCGTTCTGCAGAGCACGCCCGCTCCCATGGCACAGTTTTCGGCACAGTTCAGC
ATGTTCCAGACCATCAAAGACCAGCTAGAGCAGCGGACGCGGATCCTGCAGGCCAATATC
CGGTGGCAACAGGAAGAGCTCCACAAGATCCAGGAGCAGCTCTGCCTGGTCCAGGACTCC
AACGTCCAGATGTTCCTGCAGCAGCCAGCTGTATCCCTGAGCTTCAGCAGCACCCAGCGA
CCTGAGGCTCAGCAGCAGCTACAGCAAAGGTCAGCTGCAGTGACTCAGCCCCAGCTCGGG
GCGGGCCCCCAACTTCCAGGGCAGATCTCCTCTGCCCAGGTCACAAGCCAGCACCTGCTC
AGAGAATCAAGTGTGATATCAACCCAGGGTCCAAAGCCAATGAGAAGCTCACAGCTAATG
CAGAGCAGCGGCCGCTCTGGAAGCAGCCTAGTGTCCCCGTTCAGCAGCGCCACAGCTGCG
CTCCCGCCAAGTCTGAATCTGACCACACCTGCTTCCACCTCCCAGGATGCCAGCCAGTGC
CAGCCCAGCCCAGACTTCAGCCATGATCGGCAGCTCAGGCTGTTGCTGAGCCAGCCCATC
CAGCCCATGATGCCCGGGTCCTGTGACGCAAGGCAGCCCTCGGAAGTCAGCAGGACGGGA
CGGCAAGTCAAGTACGCCCAGAGCCAGACCGTGTTTCAAAATCCAGACGCACACCCCGCC
AACAGCAGCAGCGCCCCGATGCCCGTCCTGCTGATGGGGCAGGCGGTGCTCCACCCCAGC
TTCCCTGCCTCCCAACCATCGCCCCTGCAGCCTGCACAGGCCCGGCAGCAGCCACCGCAG
CACTACCTGCAGGTACAGGCACCAACCTCTTTGCACAGTGAGCAGCAGGACTCGCTACTT
CTCTCCACCTACTCACAACAGCCAGGGACCCTGGGCTACCCCCAACCACCCCCAGCACAG
CCCCAGCCCCTACGTCCTCCCCGAAGGGTCAGCAGTCTGTCTGAGTCGTCAGGCCTCCAG
CAGCCGCCCCGATAA

The sequence of cDNA CRY1 comprises SEQ ID No.8
>ENST00000008527.10 CRY1-201 cdna:protein_coding

ATGGGGGTGAACGCCGTGCACTGGTTCCGAAAGGGGCTCCGGCTCCACGACAACCCCGCC
CTGAAGGAGTGCATTCAGGGCGCCGACACCATCCGCTGCGTCTACATCCTGGACCCCTGG
TTCGCCGGCTCCTCCAATGTGGGCATCAACAGGTGGCGATTTTTGCTTCAGTGTCTTGAG
GATCTTGATGCCAATCTACGAAAATTAAACTCCCGTCTGTTTGTGATTCGTGGACAACCA
GCAGATGTGTTTCCCAGGCTTTTCAAGGAATGGAACATTACTAAACTTTCAATTGAGTAT
GATTCTGAGCCCTTTGGAAAGGAACGAGACGCAGCTATTAAGAAACTGGCAACTGAAGCT
GGAGTAGAAGTCATTGTAAGAATTTCACATACATTATATGACCTAGACAAGATCATAGAA
CTCAATGGTGGACAACCGCCTCTAACTTATAAAGATTCCAGACTCTCATCAGCAAAATG
GAACCACTAGAGATACCAGTAGAGACAATTACTTCAGAAGTGATAGAAAAGTGCACAACT
CCTCTGTCTGATGACCATGATGAGAAATATGGAGTCCCTTCACTGGAAGAGCTAGGTTTT
GATACAGATGGCTTATCCTCTGCAGTGTGGCCAGGTGGAGAAACTGAAGCACTTACTCGT
TTGGAAAGGCATTTGGAAAGAAAGCTTGGGTGGCAAATTTTGAAAGACCTCGAATGAAT
GCGAATTCTCTGCTTGCAAGCCCTACTGGACTTAGTCCTTATCTCCGATTTGGTTGTTTG
TCATGTCGACTGTTTTACTTCAAACTAACAGATCTCTACAAAAAGGTAAAGAAGAACAGT
TCCCCTCCCCTTTCCCTTTATGGGCAACTGTTATGGCGTGAATTTTTCTATACAGCAGCA
ACAAATAATCCACGCTTTGATAAAATGGAAGGAAACCCTATCTGTGTTCAGATTCCTTGG
GATAAAAATCCTGAGGCTTTAGCCAAATGGGCGGAAGGCCGGACAGGCTTTCCATGGATT
GATGCCATCATGACACAGCTTCGTCAGGAGGGTTGGATTCATCATCTAGCCAGGCATGCA
GTTGCTTGCTTCCTGACACGAGGGGACCTGTGGATTAGTGGGAAGAAGGAATGAAGGTA
TTTGAAGAATTATTGCTTGATGCAGATTGGAGCATAAATGCTGGAAGTTGGATGTGGCTG
TCTTGTAGTTCCTTTTTTCAACAGTTTTTTCACTGCTATTGCCCTGTTGGTTTTGGTAGG
AGAACAGATCCCAATGGAGACTATATCAGGCGTTATTTGCCTGTCCTAAGAGGCTTCCCT
GCAAAATATATCTATGATCCCTGGAATGCACCAGAAGGTATCCAAAAGGTAGCCAAATGT
TTGATAGGAGTTAATTATCCTAAACCAATGGTGAACCATGCTGAGGCAAGCCGTTTGAAT
ATCGAAAGGATGAAACAGATCTATCAGCAGCTTTCACGATATAGAGGACTAGGTCTTCTG
GCATCAGTACCTTCTAATCCTAATGGGAATGGAGGCTTCATGGGATATTCTGCAGAAAAT
ATCCCAGGTTGTAGCAGCAGTGGAAGTTGCTCTCAAGGGAGTGGTATTTTACACTATGCT
CATGGCGACAGTCAGCAAACTCACCTGTTGAAGCAAGGAAGAAGCTCCATGGGCACTGGT
CTCAGTGGTGGGAAACGTCCTAGTCAGGAAGAGGACACACAGAGTATTGGTCCTAAAGTC
CAGAGACAGAGCACTAATTAG

The sequence of cDNA CRY2 comprises SEQ ID No.9
>ENST00000616623.4 CRY2-212 cdna:protein_coding

ATGGGCGGGGTCCACGTCGCCTACCGGGGCGGAGCGGGGGTGGCTGGAGCAGTCTGGACA
GTCATGGCGGCGACTGTGGCGACGGCGGCAGCTGTGGCCCCGGCGCCAGCGCCCGGCACG
GACAGCGCCTCTTCGGTGCACTGGTTCCGCAAAGGGCTGCGACTCCACGACAACCCGGCG
TTGCTGGCGGCCGTGCGCGGGGCGCGCTGCGTGCGCTGCGTTTACATTCTCGACCCGTGG
TTCGCGGCCTCCTCCTCAGTCGGGATCAACCGATGGAGGTTCCTACTTCAGTCTCTGGAA
GATTTGGACACAAGTTTAAGGAAACTGAACTCCCGCCTGTTTGTAGTCCGGGGACAGCCA
GCCGACGTGTTCCCAAGGCTGTTCAAGGAATGGGGAGTGACCCGCTTGACCTTTGAATAT
GACTCTGAACCCTTTGGGAAGAACGGGATGCAGCCATCATGAAGATGGCCAAGGAGGCT

GGTGTGGAAGTAGTGACGGAGAATTCTCATACCCTCTATGACCTGGACAGGATCATTGAG
CTGAATGGGCAGAAGCCACCCCTTACATACAAGCGCTTTCAGGCCATCATCAGCCGCATG
GAGCTGCCCAAGAAGCCAGTGGGCTTGGTGACCAGCCAGCAGATGGAGAGCTGCAGGGCC
GAGATCCAGGAGAACCACGACGAGACCTACGGCGTGCCCTCCCTGGAGGAGCTGGGGTTC
CCCACTGAAGGACTTGGTCCAGCTGTCTGGCAGGGAGGAGAGACAGAAGCTCTGGCCCGC
CTGGATAAGCACTTGGAACGGAAGGCCTGGGTTGCCAACTATGAGAGACCCGAATGAAC
GCCAACTCCCTCCTGGCCAGCCCCACAGGCCTCAGCCCCTACCTGCGCTTTGGTTGTCTC
TCCTGCCGCCTCTTCTACTACCGCCTGTGGGACCTGTATAAAAAGGTGAAGCGGAACAGC
ACACCTCCCCTCTCCCTATTTGGGCAACTCCTATGGCGAGAGTTCTTCTACACGGCAGCT
ACCAACAACCCCAGGTTTGACCGCATGGAGGGGAACCCCATCTGCATCCAGATCCCCTGG
GACCGCAATCCTGAGGCCCTGGCCAAGTGGGCTGAGGGCAAGACAGGCTTCCCTTGGATT
GATGCCATCATGACCCAACTGAGGCAGGAGGGCTGGATCCACCACCTGGCCCGGCATGCC
GTGGCCTGCTTCCTGACCCGCGGGGACCTCTGGGTCAGCTGGGAGAGCGGGGTCCGGGTA
TTTGATGAGCTGCTCCTGGATGCAGATTTCAGCGTGAACGCAGGCAGCTGGATGTGGCTG
TCCTGCAGTGCTTTCTTCCAGCAGTTCTTCCACTGCTACTGCCCTGTGGGCTTTGGCCGT
CGCACGGACCCCAGTGGGGACTACATCAGGCGATACCTGCCCAAATTGAAAGCGTTCCCC
TCTCGATACATCTATGAGCCCTGGAATGCCCCAGAGTCAATTCAGAAGGCAGCCAAGTGC
ATCATTGGTGTGGACTACCCACGGCCCATCGTCAACCATGCCGAGACCAGCCGGCTTAAC
ATTGAACGAATGAAGCAGATTTACCAGCAGCTTTCGCGCTACCGGGGACTCTGTCTACTG
GCATCTGTCCCTTCCTGTGTGGAAGACCTCAGTCACCCTGTGGCAGAGCCCAGCTCGAGC
CAGGCTGGCAGCATGAGCAGTGCAGGCCCAAGACCACTACCCAGTGGCCCAGCATCCCCC
AAACGCAAGCTGGAAGCAGCCGAGGAACCACCTGGTGAAGAACTCAGCAAACGGGCCCGG
GTGGCAGAGTTGCCAACCCCAGAGCTGCCGAGCAAGGATGCCTGA

The sequence of cDNA NR1D1comprises SEQ ID No. 10
>ENST00000246672.4 NR1D1-201 cdna:protein_coding

ATGACGACCCTGGACTCCAACAACAACACAGGTGGCGTCATCACCTACATTGGCTCCAGT
GGCTCCTCCCCAAGCCGCACCAGCCCTGAATCCCTCTATAGTGACAACTCCAATGGCAGC
TTCCAGTCCCTGACCCAAGGCTGTCCCACCTACTTCCCACCATCCCCCACTGGCTCCCTC
ACCCAAGACCCGGCTCGCTCCTTTGGGAGCATTCCACCCAGCCTGAGTGATGACGGCTCC
CCTTCTTCCTCATCTTCCTCGTCGTCATCCTCCTCCTCCTTCTATAATGGGAGCCCCCCT
GGGAGTCTACAAGTGGCCATGGAGGACAGCAGCCGAGTGTCCCCCAGCAAGAGCACCAGC
AACATCACCAAGCTGAATGGCATGGTGTTACTGTGTAAAGTGTGTGGGGACGTTGCCTCG
GGCTTCCACTACGGTGTGCACGCCTGCGAGGGCTGCAAGGGCTTTTTCCGTCGGAGCATC
CAGCAGAACATCCAGTACAAAAGGTGTCTGAAGAATGAGAATTGCTCCATCGTCCGCATC
AATCGCAACCGCTGCCAGCAATGTCGCTTCAAGAAGTGTCTCTCTGTGGGCATGTCTCGA
GACGCTGTGCGTTTTGGGCGCATCCCCAAACGAGAGAAGCAGCGGATGCTTGCTGAGATG
CAGAGTGCCATGAACCTGGCCAACAACCAGTTGAGCAGCCAGTGCCCGCTGGAGACTTCA
CCCACCCAGCACCCCACCCCAGGCCCCATGGGCCCCTCGCCACCCCCTGCTCCGGTCCCC
TCACCCCTGGTGGGCTTCTCCCAGTTTCCACAACAGCTGACGCCTCCCAGATCCCCAAGC
CCTGAGCCCACAGTGGAGGATGTGATATCCCAGGTGGCCCGGGCCCATCGAGAGATCTTC
ACCTACGCCCATGACAAGCTGGGCAGCTCACCTGGCAACTTCAATGCCAACCATGCATCA
GGTAGCCCTCCAGCCACCACCCCACATCGCTGGGAAAATCAGGGCTGCCCACCTGCCCCC

AATGACAACAACACCTTGGCTGCCCAGCGTCATAACGAGGCCCTAAATGGTCTGCGCCAG
GCTCCCTCCTCCTACCCTCCCACCTGGCCTCCTGGCCCTGCACACCACAGCTGCCACCAG
TCCAACAGCAACGGGCACCGTCTATGCCCCACCCACGTGTATGCAGCCCCAGAAGGCAAG
GCACCTGCCAACAGTCCCCGGCAGGGCAACTCAAAGAATGTTCTGCTGGCATGTCCTATG
AACATGTACCCGCATGGACGCAGTGGGCGAACGGTGCAGGAGATCTGGGAGGATTTCTCC
ATGAGCTTCACGCCCGCTGTGCGGGAGGTGGTAGAGTTTGCCAAACACATCCCGGGCTTC
CGTGACCTTTCTCAGCATGACCAAGTCACCCTGCTTAAGGCTGGCACCTTTGAGGTGCTG
ATGGTGCGCTTTGCTTCGTTGTTCAACGTGAAGGACCAGACAGTGATGTTCCTAAGCCGC
ACCACCTACAGCCTGCAGGAGCTTGGTGCCATGGGCATGGGAGACCTGCTCAGTGCCATG
TTCGACTTCAGCGAGAAGCTCAACTCCCTGGCGCTTACCGAGGAGGAGCTGGGCCTCTTC
ACCGCGGTGGTGCTTGTCTCTGCAGACCGCTCGGGCATGGAGAATTCCGCTTCGGTGGAG
CAGCTCCAGGAGACGCTGCTGCGGGCTCTTCGGGCTCTGGTGCTGAAGAACCGGCCCTTG
GAGACTTCCCGCTTCACCAAGCTGCTGCTCAAGCTGCCGGACCTGCGGACCCTGAACAAC
ATGCATTCCGAGAAGCTGCTGTCCTTCCGGGTGGACGCCCAGTGA

The sequence of cDNA NR1D2 comprises SEQ ID No.11
>ENST00000312521.9 NR1D2-201 cdna:protein_coding

ATGGAGGTGAATGCAGGAGGTGTGATTGCCTATATCAGTTCTTCCAGCTCAGCCTCAAGC
CCTGCCTCTTGTCACAGTGAGGGTTCTGAGAATAGTTTCCAGTCCTCCTCCTCTTCTGTT
CCATCTTCTCCAAATAGCTCTAATTCTGATACCAATGGTAATCCCAAGAATGGTGATCTC
GCCAATATTGAAGGCATCTTGAAGAATGATCGAATAGATTGTTCTATGAAAACAAGCAAA
TCGAGTGCACCTGGGATGACAAAAGTCATAGTGGTGTGACAAAATTTAGTGGCATGGTT
CTACTGTGTAAAGTCTGTGGGGATGTGGCGTCAGGATTCCACTATGGAGTTCATGCTTGC
GAAGGCTGTAAGGGTTTCTTTCGGAGAAGTATTCAACAAAACATCCAGTACAAGAAGTGC
CTGAAGAATGAAAACTGTTCTATAATGAGAATGAATAGGAACAGATGTCAGCAATGTCGC
TTCAAAAAGTGTCTGTCTGTTGGAATGTCAAGAGATGCTGTTCGGTTTGGTCGTATTCCT
AAGCGTGAAAAACAGAGGATGCTAATTGAAATGCAAAGTGCAATGAAGACCATGATGAAC
AGCCAGTTCAGTGGTCACTTGCAAAATGACACATTAGTAGAACATCATGAACAGACAGCC
TTGCCAGCCCAGGAACAGCTGCGACCCAAGCCCCAACTGGAGCAAGAAAACATCAAAAGC
TCTTCTCCTCCATCTTCTGATTTTGCAAAGGAAGAAGTGATTGGCATGGTGACCAGAGCT
CACAAGGATACCTTTATGTATAATCAAGAGCAGCAAGAAAACTCAGCTGAGAGCATGCAG
CCCCAGAGAGGAGAACGGATTCCCAAGAACATGGAGCAATATAATTTAAATCATGATCAT
TGCGGCAATGGGCTTAGCAGCCATTTTCCCTGTAGTGAGAGCCAGCAGCATCTCAATGGA
CAGTTCAAAGGGAGGAATATAATGCATTACCCAAATGGTCATGCCATTTGTATTGCAAAT
GGACATTGTATGAACTTCTCCAATGCTTATACTCAAAGAGTATGTGATAGAGTTCCGATA
GATGGATTTTCTCAGAATGAGAACAAGAATAGTTACCTGTGCAACACTGGAGGAAGAATG
CATCTGGTTTGTCCAATGAGTAAGTCTCCATATGTGGATCCTCATAAATCAGGACATGAA
ATCTGGGAAGAATTTTCGATGAGCTTCACTCCAGCAGTGAAAGAAGTGGTGGAATTTGCA
AAGCGTATTCCTGGGTTCAGAGATCTCTCTCAGCATGACCAGGTCAACCTTTTAAAGGCT
GGGACTTTTGAGGTTTTAATGGTACGGTTCGCATCATTATTTGATGCAAAGGAACGTACT
GTCACCTTTTTAAGTGGAAAGAAATATAGTGTGGATGATTTACACTCAATGGGAGCAGGG
GATCTGCTAAACTCTATGTTTGAATTTAGTGAGAAGCTAAATGCCCTCCAACTTAGTGAT
GAAGAGATGAGTTTGTTTACAGCTGTTGTCCTGGTATCTGCAGATCGATCTGGAATAGAA
AACGTCAACTCTGTGGAGGCTTTGCAGGAAACTCTCATTCGTGCACTAAGGACCTTAATA

ATGAAAACCATCCAAATGAGGCCTCTATTTTTACAAAACTGCTTCTAAAGTTGCCAGAT
CTTCGATCTTTAAACAACATGCACTCTGAGGAGCTCTTGGCCTTTAAAGTTCACCCTTAA

The sequence of cDNA RORA comprises SEQ ID No. 12
>ENST00000335670.11 RORA-203 cdna:protein_coding

```
ATGGAGTCAGCTCCGGCAGCCCCCGACCCCGCCGCCAGCGAGCCAGGCAGCAGCGGCGCG
GACGCGGCCGCCGGCTCCAGGGAGACCCCGCTGAACCAGGAATCCGCCCGCAAGAGCGAG
CCGCCTGCCCCGGTGCGCAGACAGAGCTATTCCAGCACCAGCAGAGGTATCTCAGTAACG
AAGAAGACACATACATCTCAAATTGAATTATTCCATGCAAGATCTGTGGAGACAAATCA
TCAGGAATCCATTATGGTGTCATTACATGTGAAGGCTGCAAGGGCTTTTTCAGGAGAAGT
CAGCAAAGCAATGCCACCTACTCCTGTCCTCGTCAGAAGAACTGTTTGATTGATCGAACC
AGTAGAAACCGCTGCCAACACTGTCGATTACAGAAATGCCTTGCCGTAGGGATGTCTCGA
GATGCTGTAAAATTTGGCCGAATGTCAAAAAAGCAGAGAGACAGCTTGTATGCAGAAGTA
CAGAAACACCGGATGCAGCAGCAGCAGCGCGACCACCAGCAGCAGCCTGGAGAGGCTGAG
CCGCTGACGCCCACCTACAACATCTCGGCCAACGGGCTGACGGAACTTCACGACGACCTC
AGTAACTACATTGACGGGCACACCCCTGAGGGGAGTAAGGCAGACTCCGCCGTCAGCAGC
TTCTACCTGGACATACAGCCTTCCCCAGACCAGTCAGGTCTTGATATCAATGGAATCAAA
CCAGAACCAATATGTGACTACACACCAGCATCAGGCTTCTTTCCCTACTGTTCGTTCACC
AACGGCGAGACTTCCCCAACTGTGTCCATGGCAGAATTAGAACACCTTGCACAGAATATA
TCTAAATCGCATCTGGAAACCTGCCAATACTTGAGAGAAGAGCTCCAGCAGATAACGTGG
CAGACCTTTTTACAGGAAGAAATTGAGAACTATCAAAACAAGCAGCGGGAGGTGATGTGG
CAATTGTGTGCCATCAAAATTACAGAAGCTATACAGTATGTGGTGGAGTTTGCCAAACGC
ATTGATGGATTTATGGAACTGTGTCAAAATGATCAAATTGTGCTTCTAAAAGCAGGTTCT
CTAGAGGTGGTGTTTATCAGAATGTGCCGTGCCTTTGACTCTCAGAACAACACCGTGTAC
TTTGATGGGAAGTATGCCAGCCCCGACGTCTTCAAATCCTTAGGTTGTGAAGACTTTATT
AGCTTTGTGTTTGAATTTGGAAAGAGTTTATGTTCTATGCACCTGACTGAAGATGAAATT
GCATTATTTTCTGCATTTGTACTGATGTCAGCAGATCGCTCATGGCTGCAAGAAAAGGTA
AAAATTGAAAAACTGCAACAGAAAATTCAGCTAGCTCTTCAACACGTCCTACAGAAGAAT
CACCGAGAAGATGGAATACTAACAAAGTTAATATGCAAGGTGTCTACCTTAAGAGCCTTA
TGTGGACGACATACAGAAAAGCTAATGGCATTTAAAGCAATATACCCAGACATTGTGCGA
CTTCATTTTCCTCCATTATACAAGGAGTTGTTCACTTCAGAATTTGAGCCAGCAATGCAA
ATTGATGGGTAA
```

The sequence of cDNA RORB comprises SEQ ID No. 13
>ENST00000376896.8 RORB-201 cdna:protein_coding

```
ATGCGAGCACAAATTGAAGTGATACCATGCAAAATTTGTGGCGATAAGTCCTCTGGGATC
CACTACGGAGTCATCACATGTGAAGGCTGCAAGGGATTCTTTAGGAGGAGCCAGCAGAAC
AATGCTTCTTATTCCTGCCCAAGGCAGAGAAACTGTTTAATTGACAGAACGAACAGAAAC
CGTTGCCAACACTGCCGACTGCAGAAGTGTCTTGCCCTAGGAATGTCAAGAGATGCTGTG
AAGTTTGGGAGGATGTCCAAGAAGCAAAGGGACAGCCTGTATGCTGAGGTGCAGAAGCAC
CAGCAGCGGCTGCAGGAACAGCGGCAGCAGCAGAGTGGGGAGGCAGAAGCCCTTGCCAGG
GTGTACAGCAGCAGCATTAGCAACGGCCTGAGCAACCTGAACAACGAGACCAGCGGCACT
```

TATGCCAACGGGCACGTCATTGACCTGCCCAAGTCTGAGGGGTTATTACAACGTCGATTCC
GGTCAGCCGTCCCCTGATCAGTCAGGACTTGACATGACTGGAATCAAACAGATAAAGCAA
GAACCTATCTATGACCTCACATCCGTACCCAACTTGTTTACCTATAGCTCTTTCAACAAT
GGGCAGTTAGCACCAGGGATAACCATGACTGAAATCGACCGAATTGCACAGAACATCATT
AAGTCCCATTTGGAGACATGTCAATACACCATGGAAGAGCTGCACCAGCTGGCGTGGCAG
ACCCACACCTATGAAGAAATTAAAGCATATCAAAGCAAGTCCAGGGAAGCACTGTGGCAA
CAATGTGCCATCCAGATCACTCACGCCATCCAATACGTGGTGGAGTTTGCAAAGCGGATA
ACAGGCTTCATGGAGCTCTGTCAAAATGATCAAATTCTACTTCTGAAGTCAGGTTGCTTG
GAAGTGGTTTTAGTGAGAATGTGCCGTGCCTTCAACCCATTAAACAACACTGTTCTGTTT
GAAGGAAAATATGGAGGAATGCAAATGTTCAAAGCCTTAGGTTCTGATGACCTAGTGAAT
GAAGCATTTGACTTTGCAAAGAATTTGTGTTCCTTGCAGCTGACCGAGGAGGAGATCGCT
TTGTTCTCATCTGCTGTTCTGATATCTCCAGACCGAGCCTGGCTTATAGAACCAAGGAAA
GTCCAGAAGCTTCAGGAAAAAATTTATTTTGCACTTCAACATGTGATTCAGAAGAATCAC
CTGGATGATGAGACCTTGGCAAAGTTAATAGCCAAGATACCAACCATCACGGCAGTTTGC
AACTTGCACGGGGAGAAGCTGCAGGTATTTAAGCAATCTCATCCAGAGATAGTGAATACA
CTGTTTCCTCCGTTATACAAGGAGCTCTTTAATCCTGACTGTGCCACCGGCTGCAAATGA

The sequence of cDNA RORC comprises SEQ ID No. 14
>ENST00000318247.7 RORC-201 cdna:protein_coding

ATGGACAGGGCCCCACAGAGACAGCACCGAGCCTCACGGGAGCTGCTGGCTGCAAAGAAG
ACCCACACCTCACAAATTGAAGTGATCCCTTGCAAAATCTGTGGGGACAAGTCGTCTGGG
ATCCACTACGGGGTTATCACCTGTGAGGGGTGCAAGGGCTTCTTCCGCCGGAGCCAGCGC
TGTAACGCGGCCTACTCCTGCACCCGTCAGCAGAACTGCCCCATCGACCGCACCAGCCGA
AACCGATGCCAGCACTGCCGCCTGCAGAAATGCCTGGCGCTGGGCATGTCCCGAGATGCT
GTCAAGTTCGGCCGCATGTCCAAGAAGCAGAGGGACAGCCTGCATGCAGAAGTGCAGAAA
CAGCTGCAGCAGCGGCAACAGCAGCAACAGGAACCAGTGGTCAAGACCCCTCCAGCAGGG
GCCCAAGGAGCAGATACCCTCACCTACACCTTGGGGCTCCCAGACGGGCAGCTGCCCCTG
GGCTCCTCGCCTGACCTGCCTGAGGCTTCTGCCTGTCCCCCTGGCCTCCTGAAAGCCTCA
GGCTCTGGGCCCTCATATTCCAACAACTTGGCCAAGGCAGGGCTCAATGGGGCCTCATGC
CACCTTGAATACAGCCCTGAGCGGGGCAAGGCTGAGGGCAGAGAGAGCTTCTATAGCACA
GGCAGCCAGCTGACCCCTGACCGATGTGGACTTCGTTTTGAGGAACACAGGCATCCTGGG
CTTGGGGAACTGGACAGGGCCCAGACAGCTACGGCAGCCCCAGTTTCCGCAGCACACCG
GAGGCACCCTATGCCTCCCTGACAGAGATAGAGCACCTGGTGCAGAGCGTCTGCAAGTCC
TACAGGGAGACATGCCAGCTGCGGCTGGAGGACCTGCTGCGGCAGCGCTCCAACATCTTC
TCCCGGGAGGAAGTGACTGGCTACCAGAGGAAGTCCATGTGGGAGATGTGGGAACGGTGT
GCCCACCACCTCACCGAGGCCATTCAGTACGTGGTGGAGTTCGCCAAGAGGCTCTCAGGC
TTTATGGAGCTCTGCCAGAATGACCAGATTGTGCTTCTCAAAGCAGGAGCAATGGAAGTG
GTGCTGGTTAGGATGTGCCGGGCCTACAATGCTGACAACCGCACGGTCTTTTTTGAAGGC
AAATACGGTGGCATGGAGCTGTTCCGAGCCTTGGGCTGCAGCGAGCTCATCAGCTCCATC
TTTGACTTCTCCCACTCCCTAAGTGCCTTGCACTTTTCCGAGGATGAGATTGCCCTCTAC
ACAGCCCTTGTTCTCATCAATGCCCATCGGCCAGGGCTCCAAGAGAAAAGGAAAGTAGAA
CAGCTGCAGTACAATCTGGAGCTGGCCTTTCATCATCATCTCTGCAAGACTCATCGCCAA
AGCATCCTGGCAAAGCTGCCACCCAAGGGGAAGCTTCGGAGCCTGTGTAGCCAGCATGTG
GAAAGGCTGCAGATCTTCCAGCACCTCCACCCCATCGTGGTCCAAGCCGCTTTCCCTCCA

CTCTACAAGGAGCTCTTCAGCACTGAAACCGAGTCACCTGTGGGGCTGTCCAAGTGA

In one embodiment of the method, the at least one further gene involved in the metabolism of the medicament to be administered is at least one of Ugt1a1 and Abcb1.

In one embodiment of the method according to the present invention said assessing the timing of administration of said medicament to said subject comprises evaluating the predicted gene expression levels and/or evaluating expression phenotypes based on the determined and/or predicted gene expression levels.

In one embodiment of the method according to the present invention assessing the circadian rhythm of said subject comprises in the computational step determining a periodic function for each of said determined genes that approximates said gene expression levels for each of said genes, preferably comprising curve fitting of a non-linear periodic model function to the respective gene expression levels, wherein the curve fitting is preferably carried out by means of harmonic regression.

In one embodiment of the method according to the invention assessing the circadian rhythm of said subject comprises determining a periodic function for each of said determined genes, in particular at least two core clock genes, in particular for said at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, that approximates said expression levels for each of at least two core clock genes, in particular for said at least two members of the groups ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, preferably comprising curve fitting of a non-linear periodic model function to the respective expression levels, wherein the curve fitting is preferably carried out by means of harmonic regression.

It will be appreciated that other curve fitting methods may be applied for determining a mathematical function that has the best fit to the series of the measured gene expressions. The skilled person will understand that curve fitting in the context of this disclosure aims at finding a periodic function (oscillatory function) because of the periodicity of the circadian clock(s). While curve fitting may generally aim at finding an interpolation for exact fitting of the data points, methods that approximate the series of measure gene expressions will be preferred, e.g. smoothing, in which a "smooth" function is constructed that approximately fits the data.

It has been found that regression analysis methods are more appropriate here, which use statistical data, not least because the determined periodic function shall represent not only the measured data points but particularly future values. Polynomial interpolation or polynomial regression may be alternatively applied. Preferably, harmonic regression is used, which is based on the trigonometric functions sine and cosine. As will be appreciated by a person skilled in the art, various methods for minimizing an error between the fitted curve and the measured data points may be applied, such as square errors, which is set forth in more detail below. In the method of harmonic regression, the model $y(t) = m + a \cos(\cot) + b \sin(\omega t)$ is fitted to the measured data to determine the absolute ($A = \sqrt{(a2 + b2)}$) and relative amplitude as well as the phase ($\tan \varphi = b/a$), the p-value and the confidence interval. The significance level p may be selected as $p < 0.05$.

In one embodiment of the method according to the present invention the computational step comprises processing the determined gene expression levels to derive characteristic data for each of said genes, said processing comprising determining the mean expression level of expression of a gene and normalizing the gene expression levels using the mean expression level.

Particularly in view of the machine learning processes as described below, the "raw data", i.e. the measured gene expression levels, including the obtained periodic functions resulting from the curve fitting, have to be preprocessed to bring them into a form that is suitable for the intended machine learning algorithm. For instance, the preprocessing includes extracting data of interest (characteristic data) and setting the dimensionality for the machine learning, i.e. number of parameters. Further, in order to get comparable parameters, normalization is typically required to achieve a common scale for all parameters. It has been found that using the mean expression level for normalizing the measured data is a suitable approach. Further, in order not to lose the absolute values, the mean level is added to the parameter space. This will be set forth also in more detail below.

In one embodiment of the method according to the present invention said characteristic data comprise:

- the amplitude of change of expression of a gene, and/or the amplitude relative to one of the other genes, and/or
- the mean expression level of expression of a gene and/ or the mean relative to one of the other genes, and/or
- the peak expression level of a gene, and/or the peak relative to one of the other genes, and/or
- the amplitude of change of expression of ARNTL (BMAL1) and/or ARNTL2 and/or CLOCK, and/or NPAS2 and/or PER1 and/or PER2 and/or PER3 and/or CRY1 and/or CRY2 and/or NRIDI and/or NR1D2 and/or RORA and/or RORB and/or RORC over the day, and/or
- the relative difference of the amplitudes of change of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or

- the mean expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/ or
- the relative difference of the mean expression levels of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the peak expression levels of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the time of the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,
- the relative difference of the times of the peak expression level of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,

wherein the amplitude, period and phase expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC are extracted from the determined expression levels and/or the respectively fitted periodic function.

In one embodiment of the method according to the present invention the computational step further comprises

- fitting a network computational model to the derived characteristic data that comprises a representation of the periodic time course of the expression levels for each of the determined genes, in particular at least two core clock genes, in particular for said at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, as well as a representation of the periodic time course of the expression level for at least one, preferably a plurality of further gene(s) included in a gene regulatory network that includes said genes; and/or
- training a machine learning algorithm on the derived characteristic data of the network computational model, particularly optimize in terms of the representation of the periodic time course of the expression level for the at least one further gene.

In particular, the network computational model is built to obtain data for at least one further gene that has not been directly measured in the saliva samples, i.e. the network computational model represents a gene network which contains the clock elements, the metabolic elements and the drug target of the respective drug, as well as further elements, which further elements cannot (or at least not with reasonable effort) be measured particularly in saliva samples. This mathematical modelling may use differential equations and also statistical data.

In one embodiment of the method according to the present invention assessing the timing of administration of said medicament to said subject comprises in the computational step fitting a prediction computational model on data obtained from said fitted periodic functions and/or said network computational model, wherein the prediction computational model is based on machine learning including at least one classification method and/or at least one clustering method, wherein said method(s) are preferably selected from the group comprising: K-nearest neighbor algorithm, unsupervised clustering, deep neural networks, random forest algorithm, and support vector machines.

In one embodiment of the method according to the present invention assessing the timing of administration of said medicament comprises in the computational step:

- Allocating a time-dependent numerical value to said gene expressions corresponding to the respective expression levels of said genes, said genes particularly including ARNTL (BMAL1), ARNTL2, CLOCK, PER1, PER2, PER3, NPAS2, CRY1, CRY2, NR1D1, NR1D2, RORA, RORB, RORC; and
- Selecting the optimal and/or non-optimal time of administration of said medicament based on a calculation result using said allocated time-dependent numerical values, wherein a ratio of said time-dependent numerical values for a particular subject is determined, particularly using the network computational model, wherein then, particularly applying the prediction computational model, a minimum and/or maximum of said determined ratios indicates said optimal and/or non-optimal time of administration of said medicament or a range of said determined ratio indicates a period of the day indicating said optimal and/or non-optimal time of administration of said medicament.

In one embodiment of the method according to the present invention additional physiological data of the subject are provided for fitting the prediction computational model. Said physiological data maybe selected from the group comprising: body temperature, heart rate, eating/fasting patterns and/or sleep/wake patterns. It will be appreciated that one or more of the aforementioned physiological data or other physiological parameters from the subject may be provided. While such data may be obtained manually by the subject (user) and/or by medical staff, it may be envisioned to obtain at least some of the physiological data by means of a portable electronic device, particularly a wearable, such as a fitness watch, wristband or the like. Vice versa, the result of the method of the present invention may be presented on such wearable device so that the user directly sees e.g. their circadian profile (just like they are used to see other physiological or fitness data, e.g. how long and how fast their jogging was, or how their sleep quality was). Of course, the result may be provided by other electronic devices, like a smartphone, tablet or personal computer.

In one embodiment of the method according to the invention the network computational model and/or the prediction computational model form a personalized model for said subject. The personalization particularly comes from the molecular data, i.e. the measurements of the gene expression which are unique for each person. Additional physiological data like temperature, heart rate, sleep/wake cycles as mentioned above can also be used for personalization. These are all circadian events, too, meaning they vary within 24 hours. While such physiological data may be of additional value, the models, and thus predictions are primarily based on the molecular data, i.e. the gene expressions. It is noted that while the network computational model may be personalized because there is a new model for each new person (using the personal gene expressions), the prediction computational model is not. There is one prediction model relating subject data to prediction for all subjects, and this model should generalize to future subjects without being retrained.

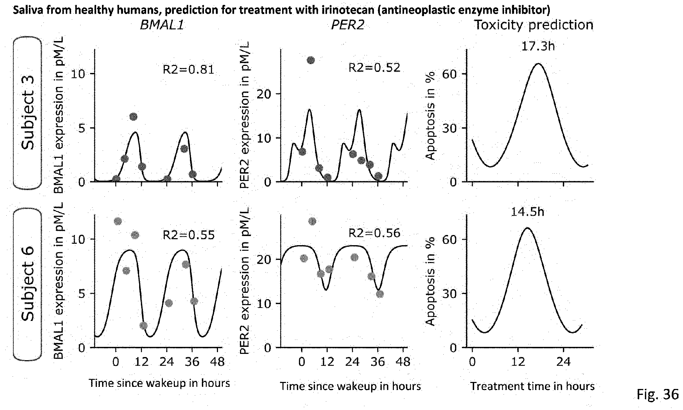

The methods of the present invention maybe used to predict toxicity curves for a person using saliva gene expression data (see new **Figure 36-37**). The data shows minimum and maximum predicted cell toxicity, which can be used to suggest treatment times for a subject based on gene expression data of clock- and drug related genes.

In one embodiment of the method according to the present invention samples of at least two consecutive days of said subject are provided and the gene expression levels of said genes in said samples are determined and used, preferably at least three samples per day, more preferably at least four samples per day.

In one embodiment of the method according to the present invention assessing the timing of administration of said medicament comprises in the computational step:

- Allocating a time-dependent numerical value to said gene expressions corresponding to the respective expression levels of said genes, said genes particularly including ARNTL (BMAL1), ARNTL2, CLOCK, PER1, PER2, PER3, NPAS2, CRY1, CRY2, NR1D1, NR1D2, RORA, RORB, RORC; and
- Selecting the optimal and/or non-optimal time of administration of said medicament based on a calculation result using said allocated time-dependent numerical values, wherein a ratio of said time-dependent numerical values for a particular subject is determined, particularly using the network computational model, wherein then, particularly applying the prediction computational model, a minimum and/or maximum of said determined ratios indicates said optimal and/or non-optimal time of administration of said medicament or a range of said determined ratio indicates a period of the day indicating said optimal and/or non-optimal time of administration of said medicament.

In one embodiment of the method according to the present invention

- the medicament is Filgrastim and the target gene is *Csf3r* and the indication is acute myeloid leukemia; or
- the medicament is Rituximab and the target gene is selected from the group comprising *Fcgr2b, Ms4a1, and Fcgr3*; and the indication is rheumatoid arthritis and Non-Hodgkin's lymphoma; or
- the medicament is bevacizumab and the target gene is *Fcgr2b, Vegfa, Fcgr3*; and the indication is Colorectal cancer and Non-small cell lung cancer; or
- the medicament is trastuzumab and the target gene is Fcgr2b, Erbb2, Egfr, Fcgr3 and the indication is Breast cancer; or
- the medicament is Imatinib and the target gene is Ptgs1, Kit, Slc22a2, Abcg2, Pdgfra, Pdgfrb, Ddr1, Abca3, Abl1, Ret, Abcb1a and the indication is Chronic myeloid leukemia; or
- the medicament is Pemetrexed and the target gene is Tyms, Atic, Gart, Slc29a1 and the indication is Mesothelioma and Non-small cell lung cancer; or
- the medicament is Capecitabine and the target gene is Cda, Tymp, Tyms, Ces1g, Dpyd and the indication is Breast cancer and colorectal cancer; or
- the medicament is Erlotinib (tyrosine kinase inhibitor, anticancer drug) and the target gene is EGFR, Ras/Raf/MAPK, and PIK3/AKT (tumour) and the indication is Tumour inhibition (ZT1 >> ZT13); or

- the medicament is Sunitinib (tyrosine kinase inhibitor, anticancer drug) and the target gene is Cyp3a11 (liver, duodenum, jejunum) abcb1a (liver, duodenum, jejunum, lung) and the indication is renal cell cancer and pancreatic neuroendocrine tumours; or
- the medicament is Lapatinib (dual tyrosine kinase inhibitor interrupting the HER2/neu and EGFR pathways, anticancer drug) and the target gene is EGFR/Ras/Raf/MAPK, Errfi 1, Dusp1 (liver), Hbegf, Tgfα, Eref (liver) and the indication is solid tumours such as breast and lung cancer; or
- the medicament is Roscovitine (seliciclib, CDK inhibitor, anticancer drug) and the target gene is Cyp3a11, Cyp3a13(liver) and the indication is non-small cell lung cancer (NSCLC) and leukemia; or
- the medicament is Everolimus (mTOR inhibitor, anticancer drug, immunosuppressant) and the target gene is mTOR/Fbxw7/P70S6K (tumour) and the indication is breast cancer; or
- the medicament is Irinotecan (Top1 inhibitor, anticancer drug) and the target gene is Ces2, Ugt1a1, abcb1a, abcb1b (liver and ileum), abcc2 (ileum) and the indication for colorectal cancer, advanced pancreatic cancer and small cell lung cancer; or
- the medicament is Tamoxifen (antiestrogenic, anticancer drug) and the target gene is Cyp2d10, Cyp2d22, Cyp3a11 (liver) and the indication is breast cancer; or
- the medicament Bleomycin (toxicant and anticancer drug) and the target gene is NRF2/glutathione antioxidant defence and the indication is pulmonary fibrosis, palliative treatment in the management malignant neoplasm (trachea, bronchus, lung), squamous cell carcinoma, and lymphomas.

The sequence of Csf3r comprises SEQ ID No. 15
> ENST00000373103.5 Csf3r-203 cdna:protein_coding

```
ATGGCAAGGCTGGGAAACTGCAGCCTGACTTGGGCTGCCCTGATCATCCTGCTGCTCCCC
GGAAGTCTGGAGGAGTGCGGGCACATCAGTGTCTCAGCCCCCATCGTCCACCTGGGGGAT
CCCATCACAGCCTCCTGCATCATCAAGCAGAACTGCAGCCATCTGGACCCGGAGCCACAG
ATTCTGTGGAGACTGGGAGCAGAGCTTCAGCCCGGGGGCAGGCAGCAGCGTCTGTCTGAT
GGGACCCAGGAATCTATCATCACCCTGCCCCACCTCAACCACACTCAGGCCTTTCTCTCC
TGCTGCCTGAACTGGGGCAACAGCCTGCAGATCCTGGACCAGGTTGAGCTGCGCGCAGGC
TACCCTCCAGCCATACCCCACAACCTCTCCTGCCTCATGAACCTCACAACCAGCAGCCTC
ATCTGCCAGTGGGAGCCAGGACCTGAGACCCACCTACCCACCAGCTTCACTCTGAAGAGT
TTCAAGAGCCGGGGCAACTGTCAGACCCAAGGGGACTCCATCCTGGACTGCGTGCCCAAG
GACGGGCAGAGCCACTGCTGCATCCCACGCAAACACCTGCTGTTGTACCAGAATATGGGC
ATCTGGGTGCAGGCAGAGAATGCGCTGGGGACCAGCATGTCCCCACAACTGTGTCTTGAT
CCCATGGATGTTGTGAAACTGGAGCCCCCCATGCTGCGGACCATGGACCCCAGCCCTGAA
GCGGCCCCTCCCCAGGCAGGCTGCCTACAGCTGTGCTGGGAGCCATGGCAGCCAGGCCTG
CACATAAATCAGAAGTGTGAGCTGCGCCACAAGCCGCAGCGTGGAGAAGCCAGCTGGGCA
CTGGTGGGCCCCCTCCCCTTGGAGGCCCTTCAGTATGAGCTCTGCGGGCTCCTCCCAGCC
ACGGCCTACACCCTGCAGATACGCTGCATCCGCTGGCCCCTGCCTGGCCACTGGAGCGAC
TGGAGCCCCAGCCTGGAGCTGAGAACTACCGAACGGGCCCCCACTGTCAGACTGGACACA
TGGTGGCGGCAGAGGCAGCTGGACCCCAGGACAGTGCAGCTGTTCTGGAAGCCAGTGCCC
CTGGAGGAAGACAGCGGACGGATCCAAGGTTATGTGGTTTCTTGGAGACCCTCAGGCCAG
GCTGGGGCCATCCTGCCCCTCTGCAACACCACAGAGCTCAGCTGCACCTTCCACCTGCCT
TCAGAAGCCCAGGAGGTGGCCCTTGTGGCCTATAACTCAGCCGGGACCTCTCGTCCCACT
```

CCGGTGGTCTTCTCAGAAAGCAGAGGCCCAGCTCTGACCAGACTCCATGCCATGGCCCGA
GACCCTCACAGCCTCTGGGTAGGCTGGGAGCCCCCCAATCCATGGCCTCAGGGCTATGTG
ATTGAGTGGGGCCTGGGCCCCCCAGCGCGAGCAATAGCAACAAGACCTGGAGGATGGAA
CAGAATGGGAGAGCCACGGGGTTTCTGCTGAAGGAGAACATCAGGCCCTTTCAGCTCTAT
GAGATCATCGTGACTCCCTTGTACCAGGACACCATGGGACCCTCCCAGCATGTCTATGCC
TACTCTCAAGAAATGGCTCCCTCCCATGCCCCAGAGCTGCATCTAAAGCACATTGGCAAG
ACCTGGGCACAGCTGGAGTGGGTGCCTGAGCCCCCTGAGCTGGGGAAGAGCCCCCTTACC
CACTACACCATCTTCTGGACCAACGCTCAGAACCAGTCCTTCTCCGCCATCCTGAATGCC
TCCTCCCGTGGCTTTGTCCTCCATGGCCTGGAGCCCGCCAGTCTGTATACATCCACCTC
ATGGCTGCCAGCCAGGCTGGGGCCACCAACAGTACAGTCCTCACCCTGATGACCTTGACC
CCAGAGGGGTCGGAGCTACACATCATCCTGGGCCTGTTCGGCCTCCTGCTGTTGCTCACC
TGCCTCTGTGGAACTGCCTGGCTCTGTTGCAGCCCCAACAGGAAGAATCCCCTCTGGCCA
AGTGTCCCAGACCCAGCTCACAGCAGCCTGGGCTCCTGGGTGCCCACAATCATGGAGGAG
CTGCCCGGACCCAGACAGGGACAGTGGCTGGGGCAGACATCTGAAATGAGCCGTGCTCTC
ACCCCACATCCTTGTGTGCAGGATGCCTTCCAGCTGCCCGGCCTTGGCACGCCACCCATC
ACCAAGCTCACAGTGCTGGAGGAGGATGAAAAGAAGCCGGTGCCCTGGGAGTCCCATAAC
AGCTCAGAGACCTGTGGCCTCCCCACTCTGGTCCAGACCTATGTGCTCCAGGGGGACCCA
AGAGCAGTTTCCACCCAGCCCCAATCCCAGTCTGGCACCAGCGATCAGGTCCTTTATGGG
CAGCTGCTGGGCAGCCCCACAAGCCCAGGGCCAGGGCACTATCTCCGCTGTGACTCCACT
CAGCCCCTCTTGGCGGGCCTCACCCCCAGCCCCAAGTCCTATGAGAACCTCTGGTTCCAG
GCCAGCCCCTTGGGGACCCTGGTAACCCCAGCCCCAAGCCAGGAGGACGACTGTGTCTTT
GGGCCACTGCTCAACTTCCCCCTCCTGCAGGGGATCCGGGTCCATGGGATGGAGGCGCTG
GGGAGCTTCTAG

The sequence of Fcgr2b comprises SEQ ID No. 16
> ENST00000358671.9 Fcgr2b-202 cdna:protein_coding

ATGGGAATCCTGTCATTCTTACCTGTCCTTGCCACTGAGAGTGACTGGGCTGACTGCAAG
TCCCCCCAGCCTTGGGGTCATATGCTTCTGTGGACAGCTGTGCTATTCCTGGCTCCTGTT
GCTGGGACACCTGCAGCTCCCCCAAAGGCTGTGCTGAAACTCGAGCCCCAGTGGATCAAC
GTGCTCCAGGAGGACTCTGTGACTCTGACATGCCGGGGGGACTCACAGCCCTGAGAGCGAC
TCCATTCAGTGGTTCCACAATGGGAATCTCATTCCCACCCACACGCAGCCCAGCTACAGG
TTCAAGGCCAACAACAATGACAGCGGGGAGTACACGTGCCAGACTGGCCAGACCAGCCTC
AGCGACCCTGTGCATCTGACTGTGCTTTCTGAGTGGCTGGTGCTCCAGACCCCTCACCTG
GAGTTCCAGGAGGGAGAAACCATCGTGCTGAGGTGCCACAGCTGGAAGGACAAGCCTCTG
GTCAAGGTCACATTCTTCCAGAATGGAAAATCCAAGAAATTTTCCCGTTCGGATCCCAAC
TTCTCCATCCCACAAGCAAACCACAGTCACAGTGGTGATTACCACTGCACAGGAAACATA
GGCTACACGCTGTACTCATCCAAGCCTGTGACCATCACTGTCCAAGCTCCCAGCTCTTCA
CCGATGGGGATCATTGTGGCTGTGGTCACTGGGATTGCTGTAGCGGCCATTGTTGCTGCT
GTAGTGGCCTTGATCTACTGCAGGAAAAAGCGGATTTCAGCTCTCCCAGGATACCCTGAG
TGCAGGGAAATGGGAGAGACCCTCCCTGAGAAACCAGCCAATCCCACTAATCCTGATGAG
GCTGACAAAGTTGGGGCTGAGAACACAATCACCTATTCACTTCTCATGCACCCGGATGCT
CTGGAAGAGCCTGATGACCAGAACCGTATTTAG

The sequence of Ms4a1 comprises SEQ ID No. 17
> ENST00000345732.9 Ms4a1-201 cdna:protein_coding

ATGACAACACCCAGAAATTCAGTAAATGGGACTTTCCCGGCAGAGCCAATGAAAGGCCCT
ATTGCTATGCAATCTGGTCCAAAACCACTCTTCAGGAGGATGTCTTCACTGGTGGGCCCC
ACGCAAAGCTTCTTCATGAGGGAATCTAAGACTTTGGGGGCTGTCCAGATTATGAATGGG
CTCTTCCACATTGCCCTGGGGGGTCTTCTGATGATCCCAGCAGGGATCTATGCACCCATC
TGTGTGACTGTGTGGTACCCTCTCTGGGGAGGCATTATGTATATTATTTCCGGATCACTC
CTGGCAGCAACGGAGAAAAACTCCAGGAAGTGTTTGGTCAAAGGAAAAATGATAATGAAT
TCATTGAGCCTCTTTGCTGCCATTTCTGGAATGATTCTTTCAATCATGGACATACTTAAT
ATTAAAATTTCCCATTTTTTAAAAATGGAGTCTGAATTTTATTAGAGCTCACACACCA
TATATTAACATATACAACTGTGAACCAGCTAATCCCTCTGAGAAAAACTCCCCATCTACC
CAATACTGTTACAGCATACAATCTCTGTTCTTGGGCATTTTGTCAGTGATGCTGATCTTT
GCCTTCTTCCAGGAACTTGTAATAGCTGGCATCGTTGAGAATGAATGGAAAAGAACGTGC
TCCAGACCCAAATCTAACATAGTTCTCCTGTCAGCAGAAGAAAAAAAGAACAGACTATT
GAAATAAAAGAAGAAGTGGTTGGGCTAACTGAAACATCTTCCCAACCAAAGAATGAAGAA
GACATTGAAATTATTCCAATCCAAGAAGAGGAAGAAGAAGAAACAGAGACGAACTTTCCA
GAACCTCCCCAAGATCAGGAATCCTCACCAATAGAAAATGACAGCTCTCCTTAA

The sequence of Fcgr3b comprises SEQ ID No.18
> ENST00000650385.1 Fcgr3b-208 cdna:protein_coding

ATGTGGCAGCTGCTCCTCCCAACTGCTCTGCTACTTCTAGTTTCAGCTGGCATGCGGACT
GAAGATCTCCCAAAGGCTGTGGTGTTCCTGGAGCCTCAATGGTACAGCGTGCTTGAGAAG
GACAGTGTGACTCTGAAGTGCCAGGGAGCCTACTCCCCTGAGGACAATTCCACACAGTGG
TTTCACAATGAGAACCTCATCTCAAGCCAGGCCTCGAGCTACTTCATTGACGCTGCCACA
GTCAACGACAGTGGAGAGTACAGGTGCCAGACAAACCTCTCCACCCTCAGTGACCCGGTG
CAGCTAGAAGTCCATATCGGCTGGCTGTTGCTCCAGGCCCCTCGGTGGGTGTTCAAGGAG
GAAGACCCTATTCACCTGAGGTGTCACAGCTGGAAGAACACTGCTCTGCATAAGGTCACA
TATTTACAGAATGGCAAAGACAGGAAGTATTTTCATCATAATTCTGACTTCCACATTCCA
AAAGCCACACTCAAAGATAGCGGCTCCTACTTCTGCAGGGGGCTTGTTGGGAGTAAAAAT
GTGTCTTCAGAGACTGTGAACATCACCATCACTCAAGGTTTGGCAGTGTCAACCATCTCA
TCATTCTCTCCACCTGGGTACCAAGTCTCTTTCTGCTTGGTGATGGTACTCCTTTTTGCA
GTGGACACAGGACTATATTTCTCTGTGAAGACAAACATTTGA

The sequence of Top1 comprises SEQ ID No. 19
> ENST00000361337.3 Top1-201 cdna:protein_coding

ATGAGTGGGGACCACCTCCACAACGATTCCCAGATCGAAGCGGATTTCCGATTGAATGAT
TCTCATAAACACAAAGATAAACACAAAGATCGAGAACACCGGCACAAAGAACACAAGAAG
GAGAAGGACCGGGAAAAGTCCAAGCATAGCAACAGTGAACATAAAGATTCTGAAAAGAAA
CACAAAGAGAAGGAGAAGACCAAACACAAAGATGGAAGCTCAGAAAAGCATAAAGACAAA
CATAAAGACAGAGACAAGGAAAAACGAAAGAGGAAAAGGTTCGAGCCTCTGGGGATGCA

AAAATAAAGAAGGAGAAGGAAAATGGCTTCTCTAGTCCACCACAAATTAAAGATGAACCT
GAAGATGATGGCTATTTTGTTCCTCCTAAAGAGGATATAAAGCCATTAAAGAGACCTCGA
GATGAGGATGATGCTGATTATAAACCTAAGAAAATTAAAACAGAAGATACCAAGAAGGAG
AAGAAAGAAAACTAGAAGAAGAAGAGGATGGTAAATTGAAAAAACCCAAGAATAAAGAT
AAAGATAAAAAAGTTCCTGAGCCAGATAACAAGAAAAAGAAGCCGAAGAAAGAAGAGGAA
CAGAAGTGGAAATGGTGGGAAGAAGAGCGCTATCCTGAAGGCATCAAGTGGAAATTCCTA
GAACATAAAGGTCCAGTATTTGCCCCACCATATGAGCCTCTTCCAGAGAATGTCAAGTTT
TATTATGATGGTAAAGTCATGAAGCTGAGCCCCAAAGCAGAGGAAGTAGCTACGTTCTTT
GCAAAAATGCTCGACCATGAATATACTACCAAGGAAATATTTAGGAAAAATTTCTTTAAA
GACTGGAGAAAGGAAATGACTAATGAAGAGAAGAATATTATCACCAACCTAAGCAAATGT
GATTTTACCCAGATGAGCCAGTATTTCAAAGCCCAGACGGAAGCTCGGAAACAGATGAGC
AAGGAAGAGAAACTGAAAATCAAAGAGGAGAATGAAAAATTACTGAAAGAATATGGATTC
TGTATTATGGATAACCACAAAGAGAGGATTGCTAACTTCAAGATAGAGCCTCCTGGACTT
TTCCGTGGCCGCGGCAACCACCCCAAGATGGGCATGCTGAAGAGACGAATCATGCCCGAG
GATATAATCATCAACTGTAGCAAAGATGCCAAGGTTCCTTCTCCTCCTCCAGGACATAAG
TGGAAAGAAGTCCGGCATGATAACAAGGTTACTTGGCTGGTTTCCTGGACAGAGAACATC
CAAGGTTCCATTAAATACATCATGCTTAACCCTAGTTCACGAATCAAGGGTGAGAAGGAC
TGGCAGAAATACGAGACTGCTCGGCGGCTGAAAAAATGTGTGGACAAGATCCGGAACCAG
TATCGAGAAGACTGGAAGTCCAAAGAGATGAAAGTCCGGCAGAGAGCTGTAGCCCTGTAC
TTCATCGACAAGCTTGCTCTGAGAGCAGGCAATGAAAAGGAGGAAGGAGAAACAGCGGAC
ACTGTGGGCTGCTGCTCACTTCGTGTGGAGCACATCAATCTACACCCAGAGTTGGATGGT
CAGGAATATGTGGTAGAGTTTGACTTCCTCGGGAAGGACTCCATCAGATACTATAACAAG
GTCCCTGTTGAGAAACGAGTTTTTAAGAACCTACAACTATTTATGGAGAACAAGCAGCCC
GAGGATGATCTTTTTGATAGACTCAATACTGGTATTCTGAATAAGCATCTTCAGGATCTC
ATGGAGGGCTTGACAGCCAAGGTATTCCGTACATACAATGCCTCCATCACGCTACAGCAG
CAGCTAAAAGAACTGACAGCCCCGGATGAGAACATCCCAGCGAAGATCCTTTCTTATAAC
CGTGCCAATCGAGCTGTTGCAATTCTTTGTAACCATCAGAGGGCACCACCAAAAACTTTT
GAGAAGTCTATGATGAACTTGCAAACTAAGATTGATGCCAAGAAGGAACAGCTAGCAGAT
GCCCGGAGAGACCTGAAAAGTGCTAAGGCTGATGCCAAGGTCATGAAGGATGCAAAGACG
AAGAAGGTAGTAGAGTCAAAGAAGAAGGCTGTTCAGAGACTGGAGGAACAGTTGATGAAG
CTGGAAGTTCAAGCCACAGACCGAGAGGAAAATAAACAGATTGCCCTGGGAACCTCCAAA
CTCAATTATCTGGACCCTAGGATCACAGTGGCTTGGTGCAAGAAGTGGGGTGTCCCAATT
GAGAAGATTTACAACAAACCCAGCGGGAGAAGTTTGCCTGGGCCATTGACATGGCTGAT
GAAGACTATGAGTTTTAG

In one embodiment of the method according to the present invention each of the time points at which said samples are obtained are at least 2-4 hours apart, and/or wherein the time points span a time period of at least 12 hours of the day, wherein preferably the time points are 4 hours apart, e.g. at 9h, 13h, 17h and 21h. The times can be chosen based on individual daily habits of the subject, such as the individual wake up time. For instance, for someone who usually wakes up at 11h, the sampling may start at 11h (and continue accordingly at 15h, 19h and 23h).

Previous studies focused on predicting the circadian time which means a 24 hours-rhythms. However, given that the prediction of the circadian time is in an application used for a second prediction, the prediction of the timing of behavior, the error accumulates with each prediction. The present invention instead relies on a direct measurement of the behavioral relevant timing directly based on the genetic expression. That means if the genes have a 20h, or 30h or 12h rhythm in expression, the method of the present invention would also be able to detect that. These would be non-circadian rhythms and include infradian and ultradian rhythms. Thus, the present invention assesses and monitors the rhythmic profile. The rhythmic profile could be a circadian or non-circadian rhythm.

According to the present invention "assessing the circadian or non-circadian rhythm" or "assessing the health status" also includes "monitoring the circadian or non-circadian rhythm" or "monitoring the health status". "Monitoring" means at least twice "assessing".

As an objective measure, gene expression may be quantified four times a day (the times mentioned in this disclosure serve as an e.g. of possible sampling times), two days in a row. In particular, four samples of saliva may be taken on two consecutive days, and the gene expression of selected genes in accordance with the present invention is determined in each of the samples. While other studies have focused on a prediction of circadian time (exact estimation of precise internal time), with the aim to allow for a subsequent prediction of the optimal time for a behavior, such as high sports performance, the present invention focuses on a direct prediction of the relevant timing including a circadian profile, without the deviation through circadian time. This means previous studies attempted to tell the exact internal time. The present invention provides a full 24h profile, it may provide a 48h profile, if measured during two consecutive days, each day e.g. 4 saliva samples are taken. If more samples are taken over more days longer profiles may be provided.

Subject matter of the present invention is use of a kit for providing the collected samples of saliva for the method of the present invention.

In an embodiment, the kit is used for sampling saliva for use in a method according to the method of the present invention comprising sampling tubes for receiving the samples of saliva, wherein each of the sampling tubes contains RNA protect reagent and is configured to enclose one of the samples of saliva to be taken together with the reagent, wherein preferably each of the sampling tubes is labelled with the time point at which the respective sample is to be taken and/or includes an indication about the amount of saliva for one sample.

The kit may further comprise at least one of a box, a cool pack, at least one form including instructions and/or information about the kit and the method for the subject.

In one embodiment of the kit the RNA protect reagent is selected from the group comprising. EDTA disodium, dihydrate; sodium citrate trisodium salt, dihydrate; ammonium sulfate, powdered; sterile water, wherein a single reagent or a combination of different reagents may be used.

In one embodiment of the kit said sampling tubes are configured to receive a sample of saliva of 1 mL in addition to 1 mL of the RNA protect reagent. The sampling tubes may be at least 2 mL tubes, preferably at least 3 mL tubes, more preferably at least 4 mL tubes, still preferably at least 5 mL tubes. While the size for the tubes of 2 mL would be sufficient, it may be more convenient for collecting the saliva samples if the tubes are bigger, such as e.g. 5 mL tubes. In order to collect the required number of samples, the kit may at least six sampling tubes, preferably at least eight sampling tubes (i.e. at least three and four, respectively, samples for two days).

While the kit is used for collecting the samples, it may be designed to be used also for storage and transport of the samples. For this purpose, it is advantageous to have a cool pack in the kit. For instance, if someone is outside and needs to collect the samples, the samples could be stored at room temperature anyway for a few hours, or if one know that there will be no fridge for the next two days, one could still freeze the cool pack before the sampling, then place the cool pack in the box and sample as needed, since the box would remain cold for several hours (maybe even for two days, depending on the outside temperature). After the sampling is completed, the same box can be used to send the samples back to a lab, if applicable with all the forms inside as well (it may be required to pack the box in a post box for sending, which however may be enough for preparing the kit to be sent).

**Detailed description of the invention**

Exemplary embodiments of the present invention will be explained by way of example with reference to the drawings. In the drawings:

Fig. 1 illustrates the circadian core-clock network;

Fig. 2 illustrates two examples of fits of saliva data to a core-clock mathematical model;

Fig. 3 illustrates time-course measurements of unstimulated saliva show fluctuations in gene expression across 45 hours;

Fig. 4 shows a scheme depicting molecular PK-PD of irinotecan (CPT11) (Dulong et al. 2015);

Fig. 5 illustrates a model extension (as an e.g. for the treatment with the drug irinotecan), in which the core-clock network is complemented with additional genes associated with the irinotecan metabolism and provides as an output cytotoxicity which can be taken to predict and optimize the time of treatment of cancer patients, for this particular drug. Similar model extensions are performed for each particular drug mentioned in the application;

Fig. 6 illustrates a 24h-period harmonic regression for experimental data from SW480 cell lines;

Fig. 7 illustrates a simulated gene expression, obtained with the mathematical model, fitted to experimental data from SW480 cell lines;

Fig. 8 shows a schematic representation for the rationale of cancer chronotherapy, applying the described experimental and computational methods;

Fig. 9 illustrates an example for a personalized model fit of core-clock genes (a) and the genes involved in the irinotecan metabolism (b) based on the experimental data. The personalized times for the particular individual (meal timing, sleep and sleep/awake times are marked for better interpretation of the results);

Fig. 10 illustrates the computed prediction result for the temporal dynamics of two additional genes involved in the irinotecan metabolism (a) the optimal treatment time with irinotecan (b) based on the gene expressions from Fig. 9 which can be taken to predict and optimize the time of treatment of cancer patients, for this particular drug. Similar model extensions are performed for each particular drug mentioned in the application;

Fig. 11 illustrates an example for a core-clock network extended with Irinotecan-treatment relevant genetic network, which can be taken to predict and optimize the time of treatment of cancer patients, for this particular drug. Similar model extensions are performed for each particular drug mentioned in the application;

Fig. 12illustrates how BMAL1- and HKDC1-KD leads to metabolic changes in SW480 colorectal cancer cells and altered drug response in a time-dependent manner; see also The Circadian Clock Regulates Metabolic Phenotype Rewiring Via HKDC1 and Modulates Tumor Progression and Drug Response in Colorectal Cancer. Fuhr L, El-Athman R, Scrima R, Cela O, Carbone A, Knoop H, Li Y, Hoffmann K, Laukkanen MO, Corcione F, Steuer R, Meyer TF, Mazzoccoli G, Capitanio N, Relógio A*. EBioMedicine. 2018

Fig. 13 illustrates how BMAL1 promoter activity is altered after treating human cancer cells with different anti-cancer drugs;

Fig. 14 illustrates how BMAL1 promoter activity is altered after treating different human cancer cells with anti-cancer drug Irinotecan;

Fig. 15 illustrates how cell proliferation for an exemplified for a human cancer cell type is affected by Cisplatin treatment in a time-dependent manner;

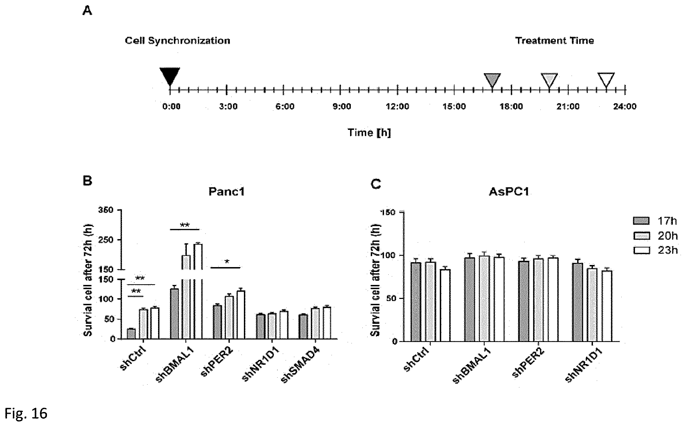

Fig. 16 illustrates how cytotoxicity assays for time-dependent gemcitabine treatment for two different types of human pancreatic cancer cell lines. The downregulation of core-clock genes and the alteration of SMAD4 expression impacts on drug response in Panc1 and AsPC1 cells. Cytotoxicity assays were performed using either an IncuCyte® Red Cytotoxicity Reagent or NucLight Rapid Red Reagent for the IncuCyte S3 Live Cell System Analysis. (A - C) 0h after cell synchronization, gemcitabine was dissolved in IncuCyte® Red Cytotoxicity Reagent and added into PDA cells containing SMAD4-KD, -OE and corresponding empty vector constructs (Panc1, $9.5\mu M$ and AsPC1, $23.9\mu M$). A cytotoxic index was calculated on IncuCyte S3 Live-Cell Analysis System in red phases. 72h after treatment, the number of viable cells per well was quantified with IncuCyte S3 Live-Cell Analysis System. Compared to the shCtrl (mean $\pm$ SEM, n = 6, one-way ANOVA, *p < 0.05, **p <0.01, ***p <0.001).

Fig. 17 illustrates BMAL1 and PER2 expression display variation during the day in human blood, hair and saliva samples.

Fig. 18 illustrates Gene expression of BMAL1 and AKT 1 covary.

Fig. 19 illustrates HST base line measurements.

Fig. 20 illustrates Myotonometric analysis shows daily variation in muscle tone (frequency, F) for female and male participants.

Fig. 21 illustrates Standard deviations of normalized sports and muscle tone data

Fig. 22 illustrates saliva RNA extraction optimization results. Saliva was collected from several healthy participants at 1pm with different ratios between saliva and RNAprotect reagent. Following ratios were used: 1) 1:1 with 1.5mL saliva; 2) 1:2 with 1.0 mL saliva; 3) 2:1 with 1.0 mL saliva; 4) 1:2 with 0.5 mL saliva. Subsequently, RNA was extracted and RNA concentration was measured. Best RNA yield was achieved by using a 1:1 ratio with 1.5 mL saliva for the majority of participants.

Fig. 23 illustrates time-course saliva RNA concentration results from healthy participants. Using a 1:1 ratio between saliva and RNA protect reagent, 1.5 mL saliva was collected at several time-points per day for two consecutive days in two healthy participants, followed by RNA extraction and saliva RNA concentration measurement. In both participants and at all time-points, saliva RNA concentration was above the minimum of 20 ng/$\mu$L, which is required for subsequent RT-PCT analysis for at least four genes.

Fig. 24 illustrates time-course core-clock gene expression using saliva in healthy participant. From participant A, saliva was collected at several time-point per day (9h, 13h, 17h and 21h) using a 1:1 ratio with 1.5 mL saliva. Subsequently, RNA was extracted followed by RT-PCR detecting core-clock genes CLOCK, NPAS and NR1D1. The results show variations in the expression of core-clock genes throughout the day.

Fig. 25 gives an overview over an exemplary model for toxicity prediction and the data on which this model was applied. (A) Irinotecan administration (left) induces a complex interaction between the drug, the core-clock and additional relevant genes and proteins captured by our model (middle), which eventually leads to DNA damage and potentially cell death (right). (B) The model is fitted to mouse liver tissue (representing mammalian healthy cells), and two cell lines derived from the same patient, from the primary colon cancer (SW480) and from the metastatic site (SW620).

Fig. 26 illustrates the transcription-translation model for predicting irinotecan toxicity (antineoplastic enzyme inhibitor).

Overview over the positive (pointy arrow) and inhibitory (arrow with non-pointy head) connections in the irinotecan model combining core clock, irinotecan-relevant genes and irinotecan pharmacokinetics and -dynamics.

Fig. 27 illustrates oscillations of core-clock genes and clock-controlled genes. Circadian gene expression profile of core-clock and irinotecan PK-PD-relevant genes for the SW480 cell line (dots) fitted with a harmonic regression (line).

Fig. 28 illustrates clock-irinotecan model fitted to the SW480 cell line. Circadian gene expression profile of core clock and irinotecan PK-PD-relevant genes for the SW480 cell line (dots) fitted by the transcription translation network shown in Fig. 26.

Fig. 29 illustrates the fit of the clock-irinotecan model, comparing the fitted clock-irinotecan network for healthy and cancer-derived cells (colorectal cancer). Selected gene expression (dots) and fit of the clock-irinotecan network (lines) for (A) liver data, (B) SW480.

Fig. 30 illustrates the fit of the clock-irinotecan model, comparing the fitted clock-irinotecan network for healthy and cancer-derived cells (colorectal cancer). (A) Selected gene expression (dots) and fit of the clock-irinotecan network (lines) for SW620. B: Comparison of the model output when fitted to liver (dash-dotted line), SW480 (full line) and SW620 (dashed line).

Fig. 31 illustrates irinotecan toxicity for different treatment times. Predictions for time-dependent treatment from human cancer cell lines and from the model. (A) Left: Experimentally measured cytotoxicity curves for SW480 cells treated at different timepoints with irinotecan (6h, 12h, 18h or 24h after synchronization) or untreated (No Drug). Right: For the cytotoxicity curve shown left, the area under the curve of treated cells normalized by the untreated case is plotted with dots, compared with the cell death predicted by the model (grey line). (B) Same data as (A), but for SW620 cells.

Fig. 32 illustrates irinotecan toxicity under alterations. Comparison of toxicity profiles for different conditions. (A) Comparison of CRC cell lines (SW480 full line, SW620 dashed line) and liver tissue (dashed-dotted line). (B) Comparison of the model fitted to the SW480 cell line (full line) with the same model but half the UGT1A1 protein level (short dashes) or double the protein level for all ABC transporter (longer dashes). (C) Increasing the maximal transcription rate of PER2 from 90% (full line) to 110% (short dash-dotted) leads to consistent changes in the toxicity phase.

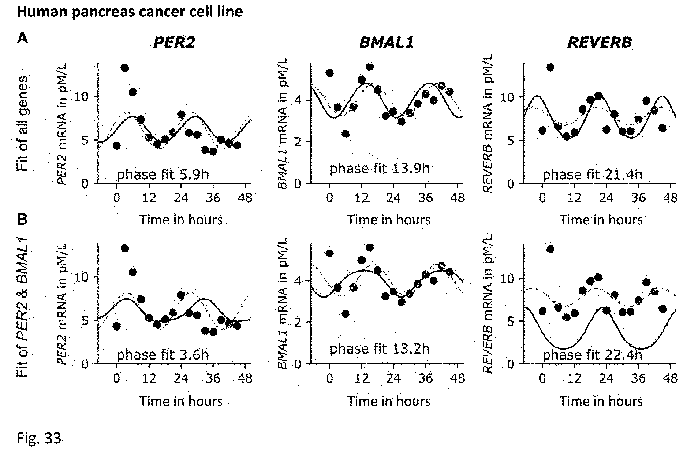

Fig. 33 illustrates a fit of the network model to a pancreas cancer cell line derived from a patient (ASPC1). (A) 48 hours time-course of gene expression for *PER2, BMAL1* and *REV-ERBα* for ASPC1 cell line measured via RT-qPCR, multiplied by the Liver concentration of *GAPDH* for consistent units (dots). The harmonic regression of the data (dashed line) resembles the fit by the mammalian network model (straight line). (B) Restricting the fit to only *PER2* and *BMAL1*, the phase for *REV-ERBα* is predicted with only one hour of error compared to the phase derived by also fitting *REV-ERBα*. Harmonic regression (dashed line) and model fit (straight line).

Fig. 34 illustrates the gemcitabine PK-PD model used to predict the cytotoxicity profile of the pancreas cancer cell line in response to time-dependent treatment with gemcitabine.

Fig. 35 illustrates for the pancreas cancer cell line ASPC1 the profile for the number of living cells in result to gemcitabine treatment predicted by the gemcitabine PK-PD model (line) in comparison to the experimental data (dots).

Fig. 36 illustrates the toxicity prediction for irinotecan treatment at the example of two healthy human subjects. The gene expression of *BMAL1* and *PER2* extracted from saliva (dots) is fitted by the mammalian transcription-translation network (lines) and the resulting periods and phases of relevant genes are used to predict cell death (right panels).

Fig. 37 illustrates the toxicity prediction for irinotecan treatment for a set of healthy human subjects. The gene expression of *PER2* (first row) and *BMAL1* (second row) extracted from saliva (dots) is fitted by the mammalian transcription-translation network (lines) and the simulated gene expression of *UGT1A1* (third row), *CES2* (fourth row) and REV-ERBA (fifth row) is used to predict irinotecan toxicity profiles (sixth row) and gemcitabine toxicity profiles (seventh row).

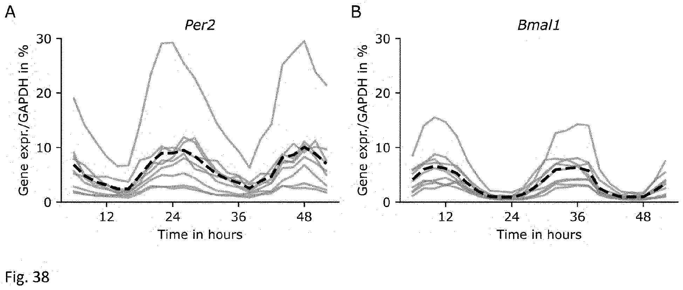

Fig. 38 illustrates the similarity of circadian oscillations in different mammalian tissues at the example of the circadian oscillation in *Per2* and *Bmal1* gene expression. Straight lines connect experimental measurements of aorta, adrenal gland, brown fat, heart, kidney, liver, lung skeletal muscle, and white fat over 48 hours, dashed curve is the resulting mean over tissues, representative of entrained mammalian tissue. Mouse were entrained by a 12:12 light:dark cycle and 12h before timepoint 0h released into constant darkness. Based on data first published by Zhang *et al.* 2014, accession numbers GSE54650 and GSE54652 [9].

Fig. 39 illustrates that saliva samples of representative healthy subjects (black dots) show similar trends as mammalian tissue (dashed). Timepoint 0h corresponds to the mean wakeup time for subjects, and for the mammalian data to the start of the first activity period during constant dark.

Fig. 40 illustrates the similarity of the toxicity predictions of the original model by Dulong et al. 2015 (straight line), which fitted the colorectal cancer cell line CACO2, and the prediction of the mammalian data (dashed line), when using the same modelling approach as for the saliva samples from healthy subjects.

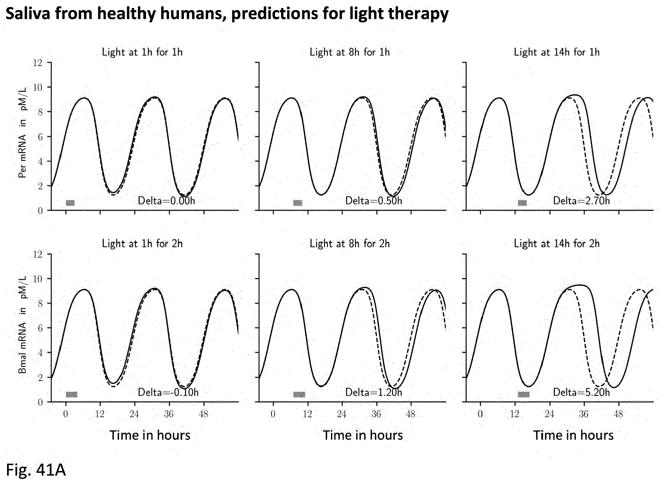

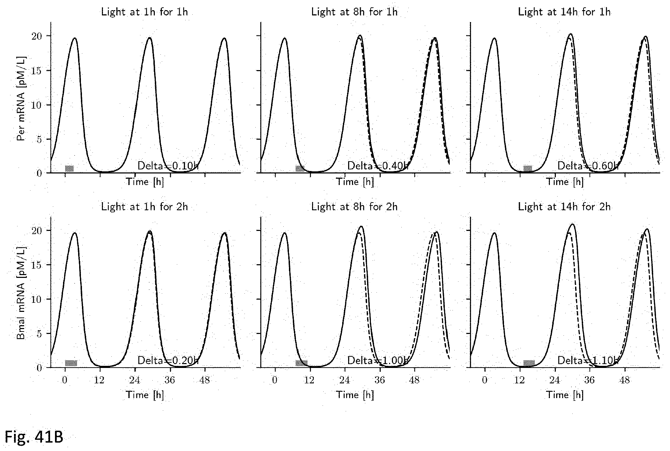

Fig. 41 illustrates that light therapy can induce changes in circadian gene expression in the mammalian core-clock model fitted to subject 6 (A) and subject 15 (B). Depending on light therapy starting time and duration, vastly different responses in the circadian rhythms (shown is BMAL1 expression) are observed, and in addition treatment results depend on individual gene expression profiles. Grey bar marks light treatment, light therapy is implemented as a transient increase in *PER2* transcription. Delta is the time difference between the phase expected without light treatment, and the phase observed with light treatment.

Fig. 42 illustrates cortisol levels and gene expression for one representative subject. Shown are experimental measurements as dots, and harmonic regression fits in interrupted lines with a period of 24 hours (p=3 * $10^{-6}$ for cortisol, not significant for gene expression). Sampling 1 and Sampling 2 were done with 3 months in between, the resulting gene expression profiles can show seasonal effects.

Fig. 43: Temporal mean of BMAL1 (A) and PER2 (B) expression versus melatonin values, considered for sampling day 1 and 2 separately. Coefficient of determination for BMAL1 is 0.05, coefficient of determination for PER2 is 0.87. Maximal gene expression of BMAL1 (C) and PER2 (D) versus melatonin values, considered for sampling day 1 and 2 separately. Coefficient of determination for BMAL1 is 0.04, coefficient of determination for PER2 is 0.69.

Fig. 44: Circadian time prediction. A Circadian period derived from the best fit of a cosinor analysis to PER2 with periods between 20h and 28h. B Due to different circadian periods, subjects pass their subjective 23h at different times of the day.

Fig. 45/46: Harmonic regression to cortisol values and gene expression using a period as predicted by the PER2 optimal period, the circadian period shown in Fig. 43.

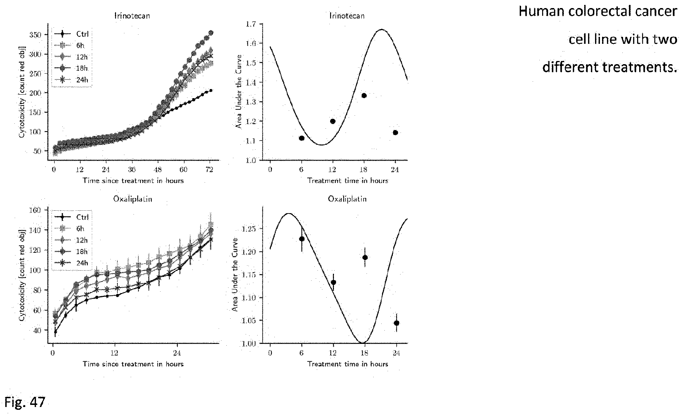

Fig. 47: Cytotoxicity as measured by Incucyte for HCT116 cells treated with Irinotecan (top row) and Oxaliplatin (bottom row), and the corresponding Area Under the Curve (AUC) values for different treatment times, normalized by the control case. The temporal variation in the AUC is predicted by the toxicity profile of computational models.

As indicated in Figure 41 the present methods may also be used to show how the circadian profile of a patient or subject looks like and in one other embodiment (e.g. if problems in the circadian profile are detected), the methods and models of the present invention may be used to decide on the point of time to apply a measure or therapy to induce the clock, e.g. by light therapy or administration of melatonin. Such a measure or therapy may make the clock of the patient or subject more robust and could improve well-being.

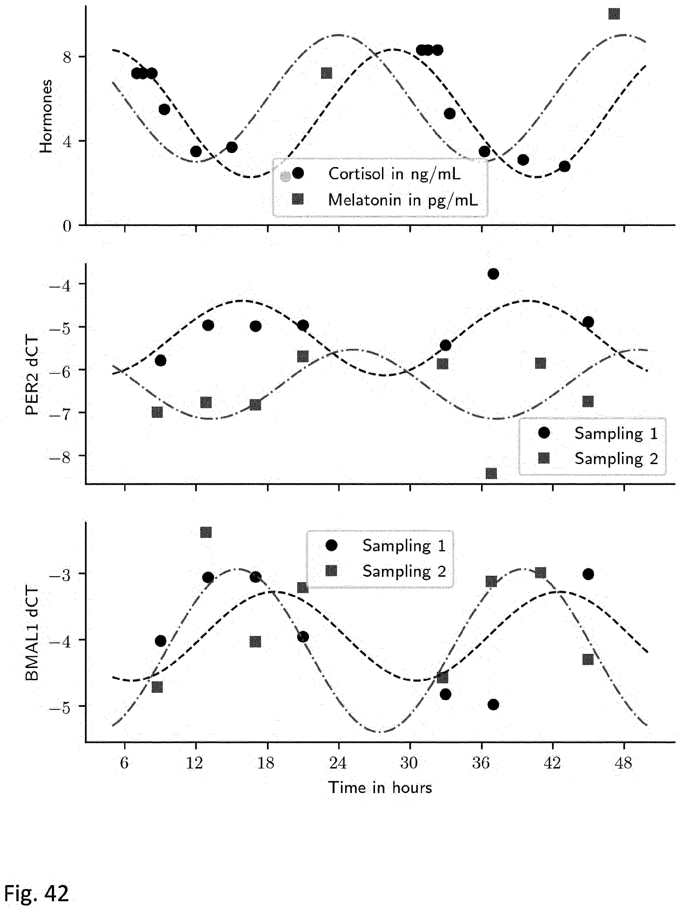

The aim of the invention is to predict, optimal timing of behavior, more specifically the timing of cancer treatment, possibly to monitor (over time) the circadian profile and adjust timing if needed. Previous studies focused on predicting the circadian time. However, given that the prediction of the circadian time is in an application used for a second prediction, the prediction of the timing of behavior, the error accumulates with each prediction. The present invention instead relies on a direct measurement of the behavioral relevant timing directly based on the genetic expression. Fig. 42 illustrates gene expression profiles for one subject and two sampling times three month apart. BMAL1 peaks in the early evening hours about 12 hours after wakeup, while PER2 seems to be more sensitive to seasonal effects. *BMAL1* gene expression seems to be in antiphase with hormonal cortisol levels, which is informative about circadian time.

Thus, in addition to the core-clock genes levels of cortisol and/or melatonin may be used and fitted into the methods and models according to the invention. Cortisol or melatonin hormone levels were measured using commercial kits from cerascreen (Cortisol Test and Melatonin Test Kits) and by providing saliva samples at different times of the day (Cortisol Test Kit) or before sleep (Melatonin Test Kit) according to the manufacturer's instructions. Samples were sent to cerascreen laboratory for the detection of hormone levels (via immunoassay, e.g. radioimmunoassay or ELISA) and results were provided after the analysis.

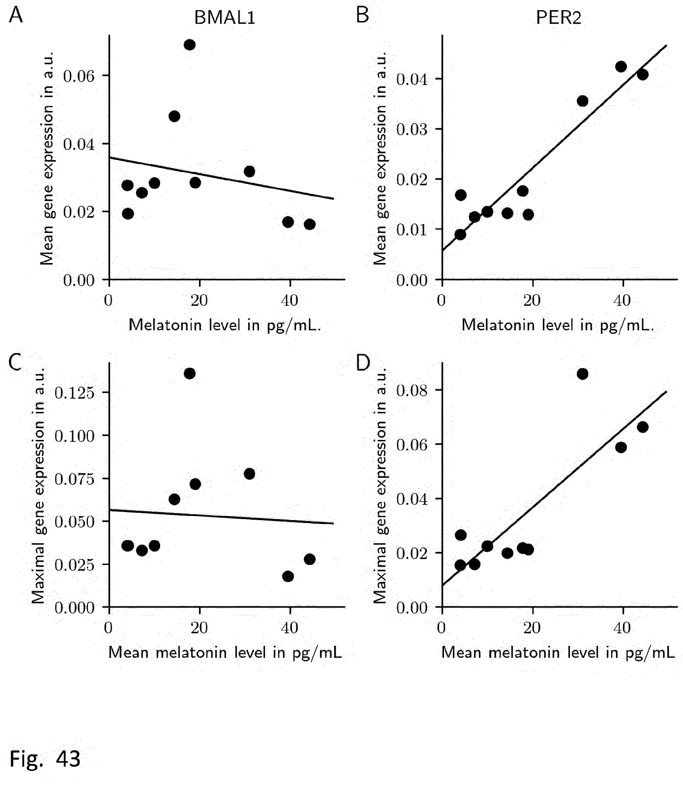

The expression profile allows to relate gene expression to melatonin levels. The coefficients of determination from Figure 43 suggest no correlation for melatonin with BMAL1, but a correlation between PER2 mean expression and Melatonin level, and potentially a weaker correlation between PER2 maximal expression and melatonin level. This relates a saliva derived gene-based measure with a hormonal level set by the central clock in the SCN.

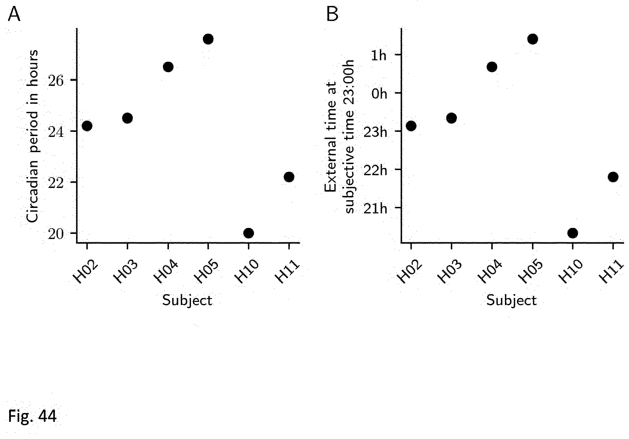

The circadian profile extracted from the saliva samples is also applicable to predict circadian time, see Figure 44.

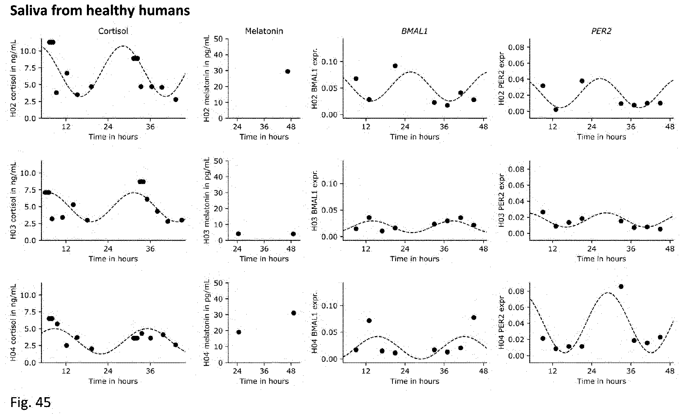

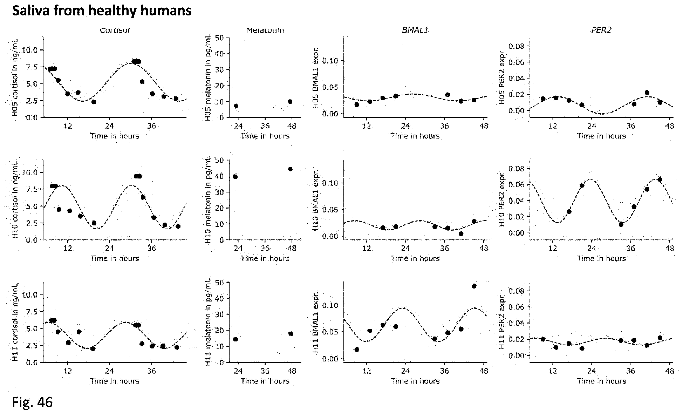

Because of the correlation between melatonin and PER2, according to the invention PER2 may be used to derive the circadian period of the subject, see Figure 44A, from the optimal period to fit PER2 gene expression with a harmonic regression. The circadian profile extracted from the saliva samples is also fit to predict circadian time based on the derived period, see Figure 44B-C. Using the individual circadian periods, the hormonal and gene expression profiles may be fitted by harmonic regressions, see Figures 45 and 46. This may be used as a test whether the extracted period of the subject indeed fits all its circadian profiles.

As an objective measure, gene expression may be quantified four times a day (the times mentioned in this disclosure serve as an e.g. of possible sampling times), two days in a row. In particular, four samples of saliva may be taken on two consecutive days, and the gene expression of selected genes in accordance with the present invention is determined in each of the samples. While other studies have focused on a prediction of circadian time, with the aim to allow for a subsequent prediction of the optimal time for a behavior, such as timing of administering a certain medicament, the present invention focuses on a direct prediction of the relevant timing, without the deviation through circadian time.

The computational analysis of the measured gene expressed obtained from the saliva samples as set forth above can be separated into three different approaches, which will be discussed in the next sections. First, experimental data can be fitted with a periodic function, in order to establish oscillatory behavior and extract oscillation properties. Machine learning can then be used to predict the timing of behavior based on the gene expression. Modeling the molecular network underlying the circadian rhythm as well as the behavior under consideration can add information. Background and general considerations in view of the present invention and the overall process will be explained first. Following, the procedure according to the present invention will be described with respect to the specific application.

A general problem in chronobiology is the screening for circadian oscillations in data, such as in the series of eight data points obtained from the saliva samples. It has to be determined whether the observed variation is due to some circadian rhythm, or only due to noise. To distinguish oscillating from non-oscillating measures, a periodic, non-constant function is fit to the data, and if the fit is significant, the measure is considered oscillatory. Successful fits allow to read off the oscillation phase, amplitude and period. Fitting the oscillatory data by curve fitting is described below.

If a trigonometric function is fit to the data, this is called harmonic regression, which may be done as set forth in the following. It will be appreciated that this is described by way of example only. Below, other approaches are briefly outlined. Circadian rhythmicity of genes may be tested (significance e.g. bounded by a fit with p-value < 0.05) and circadian parameters (phase and relative amplitude) may be determined for sample sets with at least 7 data points (3 hours sampling interval) for a period range of 20 to 28 hours with a 0.1 hour sampling interval by fitting a linear sine-cosine function to the time-course data ($\Delta\Delta$CT normalized to the mean of all time points), for instance using known tools, e.g. the R package HarmonicRegression (Lück et al. 2014). The harmonic regression procedure fits the model y(t) = m + a cos(cot) + b·sin(cot) in order to estimate absolute amplitudes (A = $\sqrt{(a2 + b2)}$) and phases ($\varphi$ = a·tan2(b,a)) along with confidence intervals and p-values (Lück et al. 2014). The fit uses a least-squares minimization. Extensions to this fit method are reviewed in as cosinor-based rhythmometry in (Cornelissen 2014).

A combination of sine waves are also used by other rhythmicity detection methods (Halberg et al., 1967; Straume, 2004; Wichert et al., 2004; Wijnen et al., 2005; Thaben and Westermark, 2014). Yet, Fourier-based methods can have the drawback that they require evenly sampled data. Other alternatives are named in the following. It will be appreciated that the invention is not limited to these packages but any other suitable method for fitting a periodic function to the measured gene expression data may be applied.

The software-packages RAIN (a robust nonparametric method for the detection of rhythms of prespecified periods in biological data that can detect arbitrary wave forms (Thaben and Westermark 2014)), which improves on older methods: a nonparametric method implemented as the program "JTK_CYCLE", which assumes symmetric curves (Hughes et al., 2010), as well as its improvement eJTK_CYCLE that includes multiple hypothesis testing and more general waveforms (Hutchison et al. 2015)[Ref: Hutchison AL, Maienschein-Cline M, Chiang AH, et al. Improved statistical methods enable greater sensitivity in rhythm detection for genome-wide data. PLoS Comput Biol 2015;11:e1004094.], while the HAYSTACK method (Michael et al., 2008) can also detect chain saw type rhythmicity, but relies on a small set of predefined wave form alternatives and is thus not really general.

BIO_CYCLE: "We first curate several large synthetic and biological time series datasets containing labels for both periodic and aperiodic signals. We then use deep learning methods to develop and train BIO_CYCLE, a system to robustly estimate which signals are periodic in high-throughput circadian experiments, producing estimates of amplitudes, periods, phases, as well as several statistical significance measures." (Agostinelli et al. 2016).

A modified version of the empirical Bayes periodicity test to detect periodic expression patterns (Kocak and Mozhui 2020). Their results demonstrate that this approach can capture cyclic patterns from relatively noisy expression data sets.

Especially to find higher harmonics, some studies have exploited Fisher's G-test and COSOPT jointly to recognize rhythmic transcripts, classified, depending on the length of the oscillation period, as circadian (24 $\pm$ 4 h) and ultradian (12 $\pm$ 2 h and 8 $\pm$ 1 h) (Hughes et al. 2009; Genov et al. 2019).

Once the curve fitting to the measured data points has been done, machine learning methods are applied to predict circadian time for human subjects, which has in principle been proposed by several studies. Some example studies are outlined to provide background for the process of the present invention, which will be described in detail thereafter.

While the present invention focusses on studies based on gene expression, continues measures of light exposure and skin temperature as well as metabolites from blood or breath sampling can be used to predict circadian time (Kolodyazhniy et al. 2012; Kasukawa et al. 2012; Sinues et al. 2014). Also skin temperature in combination with questionnaires and activity measurements can predict circadian time, by a method called INTime (Komarzynski et al. 2019). The following studies predict circadian time or time-of-the-day from gene expression data extracted from human blood: BioClock (though only mouse data so far) (Agostinelli et al. 2016): Normalization is Z-score data (subtraction of mean and then divided by standard deviation - this removes any amplitude information), their method is a deep neural network, they use BioCycle to derive rhythmicity, and standard gradient descent with momentum to train the network, the original publication uses different tissues but only from mice.

ZeitZeiger (Hughey 2017): Data normalized and batch-normalized. Discretized and scaled spline fits are used to calculate sparse principal components (SPC), predictions based on fitted splines to SPC with maximum likelihood. They use 15 genes from human blood, only two of those are part of the core-clock. Due to the batch-normalization, the incorporation of new data requires retraining of the algorithm, their algorithm was improved for humans. One sample is enough for predictions.

Partial least squares regression (PLSR) (Laing et al. 2017): Training data is batch-corrected and quantile normalization is applied. No batch correction on test set, to prevent the need for retraining whenever new data is added. Their algorithm uses 100 genes out of 26,000 available ones from blood. One sample is enough for predictions, more is better.

TimeSignature (Braun et al. 2018): Mean-normalized genes, algorithm is optimized with a least squares approach plus elastic net for regularization. They use 40 genes from two samples of blood, 12 h or less apart. This study seems to generalize well, it was validated in 3 studies, one of them with a different experimental method to measure gene expression.

Body Time (Wittenbrink et al. 2018): ZeitZeiger (see above) + NanoString platform (an experimental machine which allows for high quality counts of gene abundance without the need of an amplification step, thus it measures the original abundances). They use 12 genes from human blood, but also get good predictions for as few as 2 genes (one of which is PER2 which also is used in the sports study). Their algorithm is validated in 1 independent study that uses the same method.

TimeTeller, preprint (Vlachou et al. 2020): They aim to predict clock functionality from a single gene sample. Application to breast cancer, showing that their prediction relates to patient survival. Rhythmicity and synchronicity were analyzed to choose a set of 10-16 genes used for the prediction (all core-clock or clock-controlled). Their algorithm is trained with a set of repeated samples and extracts from them the probability to observe a particular gene expression profile given some time t. The prediction inverts this information; they use a maximal likelihood function to predict for a given gene expression profile the time t. A model of the core-clock was used to test their algorithm.

Machine learning can be used to predict some output based on a (high-dimensional) input consisting of a set of so-called features, i.e. the different dimensions of the input space. A set of input-output pairs is used to train the algorithm, i.e. the algorithm performs some kind of optimization that allows it to optimally predict the output based on the input. This set is called training set. To evaluate the performance of the algorithm, it is fed with an independent set of inputs from a so-called test set, while not presenting the associated outputs. The predictions of the algorithm are then compared to the associated outputs, and the number of correct predictions is counted. Several measures can be used to quantify prediction quality; for instance the accuracy, i.e. the number of correct predictions divided by the total number of predictions, may be used.

For small data sets, as typical with human subjects, the separation of the data into two independent training and test sets means that it would not be taken advantage of the full amount of information available for the prediction. The solution is cross-validation, for which the total set is repeatedly separated into different training and test sets. Especially for very small data sets, one can use all but one subjects to form the training set, and test the algorithm only on the left-out subject. This is called leave-one-out cross-validation (sometimes also leave-one-subject-out cross-validation). Given n subjects, the training on all-but-one subjects is repeated n times, such that each subject has been once selected as test set. The accuracy of the prediction is in this case calculated as the number of correct predictions over n.

While the application of machine learning to genetic data is generally known, the benefits and value of the results highly depend on the input data. Thus, the inventors put their focus on the input of the data, both via normalization and presentation of derived features, and also on understanding in detail what information the algorithm uses. Most published studies focus on their machine learning algorithm, why it is best suited for the prediction at hand, while mentioning their data preparation only on the side, and their discussion of the workings of the algorithm is often restricted to a single evaluation approach (for example, showing that a restricted set of genes is most relevant for prediction, but without stating which characteristics of the genes are important).

When comparing the performance of different machine learning algorithm on standard training and test sets, their performance differs often just in a few percent - an order of magnitude hardly relevant for biological data, which often consists of only few samples with a high level of noise compared to other typical machine learning applications. It has been found that the particular algorithm will not make much difference, but what makes a large difference is the way in which the input is prepared for machine learning. Many machine-learning algorithms are optimized for data with zero mean and a standard deviation of one for each dimension individually. Dimension-wise normalization makes however no sense for time-series data, where dimensions are not independent of each other, but where the information on the temporal development can only be accessed by comparing different dimensions.

As mentioned above, eight saliva samples may be collected, preferably distributed over the day, e.g. at 9h, 13h, 17h and 21h over two consecutive days. This results in a feature space with 8 dimensions. Instead of normalizing each of these dimensions independently over all subjects, the data may be normalized by their temporal mean for each subject independently. In addition, this subject-wise mean normalization of each gene has the advantage of keeping the temporal structure of the data intact (phase and relative amplitude of the oscillation), thus preserving this information for the machine learning algorithm. Yet, what is lost by this normalization is the mean values of the oscillations, and thus also their relative expression mean. To preserve this information for the machine-learning algorithm, this may be added as additional features to the feature space. Subject-wise normalization has to be reconsidered when the molecular profile of a subject was measured repeatedly over a longer interval of time. For the example of multiple measurements during disease progression, we have already published one possibility to normalize data from subsequent molecular profiles such that the result can be compared between sampling dates and even between different experimental procedures (Yalçin et al. 2020).

In many cases, machine learning algorithms are considered as "black boxes". However, as the machine learning algorithms are faced with noisy biological data, derived of a system where lots of additional information are available, the approach of the present disclosure is to let the machine learning optimize the prediction, but then to uncover the underlying information flow from input to prediction output, with the aim to double-check the generalizability of the solution found by the machine learning. Optimally, any additional information can be added as either input to the machine learning or as constraints (in form of a cost function), but in a first step, the formulation of these inputs and constraints is more difficult than to take the algorithm and check a posteriori whether any additionally known information is violated.

Once the prediction was done using the complex feature space created in the step explained above, a simplification of the feature space may be carried out. This serves to identify the relevant features. For example when predicting the optimal treatment time it may be tested whether the peak time of the genes would suffice for the prediction. It was found that this was not the case, i.e. the algorithm uses more than this information. In general, dimensionality reduction methods may be used first, which results in fewer, new features that are combinations of the original features. Then it may be tested which combinations of individual original features is sufficient for successful predictions, and compare whether that fits the features which are dominant in the features resulting from dimensionality reduction. This is an important step to understand based on which information the prediction is made by the algorithm, which is relevant to double check its generalizability to new data.

Related to the first point, machine-learning algorithms are preferred which may be called interpretable, i.e. they provide some information on the prediction. Examples for such algorithms are sparse principle components analysis as used as an intermediate step in (Hughey 2017), and partial least squares regression, as used in (Laing et al. 2017). In both cases, the prediction is made based on a combination of the features into few most informative features, and it is for example possible to plot two of them against each other in order to see how the data of the training set and test set is distributed in these features. It is expected that subjects with optimal times that are neighboring are also neighbors in this component space. If this is not the case, the algorithm is unlikely to generalize well.

Then, prediction performance may be benchmarked using a neural network model, which may be used as an approximation for an upper border of prediction performance. Neural networks do not require normalization, as they are universal computing machines and can hence implement the optimal normalization for the problem at hand on their own. However, this is at the same time the problem with neural networks. As they decide for themselves which are the relevant features of the data, there is no controlling whether they use biologically relevant information, or noise information that - by chance - fits the prediction. Furthermore, their high flexibility facilitates overfitting of the data and the resulting algorithm are difficult to interpret, such that we cannot a posteriori enhance our trust in the method by understanding the information flows from input to prediction output. Despite these disadvantages, neural networks may be used at least as benchmark algorithms, to test which performance can be expected when the information is provided without constraints. The present invention aims at providing an algorithm with a performance similar to that of the neural network, but not by means of overfitting the experimental data, as suspected for the neural network, but by means of focusing on the biologically relevant information.

A linear support-vector-machine (SVM) can be used to predict two different outputs based on a high-dimensional input data (see below for details). Linear SVMs are extremely simple compared to the non-linear methods explained above. They have the advantage of a fast implementation, and, as their complexity is low, they are not so prone to overfitting. For these reasons, a linear SVM may be used to predict the optimal treatment timing, and it turned out that this was sufficient for prediction. However, it is noted that the prediction problem was "linearly separable", and as there is no reason to assume that any application is "linearly separable", it may be preferable to use in general non-linear methods. Yet, testing how well a linear model performs compared to the non-linear model can help to benchmark how much complexity is needed for the prediction. For example, if a linear model results in an accuracy of 0.85 and a non-linear model in an accuracy of 0.9, it is probably not worth using the non-linear model for the application, as it performs only slightly better on the test set, but has a larger probability of overfitting the data, which might lead to less performance on a new set of data. If the difference is larger, a non-linear model is likely more appropriate.

As mentioned above, linear support-vector-machine (SVM) can be used to predict two different outputs based on a high-dimensional input data. For training, the linear SVM is fed with multidimensional input data and a binary output. The training set consists of n subjects, and the input with $p$ dimensions is denoted as $x_i \in R^p$, \$i . The output $y_i$ is encoded as -1 for the first type of output and as +1 for the second type of output, $y \in {1, -1}^n$. The training of the SVM fits a hyper-plane into the input space such that it separates the two output types as best as possible and such that the distance to the input data points is maximal.

Mathematically, the following minimization problem is solved:

$$\min_{w,b} \frac{1}{2} w^T w + C \sum_{i=1} \max\left(0, y_i(w^T \phi(x_i) + b)\right),$$

where $\phi$ is the identity function, $(w^T\phi(x_i) + b)$ is the predicted output for the ist input.

Predictions for some input $x_{test}$ then be calculated as $w^T\phi(x_{test}) + b$ with the w and b resulting from the above minimization, and compared with the correct output. Leave-one-subject-out cross-validation implies that this step is repeated n times, each time with another participant removed to form the training set.

The sample collection can be performed in almost any location. The samples of saliva are collected at the predetermined points in time in a tube containing an RNA stabilizing reagent followed by RNA extraction as described below. In order to minimize RNA degradation through material transfer, according to a preferred exemplary method an amount of 1mL of unstimulated saliva may be collected directly into a 5mL Eppendorf tube containing 1mL of a non-toxic RNA-stabilizing reagent called RNAprotect Tissue Reagent (Qiagen) which should be mixed immediately to stabilise the saliva RNA. The direct addition of saliva to the RNA stabilizing reagent, which is mixed immediately, was found to generate good quality/quantity RNA suitable for gene expression analysis and by using 5mL tubes instead of 2mL tubes (wider opening for sample collection), the sampling procedure was more comfortable to perform. Other tested sampling protocols had shown to lead to poor quality and quantity of RNA that was not suitable for the downstream application. For example, 200μL saliva was collected in a 50mL tube on ice, which was immediately transferred to a 2mL tube containing 1mL RNA stabilizing reagent followed by RNA. In another protocol, 1000μL saliva was collected in a 50mL tube and processed as described above, in which the extracted RNA did not pass the desired quality/quantity either.

With only four sampling time-points per day (9am, 1pm, 5pm and 9pm) and over two consecutive days, it is possible to assess precise circadian rhythms in gene expression of any individual. For best sampling quality, the individuals should refrain from eating and drinking one hour prior to sample collection. Individuals can optionally wash their mouths with water five minutes before sampling without swallowing the water. The stabilized samples can be kept at room temperature for a few days, optimally at 4°C during several weeks, for posterior molecular analyses via different possible methods, such as RT-qPCR, Nanostring, microarrays, and sequencing.

In one embodiment any other method known by a person skilled in the art to measure gene expression could be used. One could of course do the same with protein expression instead of gene expression in principle.

A method for RNA extraction from saliva samples is provided to effectively extract RNA, preferably using TRIzol (Invitrogen, Thermo Fisher Scientific) and the RNeasy Micro Kit (Qiagen). It has proven to be particularly beneficial to use a combination of both, rather than only one of them (typically either TRIzol or RNeasy Kit is used). For this, the samples were centrifuged at 10,000 x g for 10 min at room temperature to generate cell pellets. The supernatant was removed and the pellets were homogenized with 500μL TRIzol followed by the addition of 100μL chloroform and mixed for 15sec at room temperature. After a 2min incubation at room temperature, the samples were centrifuged at 12,000 x g for 15min at 4°C. The mixture will separate into a lower red phenol-chloroform phase, an interphase, and a colourless upper aqueous phase. The upper aqueous phase contains the RNA, which was transferred into a new 2.0mL microfuge tube using a 1ml pipette with filtered tip, being careful not to transfer any of the interphase layer. After the transfer, the samples were processed according to the manufacturer's instructions of the RNeasy Micro Kit (Qiagen) on a QIAcube Connect device (Qiagen). Finally, the RNA is eluted in RNA-free water and can be used directly for gene expression analysis. It has been developed the secondary purification and elution step of the saliva RNA using RNeasy Micro Kit in order to:

a) Increase sample quality and purity, which is necessary for downstream applications and is otherwise lost with the traditional and commercial methods, since the RNA content in saliva is low.

b) Automate the sample processing and RNA extraction using the QIAcube Connect automation device. With this, it is possible to perform sample handling much faster and reduce sample contamination induced by human errors.

In one embodiment, gene expression analysis is carried out via cDNA synthesis and RT-PCR as follows. For RT-qPCR analysis, the extracted RNA is reverse transcribed into cDNA using M-MLV reverse transcriptase (Invitrogen, Thermo Fisher Scientific), random hexamers (Thermo Fisher Scientific) and dNTPs Mix (Thermo Fisher Scientific). RT-PCR is performed using SsoAdvanced Universal SYBR Green Supermix (Bio-Rad laboratories) in 96-well plates (Bio-Rad laboratories). The RT-PCR reaction is performed using a CFX Connect Real-Time PCR Detection System (Bio-Rad laboratories) using primers from QuantiTect Primer Assay (Qiagen) as well as custom made primers.

The experimental data obtained from the saliva samples as explained above will be further analysed with a computational model in order to provide scientifically justified and personalized suggestions for best timing of medicine intake (wherein applications for other certain daily activities, such as light exposure, sleep, sports, or food intake may be envisioned), to avoid circadian rhythm disruption, and thus enhancing cancer treatment. As will be described in detail below, a mathematical model for the circadian clock is created, which may include core-clock and clock-controlled metabolic genes in about 50 elements, based on which models for relevant gene networks, particularly related to drug metabolism in connection to the circadian clock can be generated. By feeding each network with specific gene expression data obtained from saliva samples, accurate predictions for day-/night-time activities can be generated.

The experimental data obtained from the saliva samples as explained above will be further analysed with a computational model in order to provide scientifically justified and personalized suggestions for best timing of medicine intake (wherein applications for other certain daily activities, such as light exposure, sleep, sports, or food intake may be envisioned), to avoid circadian rhythm disruption, and thus enhancing cancer treatment. As will be described in detail below, a mathematical model for the circadian clock is created, which may include core-clock and clock-controlled metabolic genes in about 50 elements, based on which models for relevant gene networks, particularly related to physical performance in connection to the circadian clock can be generated. By feeding each network with specific gene expression data obtained from saliva samples, accurate predictions for day-/night-time activities can be generated.

Based on the experimental data from the saliva samples, more specifically the measured gene expressions and the resulting fitted oscillatory curves, a core-clock model will be generated, which may include a larger number of other genes that were not included in the measurements but that may be relevant for the desired prediction (this model is also referred to as "network computational model" in this disclosure). The core-clock is located in the brain (suprachiasmatic nucleus) and its oscillations entrain the peripheral clocks. The oscillations result from feedback loops, which can be investigated by experimental and theoretical means.

Rather than relying only on a model for the circadian rhythm that simply shows oscillations such as phase-oscillators, the present disclosure uses a molecular model, which models (part of) the molecular interactions underlying the circadian clock. This is because, as already mentioned, molecular models contain biological information that might be useful for predictions. Molecular models with simple feedback loops models are often based on Goodwin's oscillator, e.g. (Ruoff and Rensing 1996), but the level of detail may also be extensive (Forger and Peskin 2003). According to an exemplary embodiment of the present invention, a model at an intermediate state of complexity is generated, complex enough to capture a significant part of the genetic network, but not too complex, as this may affect fitting of the data to the model without significant overfitting. Relogio et al. have published a model at this level of complexity, with 19 dynamical variables, which is used in the following (Relógio et al. 2011).

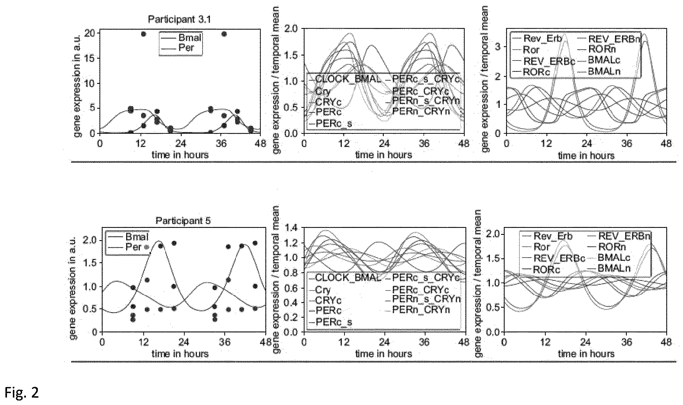

Now referring to Fig. 2, two examples of fits of the saliva data to the core-clock model are shown. (Relógio et al. 2011). Left: Saliva data is plotted as dots, including data for BMAL1 and PER2, where the measurements of both measured days within the same 24 hours are plotted, which is then plotted for two consecutive days. The curves result from the model fit. Middle and right: The model contains 19 dynamical variables, 17 are plotted in these two panels.

Fig. 2 illustrates that the dynamical model may restrict the shape of the fitted curves. In this exemplary embodiment, the curves are more complex than a simple sine-cosine function, but they are also not perfectly fitted to the data, as may happen when a spline is used to fit the data, because the model can only produce shapes that result from the interacting dynamics.

The data base for the fit are the experimental, non-logarithmic gene expression values, $2^{\Delta CT}$. In order to get the experimental values on the same scale as the model output (which is on the order of one), the gene expression of both PER2 and BMAL 1 are normalized in this exemplary embodiment by the mean of BMAL1 expression; that way the relative amplitude of both genes is preserved. In order to allow for a fairer comparison both the simulated and the experimental data may be normalized by their respective mean BMAL1 expression.

The complexity of the model with around 80 parameters makes a meaningful fit that prevents overfitting challenging. At least one of or a combination of one or more (including all) of the following approaches may be used to fit the model to the saliva data. It will be appreciated that other approaches may be used alternatively or in addition to adjust the model.

To constrain the model to parameter regions in which continuous oscillations occur, a bifurcation analysis may be used to delineate the regions with limit cycles (i.e. continuous oscillations), and restrict the parameter optimization to these regions. This prevents fits that show a (slow) relaxation to a steady state, as the model may be expected to have a stable limit cycle. Considering the bifurcation structure, fits will be faster because less parameters need to be checked and because less simulation time is required to ensure relaxation of the oscillatory behavior.

To minimize the number of parameters with large deviations from the original model, standard regression methods may be used that are also added to the cost function, such as ridge regression, which has proven useful so far.

Based on biological and dynamical considerations, certain parameters may be fixed at the original value and exclude them from the fit. This may be for example done for parameters that show only minor impact on the resulting curves, parameters for which no inter-individual variation is expected (i.e. diffusion constants which result from biochemical properties) and parameters which have been repeatedly measured in experiments for humans.

Finally, least-squares minimization (details see below) may be used to minimize the distance between experimental data and fitted curve. The associated cost function used for least-squared error minimization may be extended with additional terms that can restrict the period (should be between 20 and 28 hours for human material), amplitude (no constant amplitude, e.g.) and the position of peaks and troughs.

According to an exemplary fitting procedure, the two days of gene expression data are interpreted as replicates, and the model is fitted to both data points for 9h, 13h, 17h and 21h at the same time, as indicated by two data points for each time point in the above figure. In order to fit the model to the gene expression data, the model may be run for 72 hours with a time resolution of 0.01 hours. The last 48 hours are used for the analysis. As model and experiments have no common time, all possible time-shifts between experiments and model output are considered (0 up to 24 hours). For each shifted variant of the model output, the least-squares cost function C may be calculated between the experimental

$$C_0 = 2\sqrt{1 + \left(x_{exp} - x_{mod}\right)^2} - 2$$

values $x_{exp}$ and the model output $x_{mod}$ as      for both genes (BMAL1 and PER2), and then the time-shift with the minimal summed cost for both genes may be selected. To optimize the fit, a selection of the following additional cost function terms may be added to $C_0$:

- A regression term, that penalizes large parameters: Given the parameter vector $p = (p_i)$ where $i$ is between 1 and the number of parameters, ridge regression adds a term $C_{ridge} = c\sum p_i^2$ to the cost function, with a prefactor $c$ that was set to 1.
- A term that penalizes deviating periods. One may first measure the period $p$ of the model, and then compare it to the standard human circadian period of 24.5 hours: $C_p = c_p(p - 24.5)^2$, with weighting factor $c_p = 1$.
- A term that penalizes the amplitude deviations: For experimental and simulated amplitudes $A_{exp}$ and $A_{sim}$, the cost function is $C_a = c_a(A_{sim} - A_{exp})^2$, with weighting factor $c_a = 0.05$.
- A term that penalizes if the peak or trough position deviates. Peak times of the experimental data $t_{exp}$ and of the model trace with the optimal time-shift applied $t_{sim}$ may be derived and the cost term calculated as $C_{top} = c_t(p - 24.5)^2$, with weighting factor $c_t = 0.1$ for the peaks. The same may be done for the trough, resulting in a cost $C_{down}$, with weighting factor $c_d = 0.05$.

The cost function sums the individual costs weighted by a factor chosen to optimize the influence of each cost. $C_{total} = C_0 + C_{ridge} + C_p + C_a + C_{top} + C_{down}$.

In one embodiment the present invention provides a method which allows assessing circadian rhythm or circadian profile of a subject having cancer and/or assessing a timing of administration of a medicament to said subject having cancer, wherein said method comprises the steps of:

- Providing at least three samples of saliva, more preferably four samples of saliva, from said subject, wherein said samples have been taken at different time points over the day;
- Determining gene expression of the following genes in each of said samples:

  a. of at least two members of the core-clock network in each of said samples, in particular of at least two members of the following genes, of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, and
  b. at least one gene involved in the metabolism of a medicament to be administered to said subject having cancer, including Ces2, and
  c. at least one drug target gene that is a target of the medicament to be administered, and

- Assessing and predicting by means of a computational step based on said expression levels of said genes over the day the circadian rhythm of said subject and/or assessing a timing of administration of said medicament to said subject, comprising assessing the optimal time of administration of said medicament to said subject and/or assessing the non-optimal time of administration of said medicament to said subject.

In another embodiment the present invention provides a method, wherein gene expression is determined using a method selected from quantitative PCR (RT-qPCR), NanoString, sequencing and microarray.

In another embodiment the present invention provides a method, wherein gene expression is determined using quantitative PCR (RT-qPCR).

In another embodiment the present invention provides a method, wherein gene expression is determined using NanoString.

In another embodiment the present invention provides a method, which allows assessing the timing of administration of said medicament to said subject comprises predicting gene expression of at least one of said genes and/or at least one further gene involved in the metabolism of said medicament.

In another embodiment the present invention provides a method, wherein the at least one further gene involved in the metabolism of the medicament to be administered is at least one of Ugt1a1 and Abcb1.

In another embodiment the present invention provides a method, which allows assessing the timing of administration of said medicament to said subject comprises evaluating the predicted gene expression levels and/or evaluating expression phenotypes based on the determined and/or predicted gene expression levels.

In another embodiment the present invention provides a method, which allows assessing the circadian rhythm of said subject comprises in the computational step determining a periodic function for each of said determined genes that approximates said gene expression levels for each of said genes, preferably comprising curve fitting of a non-linear periodic model function to the respective gene expression levels, wherein the curve fitting is preferably carried out by means of harmonic regression.

In another embodiment the present invention provides a method, wherein the computational step comprises processing the determined gene expression levels to derive characteristic data for each of said genes, said processing comprising determining the mean expression level of expression of a gene and normalizing the gene expression levels using the mean expression level.

In another embodiment the present invention provides a method, wherein said characteristic data comprise:

- the amplitude of change of expression of a gene, and/or the amplitude relative to one of the other genes, and/or
- the mean expression level of expression of a gene, and/ or and/or the mean relative to one of the other genes, and/or
- the peak expression level of a gene, and/or the peak relative to one of the other genes., and/or
- the amplitude of change of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the amplitudes of change of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the mean expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/ or
- the relative difference of the mean expression levels of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the peak expression levels of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the time of the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,
- the relative difference of the times of the peak expression level of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,

wherein the amplitude, period and phase expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC are extracted from the determined expression levels and/or the respectively fitted periodic function.

In another embodiment the present invention provides a method, wherein the computational step further comprises

- fitting a network computational model to the derived characteristic data that comprises a representation of the periodic time course of the expression levels for each of said determined genes as well as a representation of the periodic time course of the expression level for at least one, preferably a plurality of further gene(s) included in a gene regulatory network that includes said genes; and/or

- training a machine learning algorithm on the derived characteristic data to form the network computational model, particularly optimize in terms of the representation of the periodic time course of the expression level for the at least one further gene.

In another embodiment the present invention provides a method, which allows assessing the timing of administration of said medicament to said subject comprises in the computational step fitting a prediction computational model on data obtained from said fitted periodic functions and/or said network computational model, wherein the prediction computational model is based on machine learning including at least one classification method and/or at least one clustering method, wherein said method(s) are preferably selected from the group comprising:
K-nearest neighbor algorithm, unsupervised clustering, deep neural networks, random forest algorithm, and support vector machines.

In another embodiment the present invention provides a method, wherein additional physiological data of the subject are provided for fitting the prediction computational model.

In another embodiment the present invention provides a method, wherein the network computational model and/or the prediction computational model form a personalized model for said subject.

In another embodiment the present invention provides a method, wherein samples of at least two consecutive days of said subject are provided and the gene expression levels of said genes in said samples are determined and used, preferably at least four samples per day.

In another embodiment the present invention provides a method, wherein

- the medicament is Filgrastim and the target gene is *Csf3r* and the indication is acute myeloid leukemia; or
- the medicament is Rituximab and the target gene is selected from the group comprising *Fcgr2b, Ms4a1, and Fcgr3;* and the indication is rheumatoid arthritis and Non-Hodgkin's lymphoma; or
- the medicament is bevacizumab and the target gene is *Fcgr2b, Vegfa, Fcgr3;* and the indication is Colorectal cancer and Non-small cell lung cancer; or
- the medicament is trastuzumab and the target gene is Fcgr2b, Erbb2, Egfr, Fcgr3 and the indication is Breast cancer; or
- the medicament is Imatinib and the target gene is Ptgs1, Kit, Slc22a2, Abcg2, Pdgfra, Pdgfrb, Ddr1, Abca3, Abl1, Ret, Abcb1a and the indication is Chronic myeloid leukemia; or
- the medicament is Pemetrexed and the target gene is Tyms, Atic, Gart, Slc29a1 and the indication is Mesothelioma and Non-small cell lung cancer; or
- the medicament is Capecitabine and the target gene is Cda, Tymp, Tyms, Ces1g, Dpyd and the indication is Breast cancer and colorectal cancer; or
- the medicament is Erlotinib (tyrosine kinase inhibitor, anticancer drug) and the target gene is EGFR, Ras/Raf/MAPK, and PIK3/AKT (tumour) and the indication is Tumour inhibition (ZT1 >> ZT13); or
- the medicament is Sunitinib (tyrosine kinase inhibitor, anticancer drug) and the target gene is Cyp3a11 (liver, duodenum, jejunum) abcb1a (liver, duodenum, jejunum, lung) and the indication is renal cell cancer and pancreatic neuroendocrine tumours; or
- the medicament is Lapatinib (dual tyrosine kinase inhibitor interrupting the HER2/neu and EGFR pathways, anticancer drug) and the target gene is EGFR/Ras/Raf/MAPK, Errfi1, Dusp1 (liver), Hbegf, Tgfα, Eref (liver) and the indication is solid tumours such as breast and lung cancer; or
- the medicament is Roscovitine (seliciclib, CDK inhibitor, anticancer drug) and the target gene is Cyp3a11, Cyp3a13(liver) and the indication is non-small cell lung cancer (NSCLC) and leukemia; or
- the medicament is Everolimus (mTOR inhibitor, anticancer drug, immunosuppressant) and the target gene is mTOR/Fbxw7/P70S6K (tumour) and the indication is breast cancer; or
- the medicament is Irinotecan (Top1 inhibitor, anticancer drug) and the target gene is Ces2, Ugt1a1, abcb1a, abcblb (liver and ileum), abcc2 (ileum) and the indication for colorectal cancer, advanced pancreatic cancer and small cell lung cancer; or
- the medicament is Tamoxifen (antiestrogenic, anticancer drug) and the target gene is Cyp2d10, Cyp2d22, Cyp3a11 (liver) and the indication is breast cancer; or
- the medicament Bleomycin (toxicant and anticancer drug) and the target gene is NRF2/glutathione antioxidant defence and the indication is pulmonary fibrosis, palliative treatment in the management malignant neoplasm (trachea, bronchus, lung), squamous cell carcinoma, and lymphomas.

In another embodiment the present invention provides a method, wherein each of the time points at which said samples are obtained are at least 2-4 hours apart, and/or wherein the time points span a time period of at least 12 hours of the day, wherein preferably the time points are 4 hours apart, e.g. at 9h, 13h, 17h and 21h.

In another embodiment the present invention provides a kit for sampling saliva, comprising

- sampling tubes for receiving the samples of saliva, wherein each of the sampling tubes contains RNA protect reagent and is configured to enclose one of the samples of saliva to be taken together with the reagent, wherein preferably each of the sampling tubes is labelled with the time point at which the respective sample is to be taken and/or includes an indication about the amount of saliva for one sample.

In another embodiment the present invention provides a kit, further comprising at least one of:

- a box,
- a cool pack,
- at least one form including instructions and/or information about the kit and the method for the subject.

In another embodiment the present invention provides a kit, wherein the RNA protect reagent is selected from the group comprising EDTA disodium, dihydrate; sodium citrate trisodium salt, dihydrate; ammonium sulfate, powdered; sterile water.

In another embodiment the present invention provides a kit, wherein said sampling tubes are configured to receive a sample of saliva of 1 mL in addition to 1 mL of the RNA protect reagent, wherein the sampling tubes preferably are at least 2 mL tubes, preferably at least 3 mL tubes, more preferably at least 4 mL tubes, still preferably at least 5 mL tubes.

In another embodiment the present invention provides a kit, comprising at least six sampling tubes, preferably at least eight sampling tubes.

In another embodiment the present invention provides a kit for collecting samples of saliva for providing the collected samples of saliva.

In another embodiment the present invention provides a method of RNA extraction for gene expression analysis from a sampling tube for receiving the sample of saliva comprising the steps of:

- Separating the sample of saliva by means of centrifugal force and generating a cell pellet;
- Separating the pellet from supernatant and homogenizing the pellet in an acidguanidinium-phenol based reagent, preferably TRIzol;
- Adding an organic compound, preferably chloroform, and mixing said homogenate with a shaking device, preferably a vortexer, and obtaining a mixture;
- Separating said mixture by means of centrifugal force resulting in a solution having more than one phase with an upper aqueous phase comprising the RNA to be extracted; and
- Removing said RNA to be extracted in said aqueous phase from said solution having more than one phase.

In another embodiment the present invention provides a method, comprising additionally the steps of:

- Performing optionally a processing step for preparation of the extracted RNA samples for determining gene expression;
- Performing gene expression analysis.

In another embodiment the present invention provides a method, wherein the extracted RNA is reverse described into cDNA and subsequently amplified.

Examples

In view of the aforementioned explanations, an exemplary embodiment for a general workflow to establish a prediction for the best time for a "behavior B" is outlined below. After that, more specific aspects of the workflow are explained for the "behavior B" being the peak time for sport performance.

1. A priori gene selection:

A set of relevant genes is selected: Core-clock genes, saliva specific oscillating genes (which may show stronger oscillations than the core-clock genes in saliva), and a set of genes that should relate to the behavior B (for sports metabolic genes; for cancer treatment-related genes or drug target genes, etc.). To identify the latter, existing databases are scanned for (1) connections to the core-clock in the genetic network, (2) oscillatory behavior (at least for some tissue, potentially from mice or human), (3) expression level in saliva of healthy human samples or saliva from non-healthy people (particularly having the type of cancer to be treated).

2. Establishment of a data set:
Subjects are asked to perform behavior B several times per day, and their performance is recorded. From the same subjects, saliva is sampled for two days 4 times a day.

3. Experimental analysis:
Gene expression is measured from the saliva samples.

4. Computational analysis (see also description above):

i. The gene expression data is screen for oscillatory behavior, non-oscillating genes are excluded from the analysis, see .e.g. Stroebel, A.M., Bergner, M., Reulbach, U., Biermann, T., Groemer, T.W., Klein, I. and Kornhuber, J., 2010. Statistical methods for detecting and comparing periodic data and their application to the nycthemeral rhythm of bodily harm: A population based study. Journal of Circadian Rhythms, 8, p.Art. 10.
ii. The expression data of oscillating genes is used to predict the optimal time for behavior B, as recorded for the subjects. The resulting machine learning algorithm can then be used to predict the optimal time for further subjects for which only saliva data is available.
iii. A core-clock model is supplemented with the genetic network that includes the oscillatory genes identified in step 4i. The model is fitted to the gene expression data.
iv. The model is used to generate data for the prediction of the optimal timing of behavior B. This data has three advantages: 1. The temporal resolution of the data is arbitrarily detailed. 2. In consequence, peak time, phase, amplitude and period can be extracted with more precision. 3. The data includes all genes implemented in the model, which are far more than the genes measured in step 2. With this data as input, step 4ii is repeated, and the performance is compared to the prediction based directly on the data, while accounting for the enhanced potential for overfitting due to a larger amount of processing (more parameters and potentially more data points for the prediction). Although also a simple fit of the data as in step i gives us curves with high temporal resolution, this is not the same as the model fit: A prediction based on the simple fit cannot outperform the prediction from step 4ii, because no information was added. By contrast, the model fit contains information on the biological interaction between the genes (the gene dynamics), which, when added to the prediction, can improve the prediction.
v. Even more important, the model can illuminate the mechanism behind the prediction of step 4ii and iv. This is an important step to enhance trustworthiness of the prediction - the more we understand, the more we can evaluate whether the prediction makes sense (see sports example below: The correlation between the peak times of PER2 and sport performance is likely to explain why PER2 can be used to predict sports performance. Although we have a small sample size of only 10 participants, this gives us confidence that the prediction is not happening by chance, but is really catching some salient feature in the data).
vi. With the idea about the working of the prediction mechanism from step 4v, genes with an even higher expected predictive power can be identified. Those genes can be added to the set from step 1, if the whole workflow is repeated to optimize the prediction even further.

5. A posteriori gene selection:
Based on the computational analysis, the a priori set of genes is stripped to the genes essential for prediction, in order to minimize the cost of the analysis.

6. Commercial application:
People provide saliva samples, and the machine learning algorithm resulting from step 4 is used to predict optimal timing of behavior B based on the restricted set of genes from step 5.

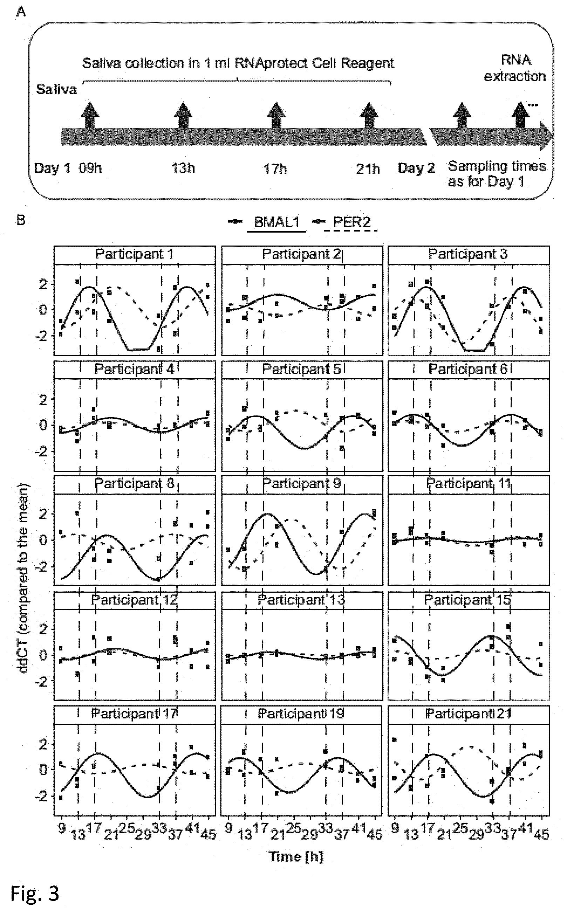

According to the present invention, an individual-based (machine learning) prediction of optimal timing for administering drugs in a cancer treatment is provided. An exemplary embodiment is explained hereinafter, wherein it is referred to the following publications where appropriate.
Time-course measurements of unstimulated saliva show fluctuations in gene expression across 45 hours are exemplarily shown in Fig. 3. (A) Sampling schemes for saliva collection at 8 time points in two consecutive days (Day 1- 9h, 13h, 17h, 21h; Day 2 - as day 1). (B) Time-course RT-qPCR measurements of human saliva normalized to the mean of all time points (∆∆CT) of *ARNTL (BMAL1)* (black) and *PER2* (grey dashed) of 15 participants (7 female and 8 male) with a fitted linear sine-cosine function. Furthermore, table C shows the harmonic regression analysis and table D provides additional information on the participants and tests performed.

(Li et al. 2013): "The application of a maximum-a-posteriori Bayesian inference method identified a linear model based on Rev-erbα and Bmal1 circadian expressions that accurately predicted for optimal irinotecan timing. The assessment of the Rev-erbα and Bmal1 regulatory transcription loop in the molecular clock could critically improve the tolerability of chemotherapy through a mathematical model-based determination of host-specific optimal timing." "The optimal circadian timing of an anticancer drug was predicted despite its variation by up to 8-hour along the 24 hours among six mouse categories. This prediction relied on a mathematical model using liver circadian expression of clock genes Rev-erbα and Bmal1 as input data and treatment tolerability as output parameter." This predicts toxicity for mice and as the database is extremely small, the authors doubt the generalizability of the prediction. The model is no molecular model, as in our case, but just a linear model for the prediction, not meant to model biological information.

(Dulong et al. 2015): "Irinotecan cytotoxicity was directly linked to clock gene BMAL1 expression: The least apoptosis resulted from drug exposure near BMAL1 mRNA nadir (P < 0.001), whereas clock silencing through siBMAL1 exposure ablated all the chronopharmacology mechanisms. Mathematical modeling highlighted circadian bioactivation and detoxification as the most critical determinants of irinotecan chronopharmacology." In this study, the circadian variation does not result from an explicit model of the core-clock, as in our case, but the circadian variation is modeled as a time-dependent cosine function fitted to the experimental data.

The behavior of the circadian network differs between individuals. Hence, it is crucial to evaluate the expression phenotypes in order to predict daily variations in cytotoxicity in a personalized manner. While related work gives a general prediction of optimal irinotecan timing (Li et al. 2013, Dulong et al. 2015), the approach of the present invention is personalized because prediction of optimal treatment time is based on the individual gene expression as measured in the saliva samples, and the model is fitted accordingly to these data, which allows for even better personalized predictions of optimal treatment time. The general predictions rely implicitly on the assumption that the regulation of the drug target gene by the core clock is the same in patients. However, by sampling directly the target gene plus circadian genes, the approach of the present invention predicts optimal treatment time better.

With reference to the drawings, a unifying model to optimize cancer treatment timing is described below.

Biological constituent of the human body form an intricate network of interactions. Because the level of interactions is extremely high, it is difficult to consider one pathway without considering its neighbors. It has been established a unifying model of the interaction between cancer and the core-clock. This improves the prediction of treatment timing in comparison to the currently existing sub-studies by explicitly considering their interactions, and allowing for a quantitative comparison of their effect size. This permits to optimize the treatment timing with respect to all relevant influences at the same time, compromising for example between the optimal cell cycle for treatment and the minimal toxicity for non-cancer cells. The model will include the following (with all cited references being from the group around the inventors of the present invention).

The core-clock network (Relógio et al. 2011). The detailed model of the core-clock allows for example to include the mutations in core-clock genes seen in around 4% of cancer patients (Yalçin et al. 2020).

Interaction between cancer and clock. A strong bidirectional interaction has been shown between cancer-related genes and circadian genes. Several potential clock-controlled genes have been identified which show a strong association with cancer and cancer progression, in particular related to metabolism (Yalçin et al. 2020, @abreu_reciprocal_2018, @fuhr_circadian_2018). In vitro, in vivo and in mathematical models we found that the core-clock impacts tumor growth (Basti et al. 2020). Furthermore, the experimental, bioinformatics and modelling data showed the inverse, that cancer-related genes impacting the core clock (Relógio et al. 2014). For example, using an inducible RAS expression system, we show that overexpression of RAS disrupts the circadian clock and leads to an increase of the circadian period while RAS inhibition causes a shortening of period length, as predicted by our mathematical simulations (Relógio et al. 2014).

The cell cycle network (El-Athman et al. 2017), which allows to take advantage of the minimization of treatment toxicity for non-cancer cells by timing treatment to their cell cycle. The relation was shown in experiments on cell cultures and in modelling simulations (El-Athman et al. 2017): The observed changes can be attributed to in silico phase shifts in the expression of core-clock elements. A genome-wide analysis revealed a set of differentially expressed genes that form an intricate network with the circadian system with enriched pathways involved in opposing cell cycle phenotypes. In addition, a machine learning approach complemented by cell cycle analysis classified the observed cell cycle fate decisions as dependent on Ink4a/Arf and the oncogene RAS and highlighted a putative fine-tuning role of Bmal1 as an elicitor of such processes, ultimately resulting in increased cell proliferation in the Ink4a/Arf knock-out scenario.

The irinotecan network (see below and (Ballesta et al. 2011)), which allows to access the impact of treatment on cancer and non-cancer cells (maximizing toxicity for cancer cells while minimizing toxicity for non-cancer cells by taking advantage of their differential circadian rhythms).

Tumor motility network. It was found that the core-clock impacts > metastasis (Basti et al. 2020) The reasoning here is that treatment timing > that suppresses metastasis might be more relevant for the > long-term survival of the patient than optimizing treatment to > kill existing cancer cells.

Host-microenvironment: Cancer cells live in an environment that contains other tissues such as fibroblasts. For fibroblasts it was shown that their communication with (benign) tumor cells affect the molecular clockwork and seem to aggravate malignant cell phenotypes (Fuhr et al. 2019). This communication will be modeled as a bath solution in which the cancer cell lives, and which also interacts with the core-clock.

Given the range of molecular time-dependent processes that it regulates, including metabolism, DNA repair and the cell cycle, the circadian clock has the potential to act as a tumour suppressor (El-Athman et al. 2017). The present invention helps to reach the full potential of the circadian clock as tumour suppressor by strengthening the circadian clock rhythm (i.e. increasing the amplitude), not only by optimizing the treatment but also by optimizing other daily activities that influence the circadian clock (eating, sleep rhythm, day light exposure, etc.).

Modelling the irinotecan network is one aspect of the prediction for optimal cancer treatment in accordance with the present invention. The 24-hour light/dark transitions of the Earth led organisms to evolve an endogenous timing-mechanism: the circadian clock, which accounts for rhythmic oscillations in physiological, behavioural, and cellular functions. Circadian rhythms influence drug pharmacology and ultimately tolerability and efficacy of drugs. This impacts the treatment of several diseases, including cancer. Here we focus on the irinotecan (CPT11) treatment of colorectal cancer (CRC). Both, the target gene of CPT11 (Top1) and the genes involved in its pharmacokinetics and pharmacodynamics (PK-PD), show a circadian oscillation in their expression. Hence, CPT11 efficacy and toxicity display circadian rhythms in cancer patients. To identify the optimal time for drug delivery, it has been established a new ODE mathematical model of the circadian clock which includes genes involved in the processing of CPT11, influencing its cytotoxicity. The personalization of the model is achieved by fitting the model parameters to experimental data of gene expression from different CRC cell lines.

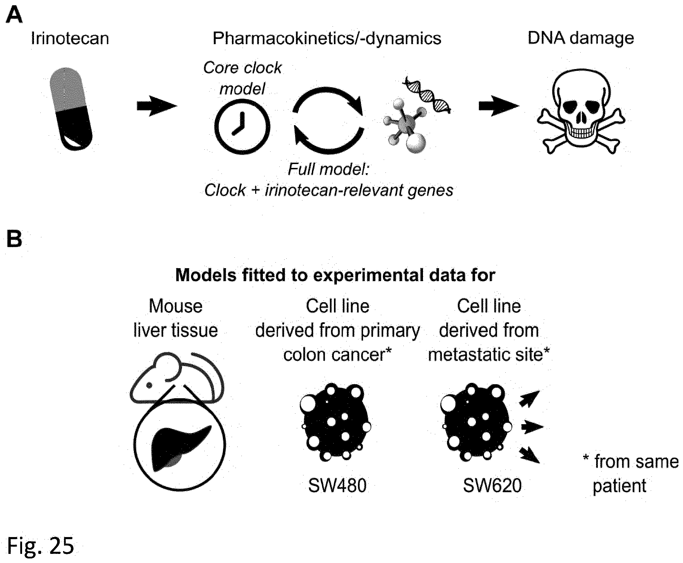

In this exemplary embodiment for the drug irinotecan for which the target gene is TOP1, the core clock network by Relogio et al. (Relógio et al. 2011) is extended with equations that are derived from the Irinotecan network published by reference (Ballesta et al. 2011), see Fig. 25. It will be appreciated that this may be applied to other drugs and their respective target gene accordingly. Basically, irinotecan (CPT11) is a TOP1 inhibitor. TOP1 binds to DNA and relaxes supercoiled strands for replication and transcription. TOP1 then dissociates from DNA allowing the reconnection of the strands. The active metabolite of CPT11 (SN38) prevent TOP1 relegation by creating DNA/TOP1/SN38 complexes, inducing lethal DNA strand breaks.

Selection of genes based on (Dulong et al. 2015).

- Drug target gene: Top 1.
- Measured genes: Bmal1, Per2, Top1, Ces2.
- Predicted genes: Ugt1a1, Abcb1.

The model simulates gene expression of the core-clock genes and clock-regulated genes via two interconnected feedback loops (Per/Cry loop and Rev-Erb/Ror/Bmal loop). The model parameters were fitted to the measured data of gene expression (see below). The estimation of irinotecan (CPT11)-induced cytotoxicity does not depend on the action of a single protein, but rather on the combinatory performance of the proteins encoded by the aforementioned genes.

CPT11 in the extracellular medium diffuses through the cell membrane and reaches the intracellular compartment, where it is bioactivated into SN38 via CES2. SN38 is detoxified into SN38G through UGT1A1. CPT11, SN38 and SN38G are transported out of the cell by ABCB1. *Top1* relaxes supercoiled DNA by creating TOP1/DNA complexes, which in combination to SN38 may form irreversible complexes ($I_{compl}$) leading to cell apoptosis.

The temporal evolution of apoptosis is calculates based on: (1) DNA repair mechanisms and apoptosis pathways, which follows a circadian rhythm and (2) intracellular concentration of irreversible SN38/DNA/TOP1 complexes, which in turns depends on the protein activity of UGT1A1, CES2, TOP1, and ABCB1.

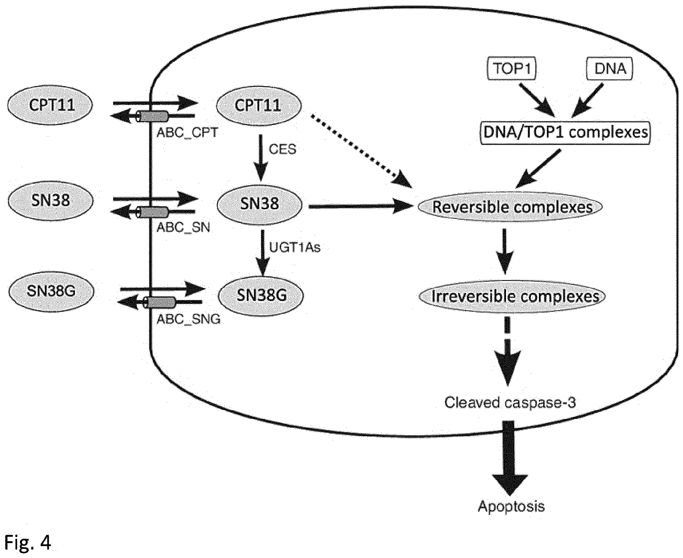

Fig. 4 (from Dulong et al. 2015) illustrates a scheme depicting molecular PK-PD of irinotecan (CPT11). CPT11 in the extracellular medium diffuses passively through the cell membrane and reaches the intracellular compartment. It is then bioactivated into SN38 through carboxylesterases (CES) enzymatic activities. SN38 is detoxified into SN38G through UGT1As. CPT11, SN38 and SN38G are effluxed outside of the cells by ABC (ATP-binding cassette) transporters (ABC_CPT, ABC_SN, and ABC_SNG, respectively). Topoisomerase 1 (TOP1) is an enzyme that relaxes supercoiled DNA by creating transient DNA/TOP1 complexes. SN38 and CPT11, to a lesser extent, stabilize them into reversible complexes, which may become irreversible after collision with replication or transcription mechanisms. This may trigger the apoptotic machinery though the cleavage of caspase-3, ultimately leading to cell apoptosis. Proteins involved in irinotecan efflux (ABC_CPT, ABC_SN, ABC_SNG), bioactivation (CES), and deactivation (UGT1As), together with irinotecan target (TOP1) present experimentally demonstrated circadian rhythms.

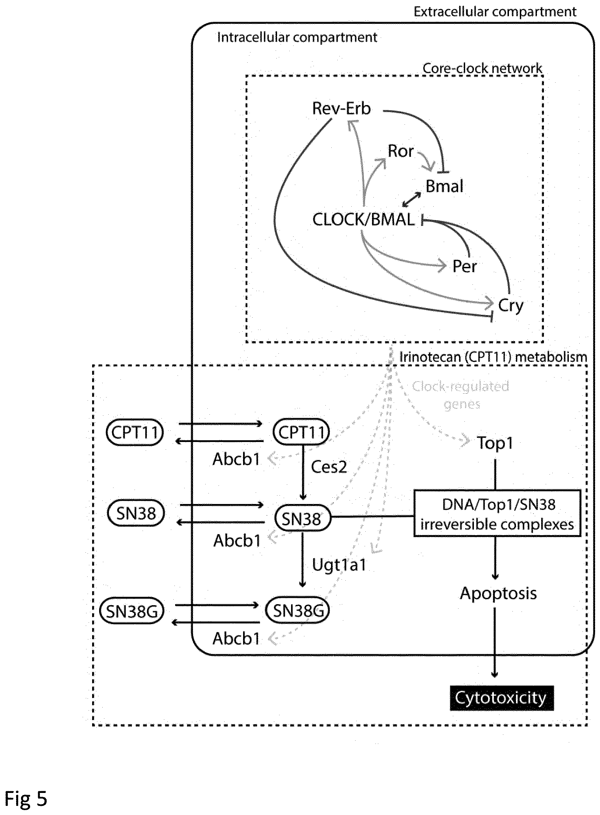

Fig. 5 schematically illustrates the model extension: The core-clock network is complemented with additional genes associated with the irinotecan metabolism.

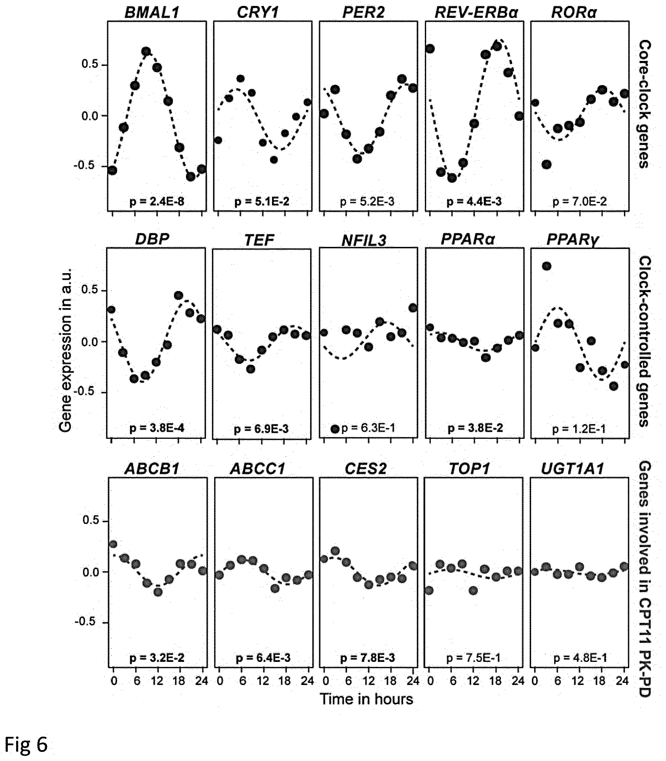

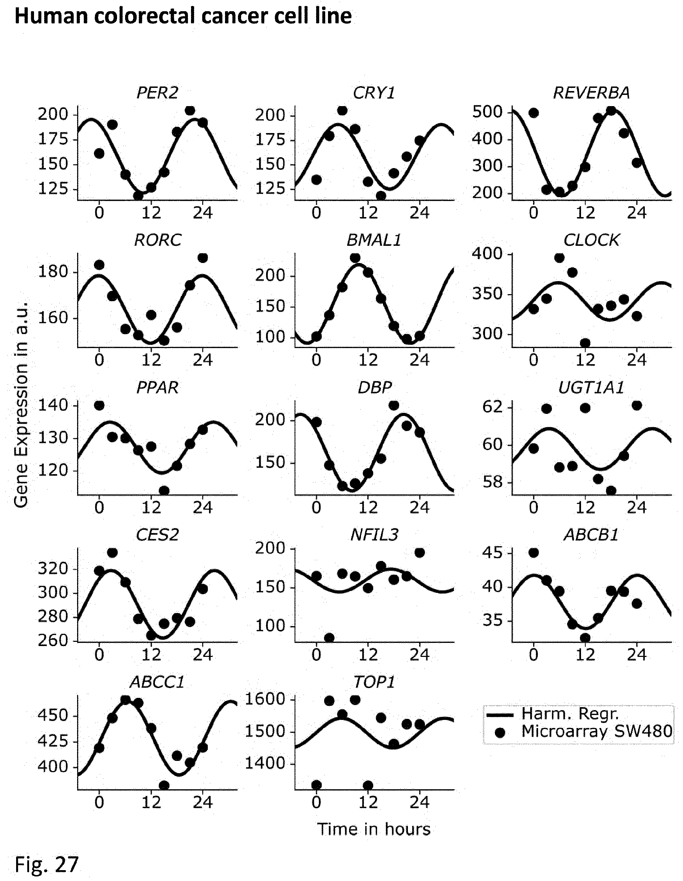

Harmonic regression is then applied to the experimental data to establish oscillatory behavior, i.e. to fit periodic (oscillatory) curves to the experimental data. The genes of the molecular clock (core-clock genes) regulates the transcription of clock-controlled genes. In turn, clock-controlled genes govern the expression of genes involved in irinotecan PK-PD. It is observed 24h-oscillations in the expression of these genes in colorectal adenocarcinoma cell lines (SW480 (Fuhr et al. 2018)). 24h-period harmonic regression (dashed line) for experimental data (dots) from SW480 cell lines is shown in Fig. 6, compare also Fig. 27.

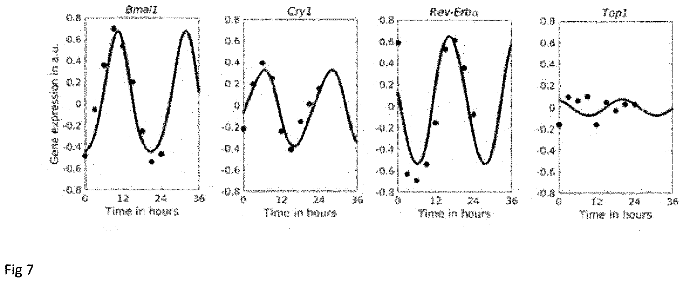

For obtaining a personalized model fit (see Fig. 7), the elements of the model are fitted to RNA experimental measurements retrieved from colorectal adenocarcinoma cell lines (SW480 (Fuhr et al. 2018)). The model fitting was achieved via a coordinate search method in which the badness of fit (BOF) was minimized by cycling through the parameters one at a time.

$$BOF = \sqrt{\sum_i^{\#genes} \sum_j^{n_i} \frac{(s_{i,j} - e_{i,j})^2}{n_i} \cdot w_1 + \sum_i^{\#genes} f_{p,i} \cdot w_2 + \sum_i^{\#genes} f_{t,i} \cdot w_3 + \sum_i^{\#genes} (A_{s,i} - A_{e,i})^2 \cdot w_4}$$

$$f_{p,i} = \begin{cases} 0 & |t_{e_{\max,i}} - t_{s_{\max,i}}| < \Delta t_e \\ (t_{e_{\max,i}} - t_{s_{\max,i}})^2, & \text{otherwise} \end{cases} \qquad f_{t,i} = \begin{cases} 0 & |t_{e_{\min,i}} - t_{s_{\min,i}}| < \Delta t_e \\ (t_{e_{\min,i}} - t_{s_{\min,i}})^2, & \text{otherwise} \end{cases}$$

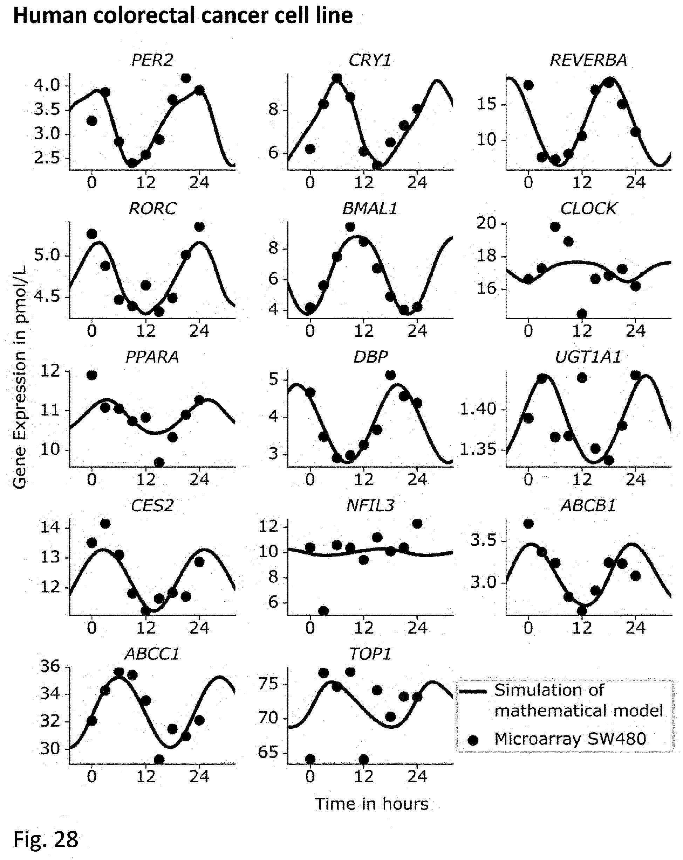

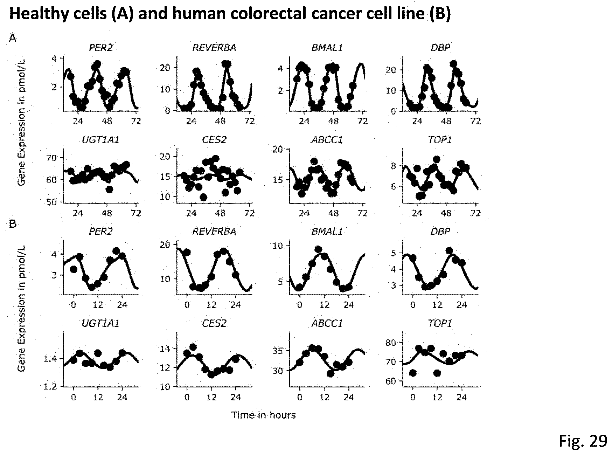

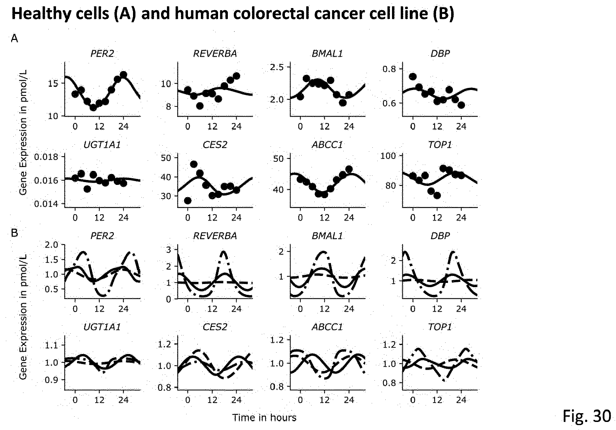

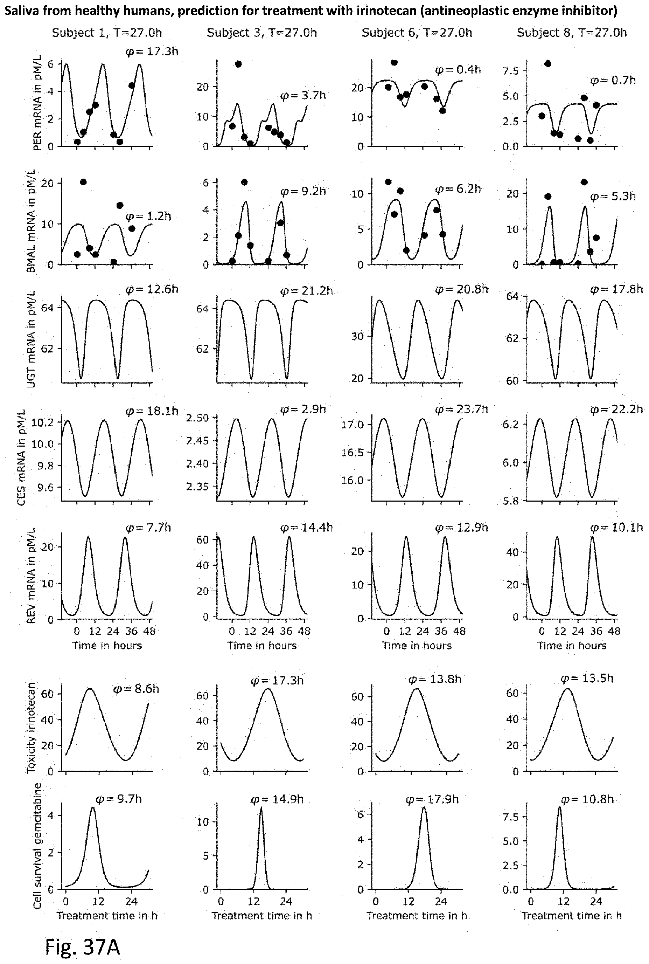

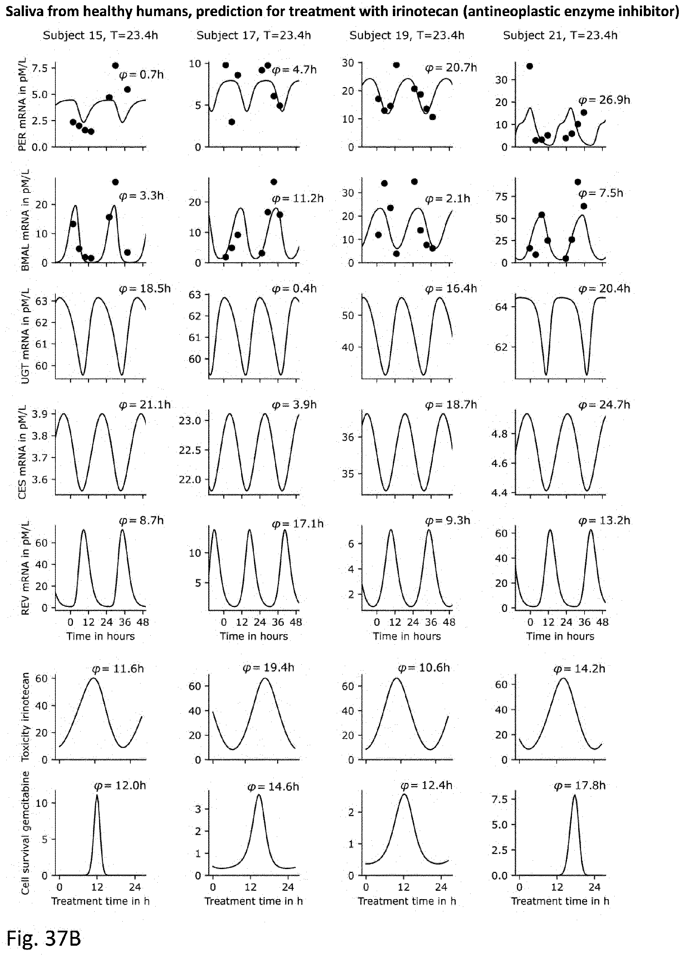

In addition, the model was fitted to the data of the SW480 based on an evolutionary algorithm (CMA-ES), see Fig. 28. Fig. 29 and 30 show the fitted model for selected genes for the SW480 and SW620 cell lines as well as for liver tissue. All the work with cell lines, apart from the mouse liver data, is done with human cells derived from cancer patients. Meaning that even though these are just "cells" they have been taken from cancer patients (but are not primary cells). At the example of two healthy subjects, figure 36 shows how our work in human cancer cell lines can be translated to human saliva samples. Due to differences in individual gene expression measured by saliva sampling, the predicted cell death peaks nearly three hours earlier in subject 6 compared to subject 3. Using the transcriptional translational network of the mouse liver tissue as a starting point, the network is fitted to the BMAL1 and PER2 expression of the saliva data by freeing parameters occurring in the dynamics of the genes BMAL1 and PER2, and the proteins BMAL1 and PER2 (mRNA and cytosolic protein). Ridge regression with a regression factor of 0.01 was used to keep parameters close to the liver value. Fig. 37 shows fits of the transcription-translation network to PER2 and BMAL1 saliva data from healthy subjects (row 1 and 2), the resulting prediction for the genes most relevant for toxicity prediction (UGT1A1 in row 3 and CES2 in row 4), and the resulting toxicity prediction (row 5). The liver data, with a shift of 12 hours to account for the night activity of mice, is for the following reasons a good starting point for human subjects:

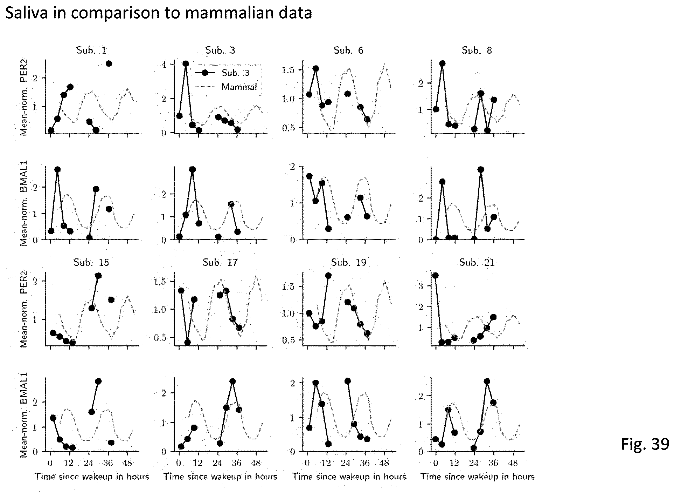

1. The oscillations of the liver are similar to the oscillations of nine other mouse tissues, see Fig. 38, this motivates that the liver data can be used as representative for mammalian circadian oscillations in any healthy tissue.
2. The saliva samples from healthy humans align with the liver data, see Fig. 39.

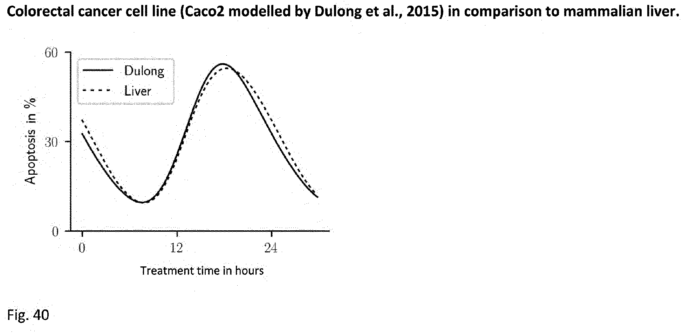

For predicting toxicity based on saliva, we calculated protein phases for irinotecan-relevant genes based on the phase of the related mRNA, plus 3 hours to account for translation. The resulting phases for the mouse liver data are very similar to those used in Dulong's original model: For UGT1A1, ABCB1, ABCG2, CES2, and apoptosis (here assumed to align with REV-ERBA dynamics), Dulong et al. 2015 used phases of [2.79, 9.97, 2.1, 13.7, 21] (with a period of 28 hours), while the liver data results in phases of [2.59, 8.01, 3.97, 1.35, 21.08] with a 23.5 h period, and indeed the toxicity prediction nearly aligns (phase difference of 0.4 hours, as shown in Fig. 40).

In one embodiment for an application of the methods and the model of the present invention, light therapy is implemented as a 5-fold increase in PER2 maximal transcription rate and a 5-fold decrease in PER2 degradation rate. Light therapy 1h after wakeup leads to no changes in the phase, or, for longer duration, to a small phase advance of 6 minutes, see Fig. 41. Light 8h after wakeup leads to half an hour of delay for one-hour treatment, and a bit more than an hour for two-hour treatment. Strongest responses occur for light therapy starting 14h after wakeup, inducing delays of up to 5h, see Fig. 41.

The present methods may be used for guidance of light therapy.

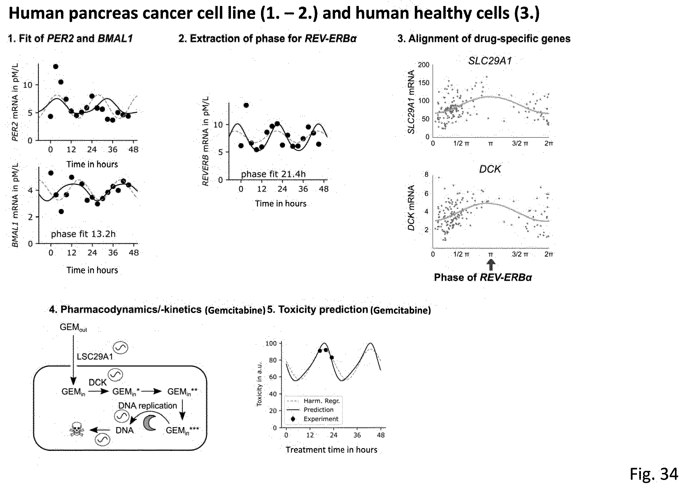

The model was also fitted to a pancreas cancer cell line (ASPC1), see Fig. 33. For this fit, the evolutionary algorithm (CMA-ES) was only allowed to change parameters related to PER2, BMAL1 and REV-ERBα, i.e. ['dy 1', 'dy3', 'dy5', 'dz2', 'dz6', 'dz8', 'V1max', 'V3max', 'V5max', 'kt1', 'ki1', 'kt3', 'ki3', 'kt5', 'ki5', 'kp1', 'kp3', 'kp5'], the set of parameters of the mRNA and cytosolic protein equations excluding Hill coefficients, fold changes, and complex formation/dissociation rates, see Fig. 33A. Fixing the parameters related to ERB-ERBα in addition, and only fitting the data of PER2 and BMAL1 results in a model output which still predicts the phase for REV-ERBα correctly, see Fig. 33B. This exemplifies that a fit of only a subset of genes can indeed predict the dynamics of the remaining genes. We used the gene expression data of the pancreas cancer cell line to model the toxicity profile in response to gemcitabine shown in Fig. 16. The fit of the gene expression data is used together with human data on *SLC29A1* and *DCK,* to predict time-dependent toxicity of gemcitabine treatment, see Fig. 34 for a model overview. Fitting the network to the gene expression of the core clock genes *PER2* and *BMAL1* (Fig. 34, step 1), we predict relevant genes, in this case *REV-ERBα* (Fig. 34, step 2). The phase of *REV-ERBα* is used to align the expression of two genes relevant for gemcitabine pharmacokinetics, *SLC29A1* and *DCK* (Fig. 34, step 3). The model for pharmacokinetics (Fig. 34, step 4) includes several elements with circadian oscillations, as marked by the oscillation wave. Two major steps activate gemcitabine: *SLC29A1* imports of the drug into the cell (import is also done by *SLC28A1* and *SLC28A3,* but these do not show oscillations in humans). *DCK* is the rate limiting step for the activation of the drug; it catalyzes the first of in total three phosphorylation steps. During DNA replication, which occurs with a circadian rhythm that peaks at night, the gemcitabine triphosphate can be incorporated into the DNA, which leads to cell death, which also occurs in a rhythmic fashion following the protein dynamics of *REV-ERBα.* The dynamics of the model are given by the following equations. Gemcitabine concentration inside cell due to import by SLC19A1 (SLC):

$$\frac{dG}{dt} = r \ \text{SLC} - d \ G - r \ G \ \text{DCK}$$

First activation of gemcitabine by DCK:

$$\frac{dG^*}{dt} = r \ G \ \text{DCK} - d \ G^* - r \ G^*$$

Second activation by additional phosphorylation:

$$\frac{dG^{**}}{dt} = r \ G^* - d \ G^{**} - r \ G^{**}$$

Final activation by third phosphorylation:

$$\frac{dG^{***}}{dt} = r \ G^{**} - d \ G^{***}$$

Number of surviving cells, effective exponential growth with rate g and cell death proportional to the number of cells that replicate DNA (N times D) times the rate of apoptosis which oscillates like REVERB (REV), times the amount of activate drug available (G***):

$$\frac{dN}{dt} = g \ N - 0.1 \ G^{***} \ N \ \text{REV} \ D$$

with $D = 1 + 0.37\cos(2\pi/T(t + TT - 19))$ the circadian variation in DNA replication, *REV* the REVERB dynamics from the network model which models circadian variation in apoptosis, $SLC = 1 + 0.25\cos(\frac{2\pi}{T(t+TT)} - 2\pi \ 24.4/T + 3.11 - \pi)$ and *DCK* = 1 + 0.25cos(2π/T(t + TTI) - 2π 24.4/T + 2.97 - π), with parameters as extracted from circaDB, with period *T* and treatment time *TT*. 24.4 hours is the phase of REVERB, 21.4 hours, plus 3 hours delay to account for the translation of the protein.

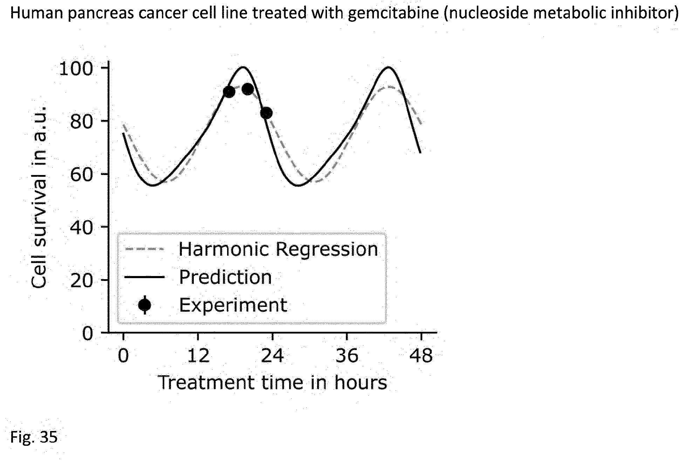

Using the fitted phase of *REV-ERBα* to align *SLC29A1* and *DCK* and the circadian variation in apoptosis, and assuming that DNA replication peaks at 19h CT, i.e. about 5 hours before synchronization at CT 0 (which corresponds to the night translated to humans), the experimental data of cell survival of ASPC1 cells following a 72-hours treatment with gemcitabine can be fitted by the model, as shown in Fig. 35.

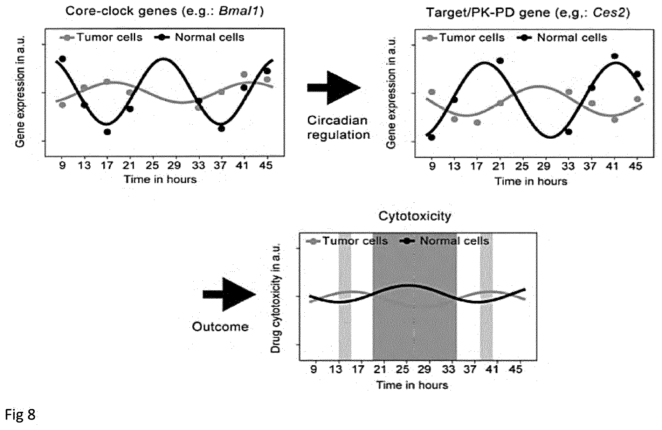

A personalized chronotherapy can be generated for any treatment, e.g. irinotecan treatment. The rhythmic expression of core-clock genes differs between tumour and normal cells. This impacts the circadian expression of the drug target gene and genes involved in the drug detoxification in tumour vs normal cells. Consequently, it is possible to predict a time window for treatment delivery that maximizes treatment efficacy and, at the same time, minimizes side effects. Fig. 8 shows a schematic representation for the rationale of cancer chronotherapy, where virtual data, not real data, have been used to create the scheme to show how the model works.

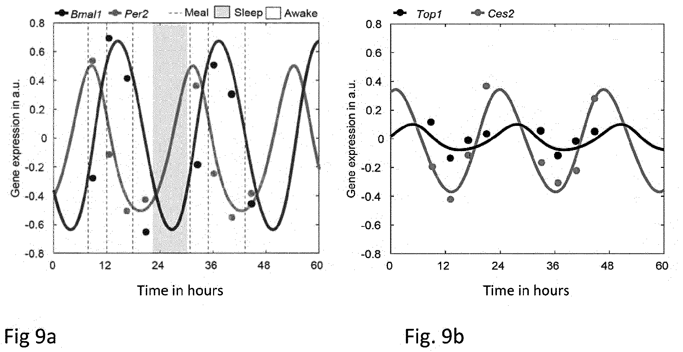

The data of the exemplary embodiment of Figs. 9a and 9b shows the gene expression measurements of two core-clock genes (*Bmal 1* and *Per2*) and two clock-regulated genes (*Top1, Ces*) crucial for irinotecan metabolism. The dots indicate the measured expression values for *Bmal1, Per2, Top1* and *Ces2*. The solid line represents the *in silico* gene expression generated with our mathematical model, which was fitted to the experimental data of the previously mentioned genes. The model additionally predicts the expression levels for *Ugt1a1* and *Abcb1*, important for irinotecan pharmacokinetics and pharmacodynamics. It is shown a personalized model fit of core-clock genes (Fig. 9a) and the genes involved in the irinotecan metabolism (9b) based on the experimental data (dots).

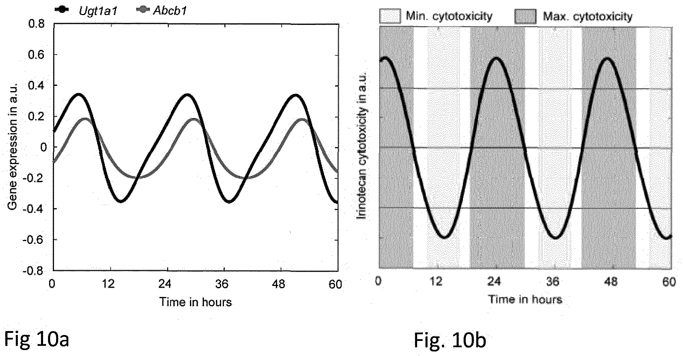

With reference to Fig. 10 it is shown how the model allows to predict in addition the temporal dynamics of two additional genes involved in the irinotecan metabolism (Fig. 10a). Overall, the model fit allows for a prediction of the optimal treatment time with irinotecan (Fig. 10b). Here, the predicted time window for irinotecan delivery to minimize cytotoxicity in healthy tissue is 10:30 - 15:00 hours.

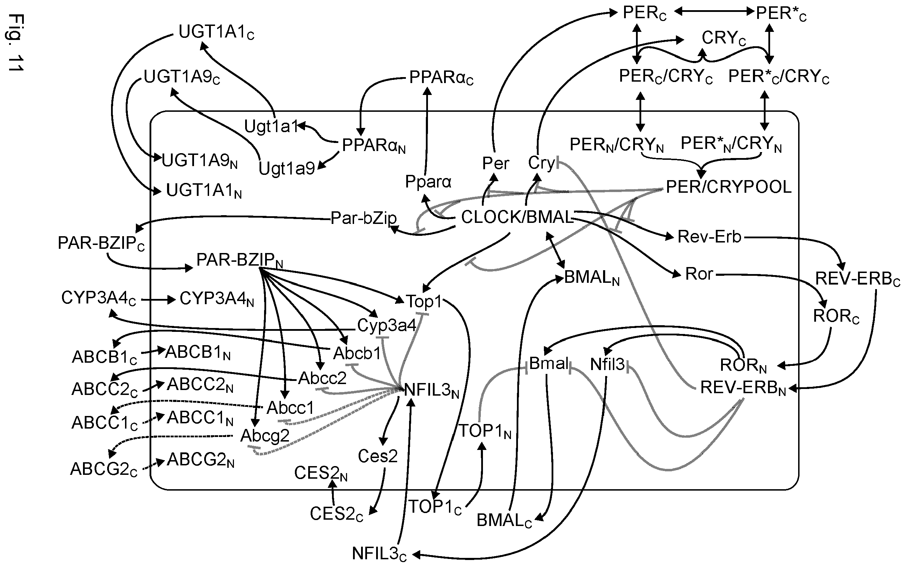

Fig. 11 illustrates a schematic representation of the core-clock network extended with Irinotecan-treatment relevant genetic network.

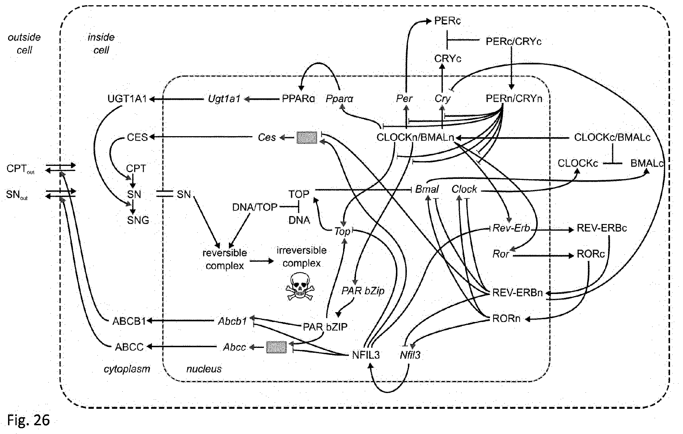

According to an exemplary embodiment of the method, the equations below are used to model the irinotecan network (illustrated in Fig. 11, Fig. 26). As the skilled person will recognize, each drug used for a treatment would have a slight different network and thus a slightly different set of equations. Each treatment has a different model. In addition and because the circadian profile of core-clock genes and the drug target for each patient are measured, this is used as input data for the network and thus a very personalized model is produced for each patient (note that this applies for the differential equations model (ODE), which is different than the machine learning approach).

The equations below are neither in the Relogio et al, Plos Comp Bio 2011, nor in the Ballesta model ("A combined experimental and mathematical approach for molecular-based optimization of irinotecan circadian delivery" Annabelle Ballesta, Sandrine Dulong, Chadi Abbara, Boris Cohen, Alper Okyar, Jean Clairambault, Francis Levi). The interactions are directly taken from the complicated interaction network in the application. For the full circadian model for irinotecan, all equations from the Relogio et al, plus the equations below, plus equations (1) to (11) from Ballesta et al. (2011) and the extension of this model in Dulong et al. (2015) are needed.

The medication pathway is regulated by the core-clock regulated genes that are modelled, and eventually results in a non-reversible triple complex which leads to DNA repair or apoptosis. The amount of this complex is the measure of toxicity (as also used in the Ballesta model).

Citation from Ballesta et al: "CPT11 activity is assessed by the amount of irreversible DNA/TOP1/SN38 complexes, chosen as the output variable because of its experimentallyproven correlation with CPT11 cytotoxicity."

For the full core-clock model reference is made to (Relogio et al. 2011). The model is extended by equations (1) to (11) from (Ballesta et al. 2011), and their equation (12) for the dynamics of protein amount is replaced by the actual core-clock regulated dynamics of the mRNA and proteins of the associated genes.

The model as stated in the following is a refined version of the model stated below. The model was refined with more biological realism, such as an additional feedback from the irinotecan-relevant genes to the core clock.

The variables and parameters of the core-clock model are used for elements belonging to the core-clock. Additional dynamic variables of the clock-irinotecan model are stated in Supplementary Table 1. For genes only the first letter is uppercase, proteins are set in uppercase, concentrations are denoted with square brackets [.]. For simplicity, the model does not explicitly differentiate between cytosolic and nuclear proteins for irinotecan PK/PD-related genes.

*List of dynamical state variables representing mRNAs and proteins. For the irinotecan-related genes, the model uses the same variable names for mRNA and proteins, the latter in uppercase.*

| Gene name | Variable name |
| --- | --- |
| *Bmal1* | $y_5$ |
| *Rev-Erb* | $y_3$ |
| *Ces2* | *Ces* |
| *Ugt1a1* | *Ugt* |
| *Abcb1* | *Abcb* |
| *Abcc* | *Abcc* |
| *Pparα* | *Ppar* |
| *Top1* | *Top* |
| *PAR bZip* | *Par* |
| *Nfil3* | *Nfil* |

| | |
|---|---|
| CES1 | CES |
| UGT1A1 | UGT |
| ABCB1 | ABCB |
| ABCC | ABCC |
| PPARα | PPAR |
| TOP1 | TOP |
| PAR bZIP | PAP |
| NFIL3 | NFIL |

The transcription of *Bmal1* and *Rev-Erb* is replaced by the following equations, which implement the feedback from *Top1* and *Nfil3*.

*Bmal1*

$$\frac{\mathrm{d}y_5}{\mathrm{d}t} = V_{5\max} \frac{1 + i\left(\frac{x_6}{k_{t_5}}\right)^b}{1 + \left(\frac{x_5}{k_{i_5}}\right)^c + \left(\frac{x_6}{k_{t_5}}\right)^b} \frac{1}{1 + \left(\frac{[\mathrm{TOP}]}{i_{\mathrm{BmalTop}}}\right)^c} - d_{y_5} y_5$$

*Rev-erb*

$$\frac{\mathrm{d}y_3}{\mathrm{d}t} = V_{3\max} \frac{1 + g\left(\frac{x_1}{k_{t_3}}\right)^b}{1 + \left(\frac{x_2}{k_{i_4}}\right)^c \left(\frac{x_1}{k_{t_3}}\right)^b + \left(\frac{x_1}{k_{t_3}}\right)^b} \frac{1}{1 + \left(\frac{[\mathrm{NFIL}]}{i_{\mathrm{RevNfil}}}\right)^c} - d_{y_3} y_3$$

The protein is degraded and grows by translation, where $d_{\mathrm{PROTEIN}}$ is the degradation rate, and $r_{\mathrm{PROTEIN}}$ is a translation rate that either describes only the translation of the gene to the cytoplasmic protein (first four variables of Table 1), or both the translation step as well as the import of this protein into the nucleus (last four variables of Table 1).

For the elements of Supplementary Table 1 the step from genes to proteins has the same structure:

$$\frac{\mathrm{d}PROTEIN}{\mathrm{d}t} = r_{\mathrm{PROTEIN}}\, Gene - d_{\mathrm{PROTEIN}}\, PROTEIN$$

*Transcription*

The transcription of all variables of Supplementary Table 1 and of *Bmal1* and *Rev-Erb* follow dynamics with the following structure:

$$\frac{\mathrm{d}Gene}{\mathrm{d}t} = V_{\mathrm{Gene}}\, \mathbb{T}(Gene) - d_{\mathrm{Gene}}\, Gene,$$

where $V_{\mathrm{Gene}}$ is the maximal transcription rate of the gene *Gene*, $d_{\mathrm{Gene}}$ is the degradation rate of the gene, and $\mathbb{T}(Gene)$ is the transcription function as defined below, that includes the interactions between different elements.

*List of model parameters. * For PPARα, TOP1, PAR bZIP and NFIL3, $r_{PROTEIN}$ is the rate of translation and*

*the import of the protein into the nucleus.*

| Parameter name | Parameter symbol |
|---|---|
| transcription function as stated below | $\mathbb{T}(Gene)$ |
| maximal transcription rate of the gene *Gene* | $V_{Gene}$ |
| degradation rate of the gene *Gene* | $d_{Gene}$ |
| transcription fold activations | $f_{Gene}$ |
| activation rates | $a_{Gene}$ |
| inhibition rates for inhibition by one protein | $i_{Gene}$ |
| inhibition rate of TOP 1 on *Bmal1* | $i_{BmalTop}$ |
| inhibition rate of NFIL3 on *Rev-Erb* | $i_{RevNfil}$ |
| activation rate of CLOCKBMAL on *Top1* | $a_{Top}$ |
| activation rate of PAR bZIP on *Top1* | $a_{TopPar}$ |
| inhibition rate of PER/CRY on *Top1* | $i_{Top}$ |
| inhibition rate of NFIL3 on *Top1* | $i_{TopNfil}$ |
| Hill coefficient of activation | *b* |
| Hill coefficient of inhibition | *c* |
| rate of translation* of the protein *PROTEIN* | $r_{PROTEIN}$ |
| degradation rate of the protein *PROTEIN* | $d_{PROTEIN}$ |
| *Transcription functions* | |

For simplicity, the Hill coefficients of transcription for activation and inhibition, b and c, are the same for all equations.

*Pparα*

$$\mathbb{T}(Ppar) = \frac{1 + f_{Ppar}\left(\dfrac{x_1}{a_{Ppar}}\right)^b}{1 + \left(\dfrac{x_2}{i_{Ppar}}\right)^c \left(\dfrac{x_1}{a_{Ppar}}\right)^b + \left(\dfrac{x_1}{a_{Ppar}}\right)^b}$$

*PAR bZip*

$$\mathbb{T}(Par) = \frac{1 + f_{Par}\left(\dfrac{x_1}{a_{Par}}\right)^b}{1 + \left(\dfrac{x_2}{i_{Par}}\right)^c \left(\dfrac{x_1}{a_{Par}}\right)^b + \left(\dfrac{x_1}{a_{Par}}\right)^b}$$

*Ugt1a1*

$$\mathbb{T}(Ugt) = \frac{1 + f_{Ugt}\left(\dfrac{[PPAR]}{a_{Ugt}}\right)^b}{1 + \left(\dfrac{[PPAR]}{a_{Ugt}}\right)^b}$$

*Nfil3*

$$\mathbb{T}(Nfil) = \frac{1 + f_{\text{Nfil}}\left(\frac{x_6}{a_{\text{Nfil}}}\right)^b}{1 + \left(\frac{x_5}{i_{\text{Nfil}}}\right)^c + \left(\frac{x_6}{a_{\text{Nfil}}}\right)^b}$$

*Ces*

$$\mathbb{T}(Ces) = \frac{1 + f_{\text{Ces}}\left(\frac{[\text{NFIL}]}{a_{\text{Ces}}}\right)^b}{1 + \left(\frac{x_5}{i_{\text{Ces}}}\right)^c + \left(\frac{[\text{NFIL}]}{a_{\text{Ces}}}\right)^b}$$

*Abcb1*

$$\mathbb{T}(Abcb) = \frac{1 + f_{\text{Abcb}}\left(\frac{[\text{PAR}]}{a_{\text{Abcb}}}\right)^b}{1 + \left(\frac{[\text{NFIL}]}{i_{\text{Abcb}}}\right)^c + \left(\frac{[\text{PAR}]}{a_{\text{Abcb}}}\right)^b}$$

*Abcc*

$$\mathbb{T}(Abcc) = \frac{1 + f_{\text{Abcc}}\left(\frac{[\text{PAR}]}{a_{\text{Abcc}}}\right)^b}{1 + \left(\frac{[\text{NFIL}]}{i_{\text{Abcc}}}\right)^c + \left(\frac{[\text{PAR}]}{a_{\text{Abcc}}}\right)^b}$$

*Top1*

$$\mathbb{T}(Top) = \frac{1 + f_{\text{Top}}\left(\frac{x_1}{a_{\text{Top}}}\right)^b}{1 + \left(\frac{x_2}{i_{\text{Top}}}\right)^c \left(\frac{x_1}{a_{\text{Top}}}\right)^b + \left(\frac{x_1}{a_{\text{Top}}}\right)^b} \frac{1 + f_{\text{TopPar}}\left(\frac{[\text{PAR}]}{a_{\text{TopPar}}}\right)^b}{1 + \left(\frac{[\text{NFIL}]}{i_{\text{TopNfil}}}\right)^c + \left(\frac{[\text{PAR}]}{a_{\text{TopPar}}}\right)^b}$$

### Implementation of posttranscriptional modifications

For the fit of the SW480 cell line and the liver tissue, we replace the equations for *CES2* and *ABCC* transcription with the following set of equations, which allow us to improve the fit of *CES2* and *ABCC*:

$$\frac{\mathrm{d}Ces^*}{\mathrm{d}t} = V_{\text{Ces}}\mathbb{T}(Ces^*) - d_{\text{Ces}}\, Ces^*,$$

$$\frac{\mathrm{d}Ces^{**}}{\mathrm{d}t} = s_{\text{Ces}^*}Ces^* - d_{\text{Ces}^*}\, Ces^{**},$$

$$\frac{\mathrm{d}Ces^{***}}{\mathrm{d}t} = s_{\text{Ces}^*}Ces^{**} - d_{\text{Ces}^*}\, Ces^{***},$$

$$\frac{\mathrm{d}Ces}{\mathrm{d}t} = s_{\mathrm{Ces}*}Ces^{***} - d_{\mathrm{Ces}*}\,Ces,$$

$$\frac{\mathrm{d}Abcc^{*}}{\mathrm{d}t} = V_{\mathrm{Abcc}}\mathbb{T}(Abcc^{*}) - d_{\mathrm{Abcc}}\,Abcc^{*},$$

$$\frac{\mathrm{d}Abcc^{**}}{\mathrm{d}t} = s_{\mathrm{Abcc}*}Abcc^{*} - d_{\mathrm{Abcc}*}\,Abcc^{**},$$

$$\frac{\mathrm{d}Abcc}{\mathrm{d}t} = s_{\mathrm{Abcc}*}Abcc^{**} - d_{\mathrm{Abcc}*}\,Abcc,$$

with $\mathbb{T}(Ces\,*)$ and $\mathbb{T}(Abcc\,*)$ given by the equations above, replacing *Ces* by *Ces* * and *Abcc* by *Abcc* *.

Another version of the model is stated in the following.

TOP, $TOP_c$ and $TOP_n$ are mRNA, cytoplasmic and nuclear proteins associated with the gene TOP1.

NF, $NF_c$ and $NF_n$ are mRNA, cytoplasmic and nuclear proteins associated with the gene NFIL3.

CES, $CES_c$ and $CES_n$ are mRNA, cytoplasmic and nuclear proteins associated with the gene CES2.

$ABC_{CPT}$, $ABC_{CPTc}$ and $ABC_{CPTn}$ are mRNA, cytoplasmic and nuclear proteins associated with the gene group ABCB1, ABCC1, ABCC2.

$ABC_{SN}$, $ABC_{SNc}$ and $ABC_{SNn}$ are mRNA, cytoplasmic and nuclear proteins associated with the gene group ABCC1, ABCC2, ABCG1.

UGT, $UGT_c$ and $UGT_n$ are Mrna, cytoplasmic and nuclear proteins associated with the gene group of UGT1As.

The protein abundances in (Ballesta et al. 2011), equation (12) correspond here to the dynamical variables with the index n associated with the same genes or gene group.

From the core-clock model:

$x_1, x_5, x_6$ and $z_4$, the pool of nuclear complexes PER/CRY, as defined in (Relogio et al. 2011).

Parameters:

*sf* = 1 is an overall scaling factor,

*ki51* = 3.3 is the inhibition constant of BMAL1 transcription by TOP1.

*o* = 1 is the Hill coefficient of inhibition of BMAL1 transcription by TOP1,

*u* = 1 is the Hill coefficient of inhibition of TOP1 transcription by $NFIL3_n$,

*ki6b* is the inhibition constant of Top transcription by $NFIL3_n$,

Furthermore the following parameters for the mRNA i are defined:

- the degradation rate of the associated nuclear protein dn(i),
- the degradation rate of the associated cytoplasmic protein dc(i),
- the production rate of the associated cytoplasmic protein *kp*(*i*),
- the import rate to the nucleus of the associated cytoplasmic protein *kn*(*i*),
- the maximal transcription rate *Vmax*(*i*),
- the degradation rate of the mRNA *d*(*i*),
- the Hill coefficient of activation of transcription *act*(*i*),
- the Hill coefficient of inhibition of transcription *inh*(*i*),
- the rate constant of transcription *kt*(*i*),
- the inhibition constant of transcription *ki*(*i*),
- and the fold activation of transcription $f_{Act}(i)$,

The nuclear protein associated with TOP1 is created by importing the cytoplasmic protein associated with TOP1 and it is degraded:

$$\frac{dTOP_n}{dt} = sf(kn(TOP)TOP_c - dn(TOP)TOP_n)$$

Production of TOP1: The cytoplasmic protein associated with TOP1 is created based on the available mRNA and is reduced by being transported into the nucleus or by degradation:

$$\frac{dTOP_c}{dt} = sf(kp(TOP)(TOP + TOP_0) - kn(TOP)TOP_c - dc(TOP)TOP_c)$$

where $TOP_0$ is the initial value of TOP1 gene expression.

The equations for the import into the nucleus and the production of the cytoplasmic protein of NF, CES, ABC_, and UGT1A1 are formulated in analogue.

To account for the activation of BMAL1 by TOP1, the transcription term for BMAL1, $V5max \dfrac{1+i(x_6/kt5)^n}{1+(x_5/ki5)^m+(x_6/kt5)^n}$ is extended as $V5max \dfrac{1+i(x_6/kt5)^n}{1+(x_5/ki5)^m+(x_6/kt5)^n} \dfrac{(x_8/ki51)^o}{1+(x_8/ki51)^o}$ , for parameters see (Relogio et al. 2011).

The transcription of the gene is given for the mRNA $i$ as

$$\frac{di}{dt} = sf(Vmax(i)trans(i) - d(i)i)$$

with *trans(i)* the following terms for transcription:

$$trans(TOP)$$
$$= \frac{1 + \left(x_1/kt\,(TOP)\right)^{act(TOP)}}{1 + \left(\left(PC/ki\,(TOP)\right)^{inh(TOP)} + 1\right)\left(x_1/kt\,(TOP)\right)^{act(TOP)}} \frac{1}{1 + (NF_n/ki\,6b)^u}$$

$$trans(NF) = \frac{1}{1 + \left(x_6/ki\,(NF)\right)^{inh(Nf)}}$$

$$trans(CES) = \frac{1 + f_{Act}(CES)\left(NF_n/kt\,(CES)\right)^{act(CES)}}{1 + \left(\left(x_5/ki\,(CES)\right)^{inh(CES)} + 1\right)\left(NF_n/kt\,(CES)\right)^{act(CES)}}$$

$$trans(ABC_{SN}) = \frac{1 + f_{Act}(ABC_{SN})\left(P\,C/k\,t(ABC_{SN})\right)^{act(ABC_{SN})}}{1 + \left(\left(NF_n/ki\,(ABC_{SN})\right)^{inh(ABC_{SN})} + 1\right)\left(PC/kt\,(ABC_{SN})\right)^{act(ABC_{SN})}}$$

$$trans(ABC_{CPT}) = \frac{1 + f_{Act}(ABC_{CPT})\left(P\,C/k\,t(ABC_{CPT})\right)^{act(ABC_{CPT})}}{1 + \left(\left(NF_n/ki\,(ABC_{CPT})\right)^{inh(ABC_{CPT})} + 1\right)\left(PC/kt\,(ABC_{CPT})\right)^{act(ABC_{CPT})}}$$

$$trans(UGT) = \frac{1 + f_{Act}(UGT)\big(PC/kt\,(UGT)\big)^{act(UGT)}}{+\big(PC/kt\,(UGT)\big)^{act(UGT)}}$$

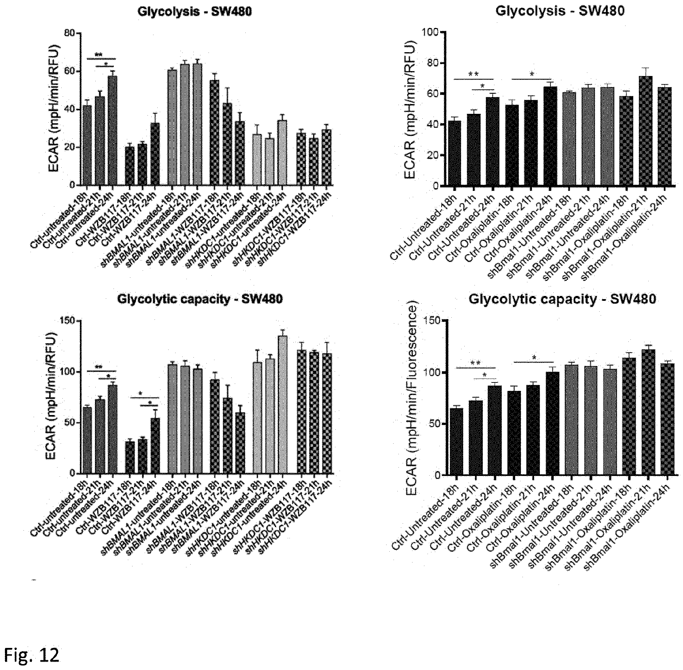

Several studies show that the timing of drug administration affects how, for example, cancer cells react to the drug. It was shown that in colorectal cancer, the effect of drug administration of Oxaliplatin, a platinum complex that is used effectively for CRC treatment and WZB 117, a glucose transporter inhibitor, is time-dependent and this effect is lost when the circadian clock of the cells is impaired after downregulating the core-clock gene *BMAL1* (Fig. 12, (Fuhr et al., 2018)).

Fig. 12 (which is Fig. 4 from Fuhr et al. EBioMedicine (2018) illustrates that BMAL1- and HKDC1-KD leads to metabolic changes in SW480 colorectal cancer cells and altered drug response in a time-dependent manner. (a) Glycolysis of SW480 control, shBMAL1 and shHKDC1 cells at different time-points after synchronization. Cells were either untreated or treated with WZB 117 or Oxaliplatin. Mean ± SEM, n = 5. (b) Glycolytic capacity of SW480 control, shBMAL1 and shHKDC1 cells treated at three different time points after synchronization (18 h, 21 h, 24 h). Cells were either untreated or treated with WZB117 or Oxaliplatin. Mean ± SEM, n = 5.

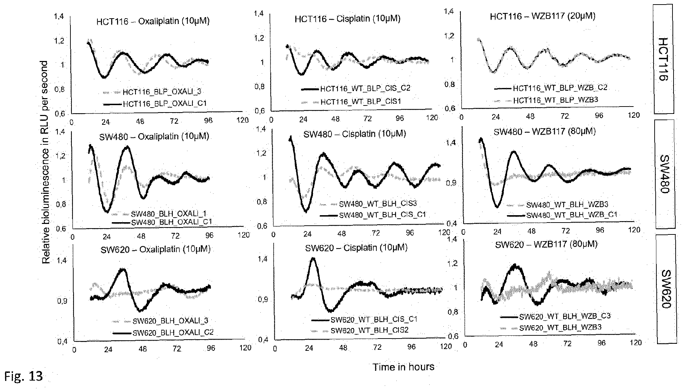

In addition to the colon cancer cell line SW480, data were obtained for two different colon cancer cell lines HCT116 and SW620, which are derived from a primary tumour and a metastatic lymph node, respectively, that point towards to regulation of drug response via the circadian core-clock gene *BMAL1*. Here, the three cell lines were treated with several anticancer drugs, including WZB 117, Oxaliplatin and cisplatin, and the rhythmic promoter activity of *BMAL1* was measured over several days (Fig. 13). The data shows that the drug application altered the rhythmicity of the core-clock gene *BMAL1* and hence the circadian clock machinery, indicating an important interplay between drug effect and the circadian clock in cancer cells. More specifically, HCT116, SW480 and SW620 colon cancer cells were treated with Oxaliplatin, Cisplatin and WZB117. BMAL1-Promoter activity was measured using live-cell bioluminescence recording over five days. Shown is one representative plot for the control (black) and the treated (grey dashed) condition, n = 3.

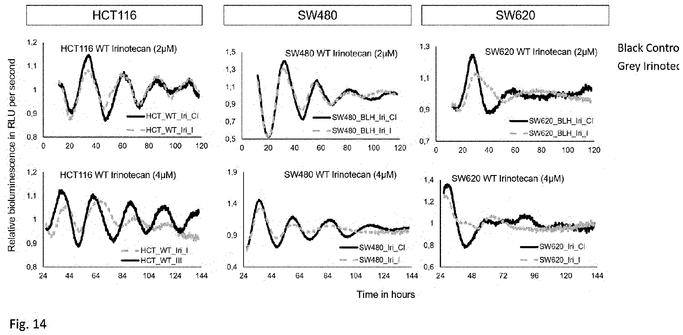

It was also shown a similar effect of drug administration on the circadian clock using the drug Irinotecan, which is widely used in combination with other anti-cancer drugs (e.g. Oxaliplatin) in treating colorectal cancer patients. It is known that Irinotecan, a topoisomerase I inhibitor with complex metabolism and known activity against colorectal cancer is directly linked to clock gene *BMAL1* expression (Dulong et al., 2015). The data shows that Irinotecan administration alters *BMAL1* rhythmic promoter activity in in three colon cancer cells investigated differently (Fig. 14). More specifically, HCT116, SW480 and SW620 colon cancer cells were treated with Irinotecan at different concentrations. BMAL1-Promoter activity was measured using live-cell bioluminescence recording over five days. Shown is one representative plot for the control (black) and the treated (grey) condition, n = 3.

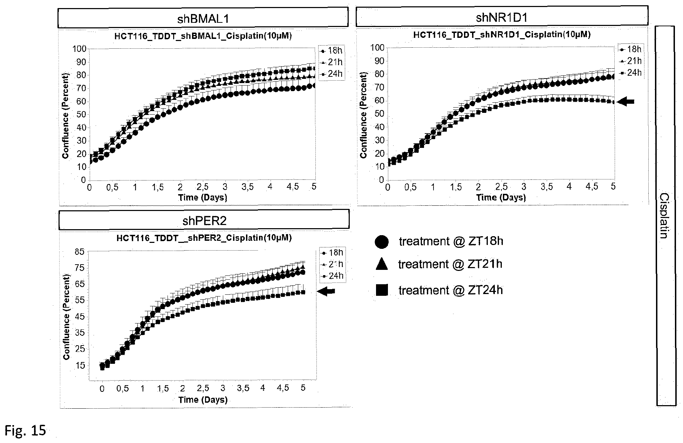

In addition to the *BMAL1* rhythmicity data, preliminary data were obtained pointing towards the differential regulation of cancer cell proliferation based on the timing of drug administration. Here stable core-clock downregulated (shBMAL1, shNR1D1 and shPER2) HCT116 cancer cell lines treated with 10μM Cisplatin at 18h, 21h and 24h after cell synchronization are used. There was detected reduced cell proliferation for shNR1D1 and shPER2 cells, when cells were treated at 24h after synchronization compared to the other timepoints (Fig. 15). Cell proliferation was measured using confluence over five days. n = 3.

In another study on pancreatic cancer, it was shown that the timing of treatment administration has different effects in cell survival depending also on the additional perturbations generated to the cells (Fig. 16). Here, it was shown that pancreatic cancer cells at different tumour stage have different response to the time-dependent administration of the chemotherapy drug gemcitabine, which is widely used for treating pancreatic cancer.

In particular, it was shown that the SMAD4 proficient pancreatic cancer cells (Panc1) are more resistant to treatment, while for the SMAD4 deficient AsPC1 cells this effect is not observed. Also, after the downregulation of core-clock gene *NR1D1* or clock-regulated gene *SMAD4* in Panc1, the differential drug response to different treatment time points is impaired.

Again, referring to Fig. 16 (Figure from Li et al. iScience (2020)), cytotoxicity assays for time-dependent gemcitabine treatment are illustrated with (A) Pipeline for the timing administration treatment and (B-C) At 17h, 20h, 23h after cell synchronization, gemcitabine was added to the cells. 72h after treatment, the number of living cells was quantified. Depicted are the comparisons to the 17h time point (mean ± SEM, n=3, *p < 0.05, **p < 0.01, ***p < 0.001).

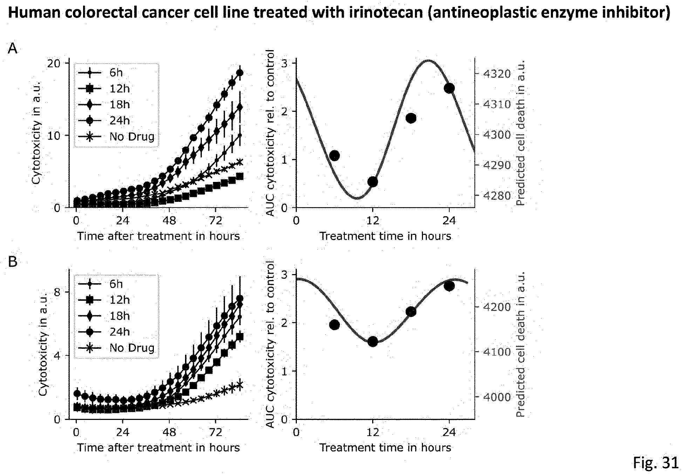

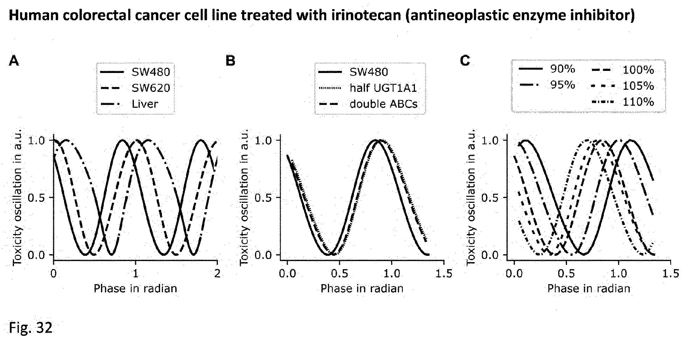

Fig. 31 shows the cytotoxicity of SW480 and SW620 cell lines in response to treatment with irinotecan at different times after synchronization (left) and the resulting circadian toxicity profile in comparison with the model predictions (right). Fig. 32 illustrates how the model can be used to simulate alterations that might occur in patients, i.e. comparing the SW480 and SW620 cell line with healthy liver tissue (Fig. 32A), investigating the effect of reduced UGT1A1 or overexpression of ABC transporters as reported for some patients (Fig. 32B), or variations in *PER2* oscillations (Fig. 32C), which might for example result from different light conditions.

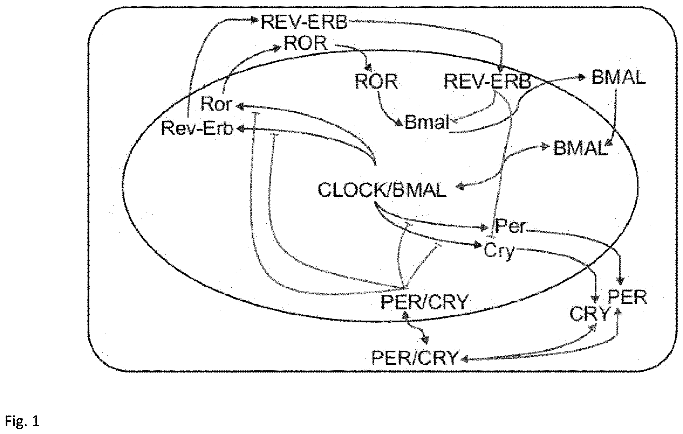

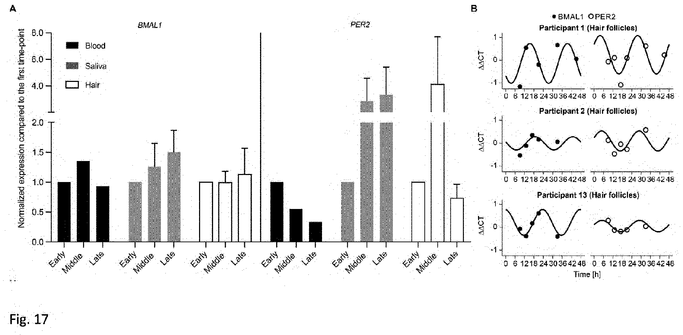

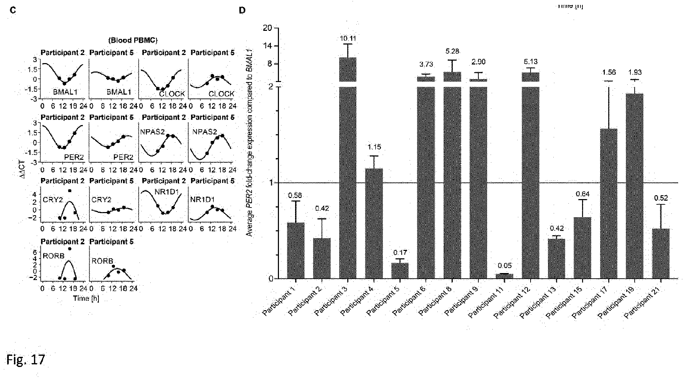

Fig. 17 illustrates BMAL1 and PER2 expression display variation during the day in human blood, hair and saliva samples. (A) Three time-point comparison of BMAL1 and PER2 expression for the averaged data of all Participants in figure 1. Expression data is compared to the first time-point (Early). For hair and saliva data Early, Middle and Late time-points represent 9h, 17h and 21h, respectively. For PBMCs data Early, Middle and Late time-points represent 10h, 16h and 19h, respectively. Depicted are mean + SEM. (B) Time-course RT-qPCR measurements normalized to the mean of all time points ($\Delta\Delta$CT) of BMAL1 (and PER2 of Participant 1, 2, and 13 with a fitted linear sine-cosine function (period = 24 h). For Participant 1, we collected one additional sample at 21h on the 2nd day. Harmonic regression best p-values for tested periods (20-28h): Participant 1; BMAL1 (0.517, period = 21.4h), PER2 (0.353, period = 24.0h). Participant 2; BMAL1 (0.038, period = 20.0h), PER2 (0.276, period = 28.0h). Participant 13; BMAL1 (0.014, period = 20h), PER2 (0.086, period = 21.4h). (C) Time-course RT-qPCR measurements of human PBMCs normalized to the mean of all time points ($\Delta\Delta$CT) of BMAL1, CLOCK, NPAS2, PER2, CRY2, NR1D1, and RORB of Participant 2 and 5 with a fitted linear sine-cosine function (period = 24 h). Harmonic regression best p-values: Participant 2; BMAL1 (3.05E-01, period = 20h), CLOCK (6.31E-02, period = 28h), NPAS2 (1.67E-01, period = 20h), PER2 (4.78E-04, period = 20.8h), CRY2 (7.17E-01, period = 20h), NR1D1 (1.48E-01, period = 28h) and RORB (7.58E-01, period = 20h). Participant 5; BMAL1 (5.56E-01, period = 20h), CLOCK (6.81E-01, period = 28h), NPAS2 (9.75E-02, period = 28h, PER2 (1.23E-01, period = 28h), CRY2 (5.40E-01, period = 28h), NR1D1 (6.43E-01, period = 28h) and RORB (7.73E-01, period = 28h). (D) Average PER2 expression compared to BMAL1 using saliva time-course data for each participant (mean + SEM).

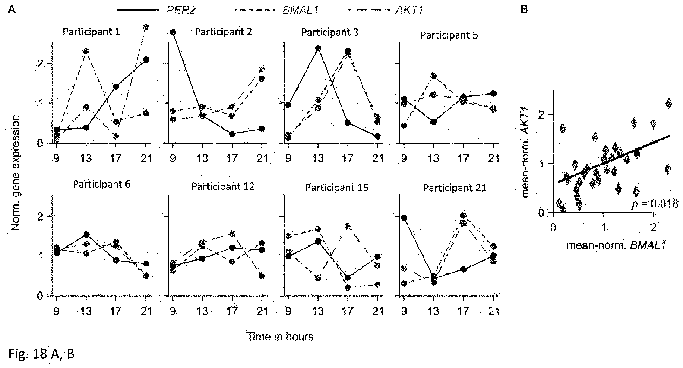

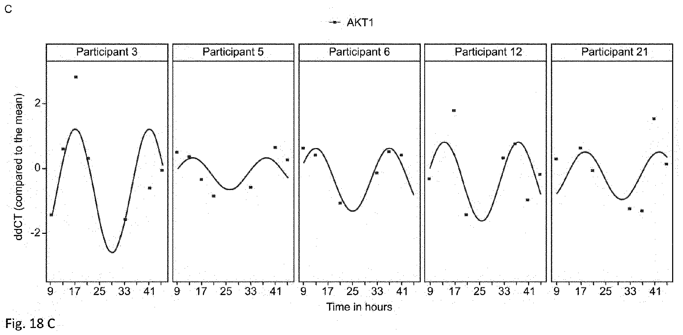

Figure 18 illustrates Gene expression of BMAL1 and AKT1 covary. (A) Mean-normalized gene expression profile for the three participants for whom the gene AKT1 was measured besides BMAL1 and PER2. The two days were treated as repetitions. The diurnal variation of AKT1 follows BMAL1. (B) The data points from the mean-normalized time-series of BMAL1 and AKT1 correlate, linear regression with p = 0.018. (C) Harmonic regression plots for the participants with at least 5 time points. Depicted values are based on individual best fitting period (20h - 28h, see also table A).

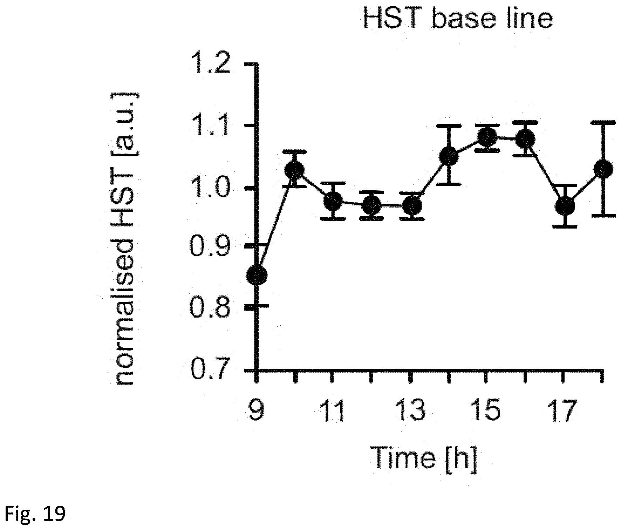

Figure 19 illustrates HST base line measurements. Depicted are mean values for three participants (9h-18h in one-hour intervals, N = 3, mean $\pm$ SEM). The HST measurements were randomly distributed across three measurement days with one day break in between. The red full circles represent the time point chosen for the subsequent training sessions.

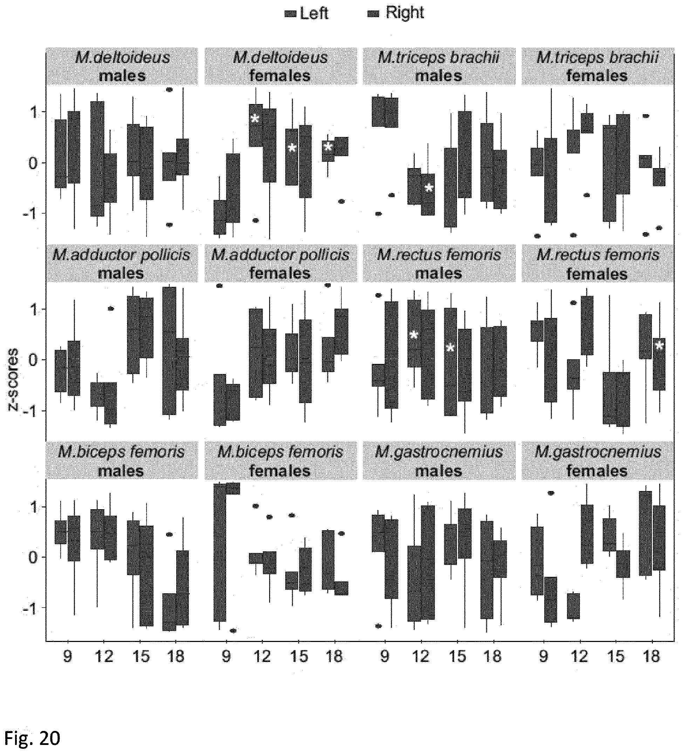

Figure 20 illustrates Myotonometric analysis shows daily variation in muscle tone (frequency, F) for female and male participants. Only participants who completed all training sessions were included in the MyotonPRO measurements (N=12). Mean of normalized scores for the myotonometric parameter frequency [Hz] for each training session (T1-9h, T2-12h, T3-15h, T4-18h) and each muscle: M. Deltoideus, M. Triceps Brachii, M.adductor pollicis, M. rectus femoris, M. biceps femoris, M. gastrocnemius. The measurements were carried out from top to bottom on the right (Right bar) and the corresponding left (Left bar) side of the body. Corresponding statistics for intrapersonal variation between each time points can be found in Supplementary Table B. * p < 0.05, compared to time point 9h. For detailed overview see table B.

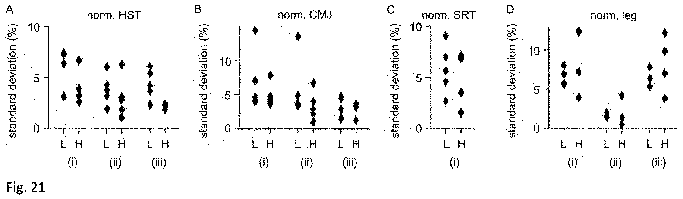

Figure 21 illustrates Standard deviations of normalized sports and muscle tone data (L: group with low BMAL1, H: group with high BMAL1). Mean standard deviation calculated on the normalized sports performance and the normalized muscle tone data for different (i) repetitions and timepoints, (ii) timepoints, (iii) repetitions (for details see Methods). (A) HST, (B) CMJ, (C) SRT (no repetitions were measured, thus the standard deviation (i) over all data is the same as (ii) over timepoints), (D) muscle tone of the leg muscles (M. rectus femoris, M. biceps femoris, M. gastrocnemius).

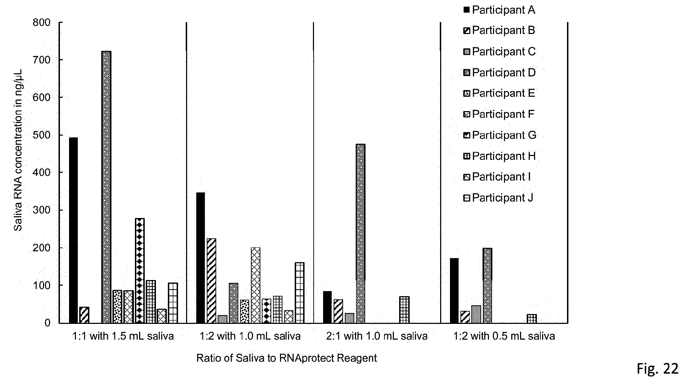

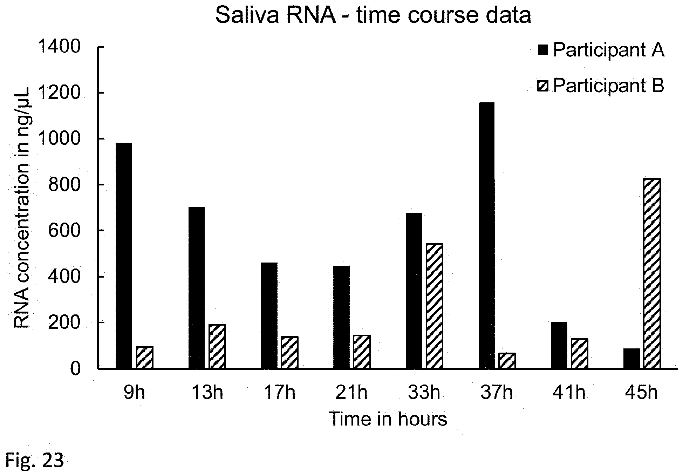

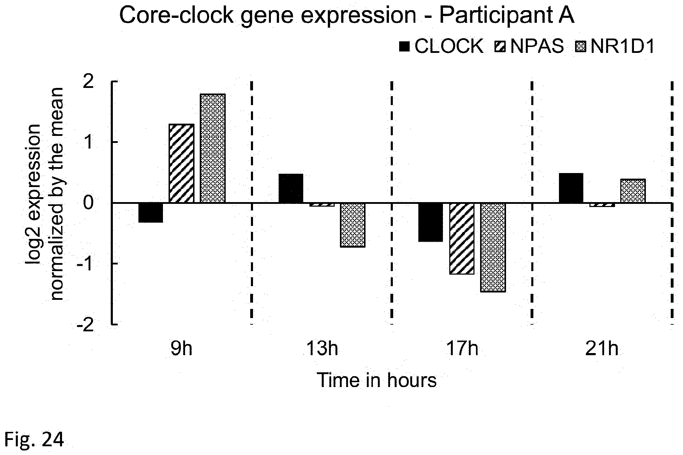

Figure 22 illustrates an optimized ratio between collected saliva and RNA stabilization reagent, which yields the best RNA concentration. Figure 23 and 24 illustrate the saliva RNA concentration measured over time with an optimized ratio determined in Fig 23 (1:1 with 1.5mL saliva) and the expression of core clock genes in these samples.

**Tables**

Table A- Harmonic regression results of AKT1 for the best fitting period (see Fig. 18).

| Participant | qvals | pvals | Acrophase [h] | amplitude | Period [h] |
|---|---|---|---|---|---|
| Participant 3 | 0.249 | 0.178 | 18 | 1.506 | 28 |
| Participant 5 | 0.061 | 0.017 | 12 | 1.042 | 28 |
| Participant 6 | 0.011 | 0.001 | 11 | 1.068 | 26.6 |
| Participant 12 | 0.696 | 0.229 | 14 | 1.067 | 20 |
| Participant 21 | 0.249 | 0.167 | 14 | 1.386 | 28 |

Table B: Harmonic regression analysis (Figure 20).

| Mesor | qvals | pvals | Acrophase [h] | Acrophase [radians] | Amplitude | Period [h] | Condition |
|---|---|---|---|---|---|---|---|
| -0.50 | 0.49 | 0.18 | 15.83 | 4.14 | 1.22 | 23.6 | BMAL1_Participant 5 |
| 0.42 | 0.60 | 0.42 | 25.32 | 6.62 | 0.92 | 26.2 | PER2_Participant 5 |
| -0.12 | 0.24 | 0.01 | 6.67 | 1.74 | 1.80 | 28 | BMAL1_Participant 15 |
| -0.03 | 0.70 | 0.54 | 6.60 | 1.72 | 0.58 | 28 | PER2_Participant 15 |
| -0.03 | 0.31 | 0.05 | 0.59 | 0.15 | 1.49 | 20.9 | BMAL1_Participant 21 |
| 0.29 | 0.35 | 0.06 | 6.30 | 1.70 | 1.48 | 20 | PER2_Participant 21 |
| -2.03 | 0.52 | 0.23 | 15.08 | 3.94 | 4.38 | 25.2 | BMAL1_Participant 8 |
| -0.63 | 0.70 | 0.58 | 12.54 | 3.28 | 1.26 | 28 | PER2_Participant 8 |
| -0.71 | 0.21 | 0.01 | 18.33 | 4.79 | 3.85 | 22.9 | BMAL1_Participant 9 |
| -0.06 | 0.51 | 0.15 | 3.29 | 0.86 | 2.33 | 20 | PER2_Participant 9 |
| 0.02 | 0.15 | 0.01 | 20.33 | 5.32 | 1.78 | 20.8 | BMAL1_Participant 17 |
| 0.38 | 0.70 | 0.40 | 0.51 | 0.13 | 0.72 | 28 | PER2_Participant 17 |
| -0.01 | 0.48 | 0.14 | 21.05 | 5.51 | 0.28 | 22.1 | BMAL1_Participant 13 |
| 0.05 | 0.56 | 0.15 | 24.33 | 6.36 | 0.11 | 28 | PER2_Participant 13 |
| -0.16 | 0.70 | 0.54 | 17.04 | 4.46 | 0.42 | 28 | PER2_Participant 11 |
| -0.16 | 0.73 | 0.63 | 15.67 | 4.10 | 0.34 | 28 | BMAL1_Participant 11 |
| -1.47 | 0.45 | 0.06 | 14.34 | 3.75 | 3.50 | 26.6 | BMAL1_Participant 1 |
| 0.29 | 0.31 | 0.02 | 21.82 | 5.71 | 1.71 | 26.9 | PER2_Participant 1 |
| -0.85 | 0.21 | 0.02 | 16.80 | 4.39 | 2.65 | 23.5 | BMAL1_Participant 3 |
| -0.61 | 0.21 | 0.01 | 13.75 | 3.59 | 1.63 | 22.6 | PER2_Participant 3 |
| -0.12 | 0.10 | 0.03 | 13.36 | 3.49 | 1.43 | 20 | BMAL1_Participant 19 |
| 0.38 | 0.56 | 0.07 | 27.44 | 7.18 | 0.76 | 28 | PER2_Participant 19 |
| 0.17 | 0.88 | 0.58 | 4.98 | 1.30 | 1.07 | 20 | BMAL1_Participant 2 |
| 0.02 | 0.69 | 0.29 | 11.28 | 2.95 | 0.50 | 20.3 | PER2_Participant 2 |
| 0.00 | 0.60 | 0.31 | 21.15 | 5.53 | 0.56 | 23.4 | BMAL1_Participant 4 |
| -0.04 | 0.63 | 0.23 | 20.07 | 5.25 | 0.28 | 20.7 | PER2_Participant 4 |
| -0.01 | 0.67 | 0.28 | 1.88 | 0.49 | 0.71 | 20 | BMAL1_Participant 12 |
| -0.13 | 0.84 | 0.53 | 18.94 | 4.95 | 0.76 | 20 | PER2_Participant 12 |
| -0.60 | 0.46 | 0.09 | 12.22 | 3.19 | 1.46 | 26.7 | BMAL1_Participant 6 |
| -0.05 | 0.40 | 0.09 | 12.83 | 3.35 | 0.42 | 22 | PER2_Participant 6 |

Table C: List of participants and tests (MEQ, Sports tests, Molecular tests, Myotonometry) performed. Y = Yes, participant has carried out the test (see Fig. 3).

| | | | sports tests | | | molecular tests | | | myotonometry |
|---|---|---|---|---|---|---|---|---|---|
| Participant# | gender | MEQ | # training sessions | # round of tests | HST_long | saliva | hair | blood | MyotonPRO |
| 1 | male | intermediate | - | - | - | Y | Y | - | - |
| 2 | male | intermediate | - | - | - | Y | Y | Y | - |
| 3 | female | intermediate | - | - | **Y** | Y | - | - | - |
| 4 | male | moderate morning | - | - | **Y** | Y | Y | - | - |
| 5 | female | moderate morning | 4 | 1 | Y | Y | - | Y | Y |
| 6 | female | moderate evening | 4 | 1 | - | Y | - | - | Y |
| 7 | female | intermediate | 4 | 1 | - | - | - | - | Y |
| 8 | female | intermediate | 4 | 1 | - | Y | - | - | Y |
| 9 | female | moderate evening | 4 | 1 | - | Y | - | - | Y |
| 10 | male | intermediate | 4 | 1 | - | - | - | - | Y |
| 11 | male | intermediate | 4 | 1 | - | Y | - | - | Y |
| 12 | female | intermediate | - | - | - | Y | - | - | - |
| 13 | male | intermediate | 4 | 1 | - | Y | Y | - | Y |
| 14 | male | intermediate | 4 | 1 | - | - | - | - | Y |
| 15 | male | intermediate | 4 | 1 | - | Y | - | - | Y |
| 16 | male | moderate evening | 4 | 1 | - | - | - | - | Y |
| 5 | female | noderate morning | 3 | 2 | - | - | - | - | - |
| 8 | female | intermediate | 3 | 2 | - | - | - | - | - |
| 17 | female | noderate evening | 4 | 2 | - | Y | - | - | - |
| 18 | male | intermediate | 3 | 2 | - | - | - | - | - |
| 10 | male | intermediate | 4 | 2 | - | - | - | - | - |
| 19 | male | noderate morning | 3 | 2 | - | Y | - | - | - |
| 20 | male | moderate evening | 4 | 2 | - | - | - | - | - |
| 21 | male | moderate morning | 4 | 2 | - | Y | - | - | - |

EP 4 204 591 B1

Altogether, the data shows that in the context of cancer treatment, the timing of drug application has a crucial role on cancer cell survival, which in turn, affects the overall outcome of the therapy in cancer patients. In this regard, the model applied in the method can be used to not only predict the most effective timing of therapy based on the individual's rhythm (e.g. of core-clock or drug target genes) but also to monitor and overcome circadian rhythm disruptions induced by (chemo)therapy regimens.

**Claims**

1. Method of assessing circadian rhythm or circadian profile of a subject having cancer and assessing a timing of administration of a medicament to said subject having cancer, wherein said method comprises the steps of:

   • Providing at least three samples of saliva, more preferably four samples of saliva, from said subject, wherein said samples have been taken at different time points over the day;
   • Determining gene expression of the following genes in each of said samples:

      a. of at least two members of the core-clock network in each of said samples, in particular of at least two members of the following genes, of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, and
      b. at least one gene involved in the metabolism of a medicament to be administered to said subject having cancer, and
      c. at least one drug target gene that is a target of the medicament to be administered, and

   • Assessing and predicting by means of a computational step based on said expression levels of said genes over the day the circadian rhythm of said subject and assessing a timing of administration of said medicament to said subject, comprising assessing the optimal time of administration of said medicament to said subject and/or assessing the non-optimal time of administration of said medicament to said subject.

2. Method according to claim 1, wherein assessing the circadian rhythm of said subject comprises in the computational step determining a periodic function for each of said determined genes that approximates said gene expression levels for each of said genes, preferably comprising curve fitting of a non-linear periodic model function to the respective gene expression levels, wherein the curve fitting is preferably carried out by means of an ODE model.

3. Method according to claim 1, wherein gene expression is determined using a method selected from quantitative PCR, RT-qPCR, NanoString, sequencing and microarray.

4. Method according to any of claims 1 to 3, wherein gene expression is determined using NanoString.

5. Method according to claim 4, wherein the at least one further gene involved in the metabolism of the medicament to be administered is at least one of Ugt1a1 and Abcb1.

6. Method according to any of claims 1 to 5, wherein said assessing the timing of administration of said medicament to said subject comprises evaluating the predicted gene expression levels and/or evaluating expression phenotypes based on the determined and/or predicted gene expression levels.

7. Method according to any of claims 1 to 6, wherein the computational step comprises processing the determined gene expression levels to derive characteristic data for each of said genes, said processing comprising determining the mean expression level of expression of a gene and normalizing the gene expression levels using the mean expression level.

8. Method according to claim 7, wherein said characteristic data comprise:

   • the amplitude of change of expression of a gene, and/or the amplitude relative to one of the other genes, and/or
   • the mean expression level of expression of a gene, and/ or and/or the mean relative to one of the other genes, and/or
   • the peak expression level of a gene, and/or the peak relative to one of the other genes., and/or
   • the amplitude of change of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
• the relative difference of the amplitudes of change of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
• the mean expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/ or
• the relative difference of the mean expression levels of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
• the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
• the relative difference of the peak expression levels of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
• the time of the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,
• the relative difference of the times of the peak expression level of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,

wherein the amplitude, period and phase expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC are extracted from the determined expression levels and/or the respectively fitted periodic function.

9. Method according to claim 7 or 8, wherein the computational step further comprises

• fitting a network computational model to the derived characteristic data that comprises a representation of the periodic time course of the expression levels for each of said determined genes as well as a representation of the periodic time course of the expression level for at least one, preferably a plurality of further gene(s) included in a gene regulatory network that includes said genes; and/or
• training a machine learning algorithm on the derived characteristic data to form the network computational model, particularly optimize in terms of the representation of the periodic time course of the expression level for the at least one further gene.

10. Method according to any of claims 7 to 9, wherein assessing the timing of administration of said medicament to said subject comprises in the computational step
fitting a prediction computational model on data obtained from said fitted periodic functions and/or said network computational model, wherein the prediction computational model is based on machine learning including at least one classification method and/or at least one clustering method, wherein said method(s) are preferably selected from the group comprising:
K-nearest neighbor algorithm, unsupervised clustering, deep neural networks, random forest algorithm, and support vector machines.

11. Method according to any of claims 1 to 10, wherein

• the medicament is Filgrastim and the target gene is *Csf3r* and the indication is acute myeloid leukemia; or
• the medicament is Rituximab and the target gene is selected from the group comprising *Fcgr2b, Ms4a1, and Fcgr3*; and the indication is rheumatoid arthritis and Non-Hodgkin's lymphoma; or
• the medicament is bevacizumab and the target gene is *Fcgr2b, Vegfa, Fcgr3*; and the indication is Colorectal cancer and Non-small cell lung cancer; or
• the medicament is trastuzumab and the target gene is Fcgr2b, Erbb2, Egfr, Fcgr3 and the indication is Breast cancer; or
• the medicament is Imatinib and the target gene is Ptgs1, Kit, Slc22a2, Abcg2, Pdgfra, Pdgfrb, Ddri, Abca3, Abl1, Ret, Abcbia and the indication is Chronic myeloid leukemia; or
• the medicament is Pemetrexed and the target gene is Tyms, Atic, Gart, Slc29a1 and the indication is Mesothelioma and Non-small cell lung cancer; or
• the medicament is Capecitabine and the target gene is Cda, Tymp, Tyms, Cesig, Dpyd and the indication is Breast cancer and colorectal cancer; or
• the medicament is Erlotinib (tyrosine kinase inhibitor, anticancer drug) and the target gene is EGFR, Ras/Raf/MAPK, and PIK3/AKT, tumour, and the indication is Tumour inhibition (ZT1 > > ZT13); or
• the medicament is Sunitinib (tyrosine kinase inhibitor, anticancer drug) and the target gene is Cyp3a11 (liver, duodenum, jejunum) abcbia (liver, duodenum, jejunum, lung) and the indication is renal cell cancer and pancreatic neuroendocrine tumours; or
• the medicament is Lapatinib (dual tyrosine kinase inhibitor interrupting the HER2/neu and EGFR pathways, anticancer drug) and the target gene is EGFR/Ras/Raf/MAPK, Errfi1, Duspi, liver, Hbegf, Tgfα, Eref, liver, and the indication is solid tumours such as breast and lung cancer; or
• the medicament is Roscovitine (seliciclib, CDK inhibitor, anticancer drug) and the target gene is Cyp3a11, Cyp3a13, liver, and the indication is non-small cell lung cancer, NSCLC, and leukemia; or
• the medicament is Everolimus (mTOR inhibitor, anticancer drug, immunosuppressant) and the target gene is mTOR/Fbxw7/P70S6K, tumour, and the indication is breast cancer; or
• the medicament is Irinotecan (Topi inhibitor, anticancer drug) and the target gene is Ces2, Ugt1a1, abcbia, abcbib (liver and ileum), abcc2, ileum, and the indication for colorectal cancer, advanced pancreatic cancer and small cell lung cancer; or
• the medicament is Tamoxifen (antiestrogenic, anticancer drug) and the target gene is Cyp2d10, Cyp2d22, Cyp3a11, liver, and the indication is breast cancer; or
• the medicament Bleomycin (toxicant and anticancer drug) and the target gene is NRF2/glutathione antioxidant defence and the indication is pulmonary fibrosis, palliative treatment in the management malignant neoplasm (trachea, bronchus, lung), squamous cell carcinoma, and lymphomas.

12. Use of a kit for collecting samples of saliva for providing the collected samples of saliva for a method of any of claim 1 or any one of claims 3 to 11.

**Patentansprüche**

1. Verfahren zur Bewertung des zirkadianen Rhythmus oder des zirkadianen Profils eines Subjekts mit Krebs und zur Bewertung des Zeitpunkts der Verabreichung eines Medikaments an das Subjekt mit Krebs, wobei das Verfahren die folgenden Schritte umfasst:

   • Bereitstellen von mindestens drei Speichelproben, bevorzugter vier Speichelproben, von dem Subjekt, wobei die Proben zu verschiedenen Zeitpunkten über den Tag entnommen wurden;
   • Bestimmen der Genexpression der folgenden Gene in jeder der Proben:

      a. von mindestens zwei Mitgliedern des Kern-Uhr-Netzwerks in jeder der Proben, insbesondere von mindestens zwei Mitgliedern der folgenden Gene, der Gruppen umfassend ARNTL (BMAL1), ARNTL2, CLOCK, PER1, PER2, PER3, NPAS2, CRY1, CRY2, NR1D1, NR1D2, RORA, RORB, RORC, insbesondere ARNTL (BMAL1) und PER2, und
      b. mindestens ein Gen, das an dem Stoffwechsel eines Medikaments beteiligt ist, das an das Subjekt mit Krebs verabreicht werden soll, und
      c. mindestens ein Arzneimittelzielgen, das ein Ziel des zu verabreichenden Medikaments ist, und

   • Bewerten und Vorhersagen des zirkadianen Rhythmus des Subjekts mittels eines Rechenschritts auf der Grundlage der Expressionsniveaus der Gene über den Tag und Bewerten des Zeitpunkts der Verabreichung des Medikaments an das Subjekt, umfassend das Bewerten des optimalen Zeitpunkts der Verabreichung des Medikaments an das Subjekt und/oder das Bewerten des nicht optimalen Zeitpunkts der Verabreichung des Medikaments an das Subjekt.

2. Verfahren nach Anspruch 1, wobei das Bewerten des zirkadianen Rhythmus des Subjekts im Rechenschritt das Bestimmen einer periodischen Funktion für jedes der bestimmten Gene umfasst, die die Genexpressionsniveaus für jedes der Gene approximiert, vorzugsweise umfassend das Kurvenanpassen einer nichtlinearen periodischen Modellfunktion an die jeweiligen Genexpressionsniveaus, wobei das Kurvenanpassen vorzugsweise mittels eines ODE-Modells durchgeführt wird.

3.   Verfahren nach Anspruch 1, wobei die Genexpression unter Verwendung eines Verfahrens ausgewählt aus quantitativer PCR, RT-qPCR, NanoString, Sequenzierung und Mikroarray bestimmt wird.

4.   Verfahren nach einem der Ansprüche 1 bis 3, wobei die Genexpression unter Verwendung von NanoString bestimmt wird.

5.   Verfahren nach Anspruch 4, wobei das mindestens eine weitere Gen, das am Stoffwechsel des zu verabreichenden Medikaments beteiligt ist, mindestens eines von Ugt1a1 und Abcb1 ist.

6.   Verfahren nach einem der Ansprüche 1 bis 5, wobei das Bewerten des Zeitpunkts der Verabreichung des Medikaments an das Subjekt das Bewerten der vorhergesagten Genexpressionsniveaus und/oder das Bewerten von Expressionsphänotypen auf der Grundlage der bestimmten und/oder vorhergesagten Genexpressionsniveaus umfasst.

7.   Verfahren nach einem der Ansprüche 1 bis 6, wobei der Rechenschritt das Verarbeiten der bestimmten Genexpressionsniveaus umfasst, um charakteristische Daten für jedes der Gene abzuleiten, wobei das Verarbeiten das Bestimmen des mittleren Expressionsniveaus der Expression eines Gens und das Normalisieren der Genexpressionsniveaus unter Verwendung des mittleren Expressionsniveaus umfasst.

8.   Verfahren nach Anspruch 7, wobei die charakteristischen Daten Folgendes umfassen:

   • die Amplitude der Änderung der Expression eines Gens und/oder die Amplitude relativ zu einem der anderen Gene und/oder
   • das mittlere Expressionsniveau der Expression eines Gens und/oder und/oder das mittlere relativ zu einem der anderen Gene und/oder
   • das Peak-Expressionsniveau eines Gens und/oder der Peak relativ zu einem der anderen Gene und/oder
   • die Amplitude der Änderung der Expression von ARNTL (BMAL1) und/oder ARNTL2 und/oder CLOCK und/oder NPAS2 und/oder PER1 und/oder PER2 und/oder PER3 und/oder CRY1 und/oder CRY2 und/oder NR1D1 und/oder NR1D2 und/oder RORA und/oder RORB und/oder RORC über den Tag und/oder
   • die relative Differenz der Amplituden der Änderung der Expression von zwei beliebigen von ARNTL (BMAL1) und/oder ARNTL2 und/oder CLOCK und/oder NPAS2 und/oder PER1 und/oder PER2 und/oder PER3 und/oder CRY1 und/oder CRY2 und/oder NR1D1 und/oder NR1D2 und/oder RORA und/oder RORB und/oder RORC und/oder
   • das mittlere Expressionsniveau der Expression von ARNTL (BMAL1) und/oder ARNTL2 und/oder CLOCK und/oder NPAS2 und/oder PER1 und/oder PER2 und/oder PER3 und/oder CRY1 und/oder CRY2 und/oder NR1D1 und/oder NR1D2 und/oder RORA und/oder RORB und/oder RORC und/oder
   • die relative Differenz der mittleren Expressionsniveaus der Expression von zwei beliebigen von ARNTL (BMAL1) und/oder ARNTL2 und/oder CLOCK und/oder NPAS2 und/oder PER1 und/oder PER2 und/oder PER3 und/oder CRY1 und/oder CRY2 und/oder NR1D1 und/oder NR1D2 und/oder RORA und/oder RORB und/oder RORC und/oder
   • das Peak-Expressionsniveau von ARNTL (BMAL1) und/oder ARNTL2 und/oder CLOCK und/oder NPAS2 und/oder PER1 und/oder PER2 und/oder PER3 und/oder CRY1 und/oder CRY2 und/oder NR1D1 und/oder NR1D2 und/oder RORA und/oder RORB und/oder RORC über den Tag und/oder
   • die relative Differenz der Peak-Expressionsniveaus von zwei beliebigen von ARNTL (BMAL1) und/oder ARNTL2 und/oder CLOCK und/oder NPAS2 und/oder PER1 und/oder PER2 und/oder PER3 und/oder CRY1 und/oder CRY2 und/oder NR1D1 und/oder NR1D2 und/oder RORA und/oder RORB und/oder RORC und/oder
   • die Zeit des Peak-Expressionsniveaus von ARNTL (BMAL1) und/oder ARNTL2 und/oder CLOCK und/oder NPAS2 und/oder PER1 und/oder PER2 und/oder PER3 und/oder CRY1 und/oder CRY2 und/oder NR1D1 und/oder NR1D2 und/oder RORA und/oder RORB und/oder RORC,
   • die relative Differenz der Zeiten des Peak-Expressionsniveaus von zwei beliebigen von ARNTL (BMAL1) und/oder ARNTL2 und/oder CLOCK und/oder NPAS2 und/oder PER1 und/oder PER2 und/oder PER3 und/oder CRY1 und/oder CRY2 und/oder NR1D1 und/oder NR1D2 und/oder RORA und/oder RORB und/oder RORC,

wobei das Amplituden-, Perioden- und Phasen-Expressionsniveau der Expression von ARNTL (BMAL1) und/oder ARNTL2 und/oder CLOCK und/oder NPAS2 und/oder PER1 und/oder PER2 und/oder PER3 und/oder CRY1 und/oder CRY2 und/oder NR1D1 und/oder NR1D2 und/oder RORA und/oder RORB und/oder RORC aus den bestimmten Expressionsniveaus und/oder der jeweils angepassten periodischen Funktion extrahiert werden.

9. Verfahren nach Anspruch 7 oder 8, wobei der Rechenschritt ferner Folgendes umfasst:

• Anpassen eines Netzwerkrechenmodells an die abgeleiteten charakteristischen Daten, das eine Darstellung des periodischen Zeitverlaufs der Expressionsniveaus für jedes der bestimmten Gene sowie eine Darstellung des periodischen Zeitverlaufs des Expressionsniveaus für mindestens ein, vorzugsweise eine Mehrzahl von weiteren Gen(en) umfasst, die in einem Genregulationsnetzwerk enthalten sind, das die Gene enthält; und/oder
• Trainieren eines Maschinenlernalgorithmus an den abgeleiteten charakteristischen Daten, um das Netzwerkrechenmodell zu bilden, insbesondere im Hinblick auf die Darstellung des periodischen Zeitverlaufs des Expressionsniveaus für das mindestens eine weitere Gen zu optimieren.

10. Verfahren nach einem der Ansprüche 7 bis 9, wobei das Bewerten des Zeitpunkts der Verabreichung des Medikaments an das Subjekt in dem Rechenschritt Folgendes umfasst: Anpassen eines Vorhersagerechenmodells an Daten, die aus den angepassten periodischen Funktionen und/oder dem Netzwerkrechenmodell erhalten werden, wobei das Vorhersagerechenmodell auf maschinellem Lernen basiert, das mindestens ein Klassifizierungsverfahren und/oder mindestens ein Clustering-Verfahren enthält, wobei das Verfahren bzw. die Verfahren vorzugsweise ausgewählt sind aus der Gruppe umfassend:
K-Nächster-Nachbar-Algorithmus, unbeaufsichtigtes Clustering, tiefe neuronale Netzwerke, Random-Forest-Algorithmus und Support-Vektor-Maschinen.

11. Verfahren nach einem der Ansprüche 1 bis 10, wobei

• das Medikament Filgrastim ist und das Zielgen *Csf3r* ist und die Indikation akute myeloische Leukämie ist; oder
• das Medikament Rituximab ist und das Zielgen ausgewählt ist aus der Gruppe umfassend *Fcgr2b, Ms4a1 und Fcgr3*; und die Indikation rheumatoide Arthritis und Non-Hodgkin-Lymphom ist; oder
• das Medikament Bevacizumab ist und das Zielgen *Fcgr2b, Vegfa, Fcgr3* ist; und die Indikation Kolorektalkrebs und nicht-kleinzelliger Lungenkrebs ist; oder
• das Medikament Trastuzumab ist und das Zielgen Fcgr2b, Erbb2, Egfr, Fcgr3 ist und die Indikation Brustkrebs ist; oder
• das Medikament Imatinib ist und das Zielgen Ptgs1, Kit, Slc22a2, Abcg2, Pdgfra, Pdgfrb, Ddri, Abca3, Abl1, Ret, Abcb1a ist und die Indikation chronische myeloische Leukämie ist; oder
• das Medikament Pemetrexed ist und das Zielgen Tyms, Atic, Gart, Slc29a1 ist und die Indikation Mesotheliom und nicht-kleinzelliger Lungenkrebs ist; oder
• das Medikament Capecitabin ist und das Zielgen Cda, Tymp, Tyms, Cesig, Dpyd ist und die Indikation Brustkrebs und Kolorektalkrebs ist; oder
• das Medikament Erlotinib (Tyrosinkinaseinhibitor, Antikrebsarzneimittel) ist und das Zielgen EGFR, Ras/Raf/MAPK und PIK3/AKT, Tumor ist und die Indikation Tumorinhibition (ZT1 >> ZT13) ist; oder
• das Medikament Sunitinib (Tyrosinkinaseinhibitor, Antikrebsarzneimittel) ist und das Zielgen Cyp3a11 (Leber, Duodenum, Jejunum) abcbia (Leber, Duodenum, Jejunum, Lunge) ist und die Indikation Nierenzellkrebs und neuroendokrine Pankreastumore ist; oder
• das Medikament Lapatinib (dualer Tyrosinkinaseinhibitor, der die HER2/neu- und EGFR-Wege unterbricht, Antikrebsarzneimittel) ist und das Zielgen EGFR/Ras/Raf/MAPK, Errfi1, Dusp1, Leber, Hbegf, Tgfα, Eref, Leber ist und die Indikation solide Tumore wie Brust- und Lungenkrebs ist; oder
• das Medikament Roscovin (Seliciclib, CDK-Inhibitor, Antikrebsarzneimittel) ist und das Zielgen Cyp3a11, Cyp3a13, Leber ist und die Indikation nicht-kleinzelliger Lungenkrebs, NSCLC und Leukämie ist; oder
• das Medikament Everolimus (mTOR-Inhibitor, Antikrebsarzneimittel, Immunsuppressivum) ist und das Zielgen mTOR/Fbxw7/P70S6K, Tumor ist und die Indikation Brustkrebs ist; oder
• das Medikament Irinotecan (Top1-Inhibitor, Antikrebsarzneimittel) ist und das Zielgen Ces2, Ugt1a1, abcb1a, abcbib (Leber und Ileum), abcc2, Ileum ist und die Indikation für Kolorektalkrebs, fortgeschrittenen Pankreaskrebs und kleinzelligen Lungenkrebs ist; oder
• das Medikament Tamoxifen (Antiöstrogen, Antikrebsarzneimittel) ist und das Zielgen Cyp2d10, Cyp2d22, Cyp3a11, Leber ist und die Indikation Brustkrebs ist; oder
• das Medikament Bleomycin (Arzneimittel und Antikrebsarzneimittel) ist und das Zielgen NRF2/Glutathion-Antioxidans-Abwehr ist und die Indikation Lungenfibrose, palliative Behandlung beim Management von malignem Neoplasma (Luftröhre, Bronchus, Lunge), Plattenepithelkarzinom und Lymphomen ist.

12. Verwendung eines Kits zum Sammeln von Speichelproben zum Bereitstellen der gesammelten Speichelproben für ein Verfahren nach einem der Ansprüche 1 oder einem der Ansprüche 3 bis 11.

**Revendications**

1. Méthode d'évaluation du rythme circadien ou du profil circadien d'un sujet atteint d'un cancer et d'évaluation d'un moment d'administration d'un médicament audit sujet atteint d'un cancer, dans laquelle ladite méthode comprend les étapes consistant à :

   • fournir au moins trois échantillons de salive, plus préférablement quatre échantillons de salive, provenant dudit sujet, lesdits échantillons ayant été prélevés à différents moments au cours de la journée ;
   • déterminer l'expression génique des gènes suivants dans chacun desdits échantillons :

      a. Parmi au moins deux membres du réseau d'horloge centrale dans chacun desdits échantillons, en particulier d'au moins deux membres des gènes suivants, des groupes comprenant ARNTL (BMAL1), ARNTL2, CLOCK, PER1, PER2, PER3, NPAS2, CRY1, CRY2, NR1D1, NR1D2, RORA, RORB, RORC, en particulier ARNTL (BMAL1) et PER2, et
      b. au moins un gène impliqué dans le métabolisme d'un médicament à administrer audit sujet atteint d'un cancer, et
      c. au moins un gène cible de médicament qui est une cible du médicament à administrer, et

   • évaluer et prédire au moyen d'une étape de calcul basée sur lesdits niveaux d'expression desdits gènes au cours de la journée le rythme circadien dudit sujet et évaluer un moment d'administration dudit médicament audit sujet, comprenant l'évaluation du moment optimal d'administration dudit médicament audit sujet et/ou l'évaluation du moment non optimal d'administration dudit médicament audit sujet.

2. Méthode selon la revendication 1, dans laquelle l'évaluation du rythme circadien dudit sujet comprend dans l'étape de calcul la détermination d'une fonction périodique pour chacun desdits gènes déterminées qui s'approche desdits niveaux d'expression génique pour chacun desdits gènes, comprenant de préférence un ajustement de courbe d'une fonction de modèle périodique non linéaire par rapport aux niveaux d'expression génique respectifs, dans laquelle l'ajustement de courbe est de préférence réalisé au moyen d'un modèle ODE.

3. Méthode selon la revendication 1, dans laquelle l'expression génique est déterminée en utilisant une méthode choisie parmi une PCR quantitative, une RT-qPCR, NanoString, un séquençage et une micropuce.

4. Méthode selon l'une quelconque des revendications 1 à 3, dans laquelle l'expression génique est déterminée en utilisant NanoString.

5. Méthode selon la revendication 4, dans laquelle l'au moins un gène supplémentaire impliqué dans le métabolisme du médicament à administrer est au moins l'un parmi Ugt1a1 et Abcb1.

6. Méthode selon l'une quelconque des revendications 1 à 5, dans laquelle ladite évaluation du moment d'administration dudit médicament audit sujet comprend l'évaluation des niveaux d'expression génique prédits et/ou l'évaluation des phénotypes d'expression sur la base des niveaux d'expression génique déterminés et/ou prédits.

7. Méthode selon l'une quelconque des revendications 1 à 6, dans laquelle l'étape de calcul comprend le traitement des niveaux d'expression génique déterminés pour dériver des données caractéristiques pour chacun desdits gènes, ledit traitement comprenant la détermination du niveau d'expression moyen d'expression d'un gène et la normalisation des niveaux d'expression génique en utilisant le niveau d'expression moyen.

8. Méthode selon la revendication 7, dans laquelle lesdites données caractéristiques comprennent :

   • l'amplitude de changement d'expression d'un gène, et/ou l'amplitude relative à l'un des autres gènes, et/ou
   • le niveau d'expression moyen d'expression d'un gène, et/ou et/ou la moyenne relative à l'un des autres gènes, et/ou
   • le niveau d'expression maximal d'un gène, et/ou le maximum relatif à l'un des autres gènes, et/ou
   • l'amplitude de changement d'expression d'ARNTL (BMAL1) et/ou ARNTL2 et/ou CLOCK, et/ou NPAS2 et/ou PER1 et/ou PER2 et/ou PER3 et/ou CRY1 et/ou CRY2 et/ou NR1D1 et/ou NR1D2 et/ou RORA et/ou RORB et/ou RORC au cours de la journée, et/ou

• la différence relative des amplitudes de changement d'expression de deux quelconques parmi ARNTL (BMAL1) et/ou ARNTL2 et/ou CLOCK, et/ou NPAS2 et/ou PER1 et/ou PER2 et/ou PER3 et/ou CRY1 et/ou CRY2 et/ou NR1D1 et/ou NR1D2 et/ou RORA et/ou RORB et/ou RORC, et/ou

• le niveau d'expression moyen d'expression d'ARNTL (BMAL1) et/ou ARNTL2 et/ou CLOCK, et/ou NPAS2 et/ou PER1 et/ou PER2 et/ou PER3 et/ou CRY1 et/ou CRY2 et/ou NR1D1 et/ou NR1D2 et/ou RORA et/ou RORB et/ou RORC, et/ou

• la différence relative des niveaux d'expression moyens d'expression de deux quelconques parmi ARNTL (BMAL1) et/ou ARNTL2 et/ou CLOCK, et/ou NPAS2 et/ou PER1 et/ou PER2 et/ou PER3 et/ou CRY1 et/ou CRY2 et/ou NR1D1 et/ou NR1D2 et/ou RORA et/ou RORB et/ou RORC, et/ou

• le niveau d'expression maximal d'ARNTL (BMAL1) et/ou ARNTL2 et/ou CLOCK, et/ou NPAS2 et/ou PER1 et/ou PER2 et/ou PER3 et/ou CRY1 et/ou CRY2 et/ou NR1D1 et/ou NR1D2 et/ou RORA et/ou RORB et/ou RORC au cours de la journée, et/ou

• la différence relative des niveaux d'expression maximaux de deux quelconques parmi ARNTL (BMAL1) et/ou ARNTL2 et/ou CLOCK, et/ou NPAS2 et/ou PER1 et/ou PER2 et/ou PER3 et/ou CRY1 et/ou CRY2 et/ou NR1D1 et/ou NR1D2 et/ou RORA et/ou RORB et/ou RORC, et/ou

• le temps du niveau d'expression maximal de ARNTL (BMAL1) et/ou ARNTL2 et/ou CLOCK, et/ou NPAS2 et/ou PER1 et/ou PER2 et/ou PER3 et/ou CRY1 et/ou CRY2 et/ou NR1D1 et/ou NR1D2 et/ou RORA et/ou RORB et/ou RORC,

• la différence relative des temps du niveau d'expression maximal de deux quelconques parmi ARNTL (BMAL1) et/ou ARNTL2 et/ou CLOCK, et/ou NPAS2 et/ou PER1 et/ou PER2 et/ou PER3 et/ou CRY1 et/ou CRY2 et/ou NR1D1 et/ou NR1D2 et/ou RORA et/ou RORB et/ou RORC,

dans laquelle l'amplitude, la période et le niveau d'expression de phase d'expression d'ARNTL (BMAL1) et/ou ARNTL2 et/ou CLOCK, et/ou NPAS2 et/ou PER1 et/ou PER2 et/ou PER3 et/ou CRY1 et/ou CRY2 et/ou NR1D1 et/ou NR1D2 et/ou RORA et/ou RORB et/ou RORC sont extraits des niveaux d'expression déterminés et/ou de la fonction périodique respectivement ajustée.

9. Méthode selon la revendication 7 ou 8, dans laquelle l'étape de calcul comprend en outre

• l'ajustement d'un modèle de calcul de réseau aux données caractéristiques dérivées qui comprend une représentation de l'évolution temporelle périodique des niveaux d'expression pour chacun desdits gènes déterminés ainsi qu'une représentation de l'évolution temporelle périodique du niveau d'expression pour au moins un, de préférence une pluralité de gènes supplémentaires inclus dans un réseau de régulation génique qui comprend lesdits gènes ; et/ou

• l'entraînement d'un algorithme d'apprentissage automatique sur les données caractéristiques dérivées pour former le modèle de calcul de réseau, en particulier l'optimisation en termes de représentation de l'évolution temporelle périodique du niveau d'expression pour l'au moins un gène supplémentaire.

10. Méthode selon la revendication 7 à 9, dans laquelle l'évaluation du moment d'administration dudit médicament audit sujet comprend dans l'étape de calcul

l'ajustement d'un modèle de calcul de prédiction sur des données obtenues à partir desdites fonctions périodiques ajustées et/ou dudit modèle de calcul de réseau, le modèle de calcul de prédiction étant basé sur l'apprentissage automatique comprenant au moins une méthode de classification et/ou au moins une méthode de regroupement, ladite ou lesdites méthodes étant de préférence choisies dans le groupe comprenant :

l'algorithme des K plus proches voisins, le regroupement non supervisé, les réseaux neuronaux profonds, l'algorithme de forêt aléatoire et les machines à vecteurs de support.

11. Méthode selon l'une quelconque des revendications 1 à 10, dans laquelle

• le médicament est le filgrastim et le gène cible est *Csf3r* et l'indication est la leucémie myéloïde aiguë ; ou

• le médicament est le rituximab et le gène cible est choisi dans le groupe comprenant *Fcgr2b, Ms4a1* et *Fcgr3* ; et l'indication est la polyarthrite rhumatoïde et le lymphome non hodgkinien ; ou

• le médicament est le bévacizumab et le gène cible est *Fcgr2b, Vegfa, Fcgr3* ; et l'indication est le cancer colorectal et le cancer du poumon non à petites cellules ; ou

• le médicament est le trastuzumab et le gène cible est Fcgr2b, Erbb2, Egfr, Fcgr3 et l'indication est le cancer du sein ; ou

• le médicament est l'imatinib et le gène cible est Ptgs1, Kit, Slc22a2, Abcg2, Pdgfra, Pdgfrb, Ddr1, Abca3, Abl1, Ret, Abcbia et l'indication est la leucémie myéloïde chronique ; ou

• le médicament est le pémétrexed et le gène cible est Tyms, Atic, Gart, Slc29a1 et l'indication est le mésothéliome et le cancer du poumon non à petites cellules ; ou

• le médicament est la capécitabine et le gène cible est Cda, Tymp, Tyms, Cesig, Dpyd et l'indication est le cancer du sein et le cancer colorectal ; ou

• le médicament est l'erlotinib (inhibiteur de la tyrosine kinase, médicament anticancéreux) et le gène cible est EGFR, Ras/Raf/MAPK et PIK3/AKT, tumeur, et l'indication est l'inhibition tumorale (ZT1 > > ZT13) ; ou

• le médicament est le sunitinib (inhibiteur de la tyrosine kinase, médicament anticancéreux) et le gène cible est Cyp3a11 (foie, duodénum, jéjunum) abcbia (foie, duodénum, jéjunum, poumon) et l'indication est le cancer des cellules rénales et les tumeurs neuroendocrines du pancréas ; ou

• le médicament est le lapatinib (inhibiteur double de la tyrosine kinase interrompant les voies HER2/neu et EGFR, médicament anticancéreux) et le gène cible est EGFR/Ras/Raf/MAPK, Errfi1, Dusp1, foie, Hbegf, Tgf$\alpha$, Eref, foie, et l'indication est les tumeurs solides telles que le cancer du sein et du poumon ; ou

• le médicament est la roscovitine (séliciclib, inhibiteur de CDK, médicament anticancéreux) et le gène cible est Cyp3a11, Cyp3a13, foie, et l'indication est le cancer du poumon non à petites cellules, CPNPC, et la leucémie ; ou

• le médicament est l'évérolimus (inhibiteur de mTOR, médicament anticancéreux, immunosuppresseur) et le gène cible est mTOR/Fbxw7/P70S6K, tumeur, et l'indication est le cancer du sein ; ou

• le médicament est l'irinotécan (inhibiteur de Top1, médicament anticancéreux) et le gène cible est Ces2, Ugt1a1, abcbia, abcbib (foie et iléon), abcc2, iléon, et l'indication pour le cancer colorectal, le cancer avancé du pancréas et le cancer du poumon à petites cellules ; ou

• le médicament est le tamoxifène (anti-œstrogène, médicament anticancéreux) et le gène cible est Cyp2d10, Cyp2d22, Cyp3a11, foie, et l'indication est le cancer du sein ; ou

• le médicament la bléomycine (médicament toxique et anticancéreux) et le gène cible est la défense antioxydante NRF2/glutathion et l'indication est la fibrose pulmonaire, traitement palliatif dans la prise en charge du néoplasme malin (trachée, bronches, poumon), le carcinome épidermoïde et les lymphomes.

12. Utilisation d'un kit de collecte d'échantillons de salive pour fournir les échantillons de salive collectés pour une méthode selon l'une quelconque de la revendication 1 ou l'une quelconque des revendications 3 à 11.

Fig. 1

Fig. 2

Fig. 3

Fig. 4

Fig 5

Fig 6

Fig 7

Fig 8

Fig 9a

Fig. 9b

Fig 10a

Fig. 10b

Fig. 11

Fig. 12

Fig. 13

Fig. 14

Fig. 15

**A**

Cell Synchronization                                                                                      Treatment Time

0:00          3:00          6:00          9:00          12:00          15:00          18:00          21:00          24:00

Time [h]

**B**                    Panc1                                    **C**                    AsPC1

Fig. 16

Fig. 17

Fig. 17

Fig. 18 A, B

C

Fig. 18 C

HST base line

Fig. 19

Fig. 20

Fig. 21

Fig. 22

Fig. 23

Fig. 24

**A**

Irinotecan Pharmacokinetics/-dynamics DNA damage

*Core clock model*

*Full model:*
*Clock + irinotecan-relevant genes*

**B**

**Models fitted to experimental data for**

Mouse
liver tissue

Cell line
derived from primary
colon cancer*

Cell line
derived from
metastatic site*

SW480

SW620

* from same
patient

Fig. 25

Fig. 26

# Human colorectal cancer cell line

Fig. 27

# Human colorectal cancer cell line

Fig. 28

## Healthy cells (A) and human colorectal cancer cell line (B)

Fig. 29

## Healthy cells (A) and human colorectal cancer cell line (B)

Fig. 30

**Human colorectal cancer cell line treated with irinotecan (antineoplastic enzyme inhibitor)**

Fig. 31

**Human colorectal cancer cell line treated with irinotecan (antineoplastic enzyme inhibitor)**

Fig. 32

**Human pancreas cancer cell line**

Fig. 33

# Human pancreas cancer cell line (1. – 2.) and human healthy cells (3.)

**1. Fit of *PER2* and *BMAL1***

**2. Extraction of phase for *REV-ERBα***

**3. Alignment of drug-specific genes**

**4. Pharmacodynamics/-kinetics (Gemcitabine)**
**5. Toxicity prediction (Gemcitabine)**

Fig. 34

Human pancreas cancer cell line treated with gemcitabine (nucleoside metabolic inhibitor)

Fig. 35

Saliva from healthy humans, prediction for treatment with irinotecan (antineoplastic enzyme inhibitor)

Fig. 36

Saliva from healthy humans, prediction for treatment with irinotecan (antineoplastic enzyme inhibitor)

Fig. 37A

**Saliva from healthy humans, prediction for treatment with irinotecan (antineoplastic enzyme inhibitor)**

Fig. 37B

Fig. 38

Saliva in comparison to mammalian data

Fig. 39

**Colorectal cancer cell line (Caco2 modelled by Dulong et al., 2015) in comparison to mammalian liver.**

Fig. 40

## Saliva from healthy humans, predictions for light therapy

Fig. 41A

Fig. 41B

Fig. 42

Fig. 43

Fig. 44

**Saliva from healthy humans**

Fig. 45

## Saliva from healthy humans

Fig. 46

Fig. 47

Human colorectal cancer cell line with two different treatments.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011094745 A2

### Non-patent literature cited in the description

- **KIM, JAE KYOUNG ; DANIEL B FORGER.** A Mechanism for Robust Circadian Timekeeping via Stoichiometric Balance. *Molecular Systems Biology,* 2012, vol. 8, 630, https://doi.org/10.1038/msb.2012.62
- **I. A. SOLOVYOV.** *Genetic Control of Circadian Rhythms and Aging*
- **JESSICA A. DEITZ et al.** Optimal Techniques for mRNA Extraction from Neonatal Salivary Supernatant. *Neonatology,* 2012
- **CONSTANCE AHOWESSO.** *Sex and Dosing-Time Dependencies in Irinotecan-Induced Circadian Disruption*
- **GEISS G et al.** Direct multiplexed measurement of gene expression with color-coded probe pairs. *Nature Biotechnology,* 08 February 2008, vol. 26, 317-25
- **STROEBEL, A.M. ; BERGNER, M. ; REULBACH, U. ; BIERMANN, T. ; GROEMER, T.W. ; KLEIN, I. ; KORNHUBER, J.** Statistical methods for detecting and comparing periodic data and their application to the nycthemeral rhythm of bodily harm: A population based study. *Journal of Circadian Rhythms,* 2010, vol. 8, 10
- **RELOGIO et al.** *Plos Comp Bio,* 2011
- **BALLESTA et al.** *CPT11 activity is assessed by the amount of irreversible DNA/TOP1/SN38 complexes, chosen as the output variable because of its experimentallyproven correlation with CPT11 cytotoxicity*
- **FUHR et al.** *EBioMedicine,* 2018
- **LI et al.** *iScience,* 2020